# EUROPEAN PATENT APPLICATION

(11) **EP 3 623 379 A1**
(43) Date of publication of application: **18.03.2020**
(21) Application number: 18193892.9
(22) Date of filing: 11.09.2018
(51) Int. Cl.: C07K 14/415, C12Q 1/6895, C12N 15/82

(54) **BEET NECROTIC YELLOW VEIN VIRUS (BNYVV)-RESISTANCE MODIFYING GENE**

(71) Applicant: KWS SAAT SE & Co. KGaA, 37574 Einbeck (DE)
(72) Inventor: Münnekhoff, Ann-Katrin, 37574 Einbeck (DE); Stirnweis, Daniel Fabian, 37085 Göttingen (DE); Schulz, Britta, 37574 Einbeck (DE); Borchardt, Dietrich, 37574 Einbeck (DE); Törjék, Ottó, 37574 Einbeck (DE)

(57) **Abstract**

A method for establishing, restoring or increasing the Rz2 gene mediated resistance to the *Beet necrotic yellow vein virus* (BNYVV) in the target plant is enabled via the identification and provision of a gene which modulates said resistance according to the invention. In particular, the BNYVV resistance mediated by the Rz2 resistance gene depends on the presence of the gene of the present invention. The gene being involved in conferring said resistance, and embodiments of the present invention that are described in the preceding, offer additional applications, e.g., the use of the resistance modifying gene allele in *cis*-genetic or *trans*-genetic approaches, with the goal of developing new resistant cultivars.

SEQUENCE LISTING

<110>  KWS SAAT SE

<120>  Beet necrotic yellow vein virus (BNYVV)-resistance modifying gene

<130>  KWS0305EP

<160>  45

<170>  PatentIn version 3.5

<210>  1
<211>  9105
<212>  DNA
<213>  Beta vulgaris

<400>  1
atagatgacg tggtttaact tttgtactat ttacactcac tcctttgatc atgttttatg      60

atttttacgt ttgaaaaata tatttatgtc ggatattatt agattcatct tgatatatat     120

tttcaaaata ttaacttttt ataattatta tatgtaggga gttagatata ttaatagtta     180

agtttctgcg ttggcaagtg cgtccggaat tgtgtctcaa gtatgttgaa acgaaggaag     240

tatatatgtc attacaaagg tcacgatgta taaaaaagtg attgcattta catatgggtc     300

actatgtgtt ccacgtacaa tatttgacat gcataacctt ttaatccaca ataacattat     360

tacaatttgc aaaccaggtc aacataaatc ttactctaaa ctattaaaca tggggaaaat     420

caagtaatta acatattcct tttaacttca ccccaaaaga taaacatttt tcttagtggt     480

cgtttggtaa atacgaaatt atgtaggcaa tagaaattcc catgattttc atatttgtgt     540

agttgtttag ttgacagata gtgaggaaaa cgggggcaat gcaaattcat gagaatttga     600

aattcccaca aaatatacga atttatttac ctactcttcc cttggtcact cacaactgct     660

acataaattc ttatgaatta caactttcct actaccaaac aatttaaact aatttatagg     720

aatttcataa aggaattaat tttctacaaa ttgtaaattc ctatagaaat ttaattccca     780

ttgcaaccaa acaaacacta acactccgag agaaccaaca atccaactcc cgatcctaaa     840

accataacta caagctattt ttactactct attcctttta atataatcat gattcattat     900

cactagcctt tacaaattac aatattagta acagtggatt attttccaat aaaaaagaat     960

gattaatgtg tgattttgtt ataagttctt aaaactaata tggtttatgc tcgtgtgcaa    1020

tcattatcct atttttgcaa caaaatgcta agagcttttg ggttcaatgg tatcacttcc    1080

tattttctat atggaaaatt aaggatcaaa atttggtgga agcataattt caatgttgtc    1140

tggggtgggg attgggtaga ggtgatcatt ttggcccggc ccgtttttgg gccgggtttt    1200

gggcagaatt tttcggcccg cgggccgggc cgggccgggc ccgccccggt cgtttttttt    1260

tttgtttttg ggcgggcttt gggcaagcta aaaacgactt tttagtctat tttttggccc    1320

ggcccgtttt ggcccgcaaa agcccgctaa gcccgacccg aaatagcggg ctttggacac    1380

aaaaattcgg tccgaattta ggcctggccc ggccaatggg caaatttttt tggccgcggc 1440

ccggcccatg atcacctcta ggattcgggt acttgttgga catctgggtg agagtcttgt 1500

gcaagaacaa cccaaaagta aattcttctt accatgtggt ttccgaaagg aggcccaaaa 1560

aaatcaacaa aataacaaaa aacaaagatc aatgacaact actttttatt tttggtgtaa 1620

aggaggatca acgtaataat cttgtcgagt ttgatcccaa gtcttaactc ttaagtatta 1680

ttccacgaga tattactaac taggactgtt ctctaattct cttcaccatt aaaaaaaaaa 1740

tccgagccaa ataagttcaa ataatatcta actaaaacta atacaatact taaaaataag 1800

tttcgataaa attgaatcag gtctaattaa ttcaattcaa ataagttcgg attttttgag 1860

atgaaaacaa cgctaccttg gcatccacaa gaatgacaac tatgaaaagg tcattttggc 1920

tctattattt ttggtttgat tgcttgcacc aactctcaac tctcaactct ctagtctctc 1980

ctcgttctta ttcattccag ctaatgaagc tgctatcgtt atcataaact tccctctaac 2040

tcctttctaa cttcccttcc actactcttc ttcattttct ctctcctccc tcctttcttt 2100

tcttcctctt ttttctagga acttcacaat taaaaaaact taaatatact tggaagtact 2160

tttgattgta atacatatgg caacagagga agcaaataaa gtggtgactg aaacaccggc 2220

accaccggta actccgccac aagaaccagc agcaacagtt tcggaaaccc gggaatcacc 2280

ggttgcagtg gaagagaaaa gtttagtccc tgtatcaacc cctcttcctt ccaccactga 2340

aaaaactcct gcttcggttg aaagttagtt aacaattttt tttttaatca tctatgattt 2400

taattattat tatatgatga tcattatatt atagtatatg gctaattttg ctttattcaa 2460

gaatatttag tgatttaagg gcttttagaa taattagtga aatttagtta gttttttccc 2520

ctgtgatata aagatgggtc atgggtggta gttttctgtt ttccaattat atatttttac 2580

tatttttggt ggatgggtgt ttttattttt ccttaatttg gtattttttc tgcatattat 2640

ccatgtttgt taaggagatt acttgcttat tttaagaata tcatgagtgt tatttgggaa 2700

tattagggac ttatcttagg gctttttctg gatcatgagg tttgaagtaa agtggttggg 2760

cacttttata gtatattcaa aactattttt ttcgcttatt gctcatttaa acgatttcaa 2820

atgagcaaat aacaactata attgaagaaa ttagcctaag ctcatggtat gcctgagtgt 2880

atggtgtata atttgattat cttagacttt cttgtgcgtt gcgcagtgat taaagagtaa 2940

aggcaattcc agctaatttt ggttatacta agctttttta ctgctaattg atcctttgtt 3000

actttgtcct ttgatgataa acctatgtaa gcaaagtgca ctattcatga atcatgatac 3060

tggcctggcc attagctatt atctatgtta cttagacaca cggattgggt gtctactcgg 3120

atgtgggtgt ccaagtgtct gactcggcta ttttggaaaa aagtcagttt ccggtttaaa 3180

atgaagtata caagtaaagt gtcgtgttgg acacgacacg atacgacacc ccgtttttga 3240

ggtaaatgaa ggtgtcggag taacacaagc tattatcatt catcaaacag aaattagaag 3300

ctattacatc tttaactata cttggacaac cacctagcta tacacgagtc gggaactcaa 3360

ttagagtaag gtgagccata tgagccgcta agttggctgt ctataatcat gaataaacct 3420

aactaaatta aaagaactag taagagatca ggtatcctta acaaccattc ctgcataaga 3480

ttcgtcacac ttattttcat taatcatctt tactacctgc agtcaatcac tttcaattat 3540

aatatcccac gcatagcatt ggatagccat tttcattccg agcactatag ccttcccttc 3600

cgtcacattc gtttccctca agtgtttcac tttttgacac gccatacgaa tgatatgccc 3660

accatgatct tgtattcttt ttacaattcc cattccgatt cccacctcct taaagatcgc 3720

agcatctaca ttgatttcca gcacattata gcttggacag caccacacca tgatagaacg 3780

ctgcccacca gtctgatctg cagcaggcag actcatgtgt ctgctgtcct tcccctctgt 3840

acgagccagt tcaaaggcag atggagctgt acacagtcct gtagggtgac gggcaatgct 3900

gccatgataa acattgttcc gtttattcca aatcatccat gctattattg caactttaca 3960

acgctcctcc tatataacat ccatccacca tgcagcccag tcactagcac atcattggga 4020

ttctagaata ctctccaacc cgcttcttcc cacaggaata cagggtagtt ctaactcaac 4080

gcaagggtgt ttgttacata aatctttata attcttgcat agcatttcct tatgtaaatg 4140

aacatgctca aaatgtttta ttaaactatt ttgtttgaca tataatagaa tttcccttga 4200

agtaccagtg atgatttgca cagtatcagc aggagcacaa aagttgcaaa agcttagcta 4260

tgatgatgct ataaacttaa gagtatatac taggttatta tttataaact aaacttgagt 4320

cttaaggagg gttgaacttg gaattttaag ctcaaggaca ctcgagctca aattactaca 4380

aggcacaagc tgagttgaga tatcccaagt ttaagctgtc tttaacaccc ctacctatgc 4440

acgactgttg ctgtatactg ttttcttggt ttataaggag cctagagggc aatgatgcac 4500

atggtttgaa actttgttct tgtgttccag tgtatcacta cattaaaaaa tttcatctga 4560

gctagccgat gtatgctcat tatatcctgt aataaataca gttatcactt acttttttct 4620

atctgaggtt ggtttgctag attatgactt tggactctca gtatcgcaat atacgtataa 4680

atgaaatcaa agactcattt ttatctcata attttctctt taaatttgta ttttcattca 4740

gcgtgtgtta cattggctta aaatttaaaa taattcactt tttattgacc tcttctttga 4800

ttcactttta ttaactagta aactgtatct gatggacaga ggttacagaa ttatctgaag 4860

aaaagactct agaggaaaag cccatagagg agaagccttt agagggctca gttggtagag 4920

gtaatagttt cactcatctt gttgtaaacg agcttagtaa gtttgatttc agcttatatc 4980

tcattgttgt tgtattcaga tgctcagcta gcacgagttc aaactgagaa gaggatttca 5040

ctgatcaaag catgggaaga aagtgaaaaa tgcaaggccg aaaacaagta agagtacttt 5100

gtatggatat gattagatat tagtgctaca cccacaggct atacaaatat atattattat 5160

gtcatttaga gatcacatac tcaaaatagc aatgggaatc ttaagggtgc ttgaaccatg 5220

actgaattat ttggcttaag ttgttttttg gtgctaaaga atacgagact cctactccta 5280

gcaagaaaag aagaatctaa taagtcttaa ctccacaatt gcaaggtcgt taacaataga 5340

agaactcttg tgacttaatt ttagttaacc aagcgatagg taggcctcat gccaagttac 5400

aagatttttc acacatacgc gtgatatata tagctgtcta gcttgctctg agcctctgaa 5460

caataagctt ggcgaggttc ttaagtggtt tgtcacattg ctcgtaccaa ccagtatcac 5520

tcttgtcacc tgtcagtgca tcaattaggt ctccctatat acttgctgga cctgtgataa 5580

ccattctaca ctctgtatca attatttgta ttatacttga ataggtctaa aggccaacat 5640

ttgggttagt ctctaaaagt tgctggagta aatttgtgcc tatagttgaa tggttctgtg 5700

cttaaacctt ccctttgaat attgctaaga gttcagtctg tggcacatca catcggccat 5760

atatttagct gagtttagtg ctgatacaat tgcttaaccg ctccttcccc tacccttcag 5820

caactcttgg actgcctcct cctctgcatc agtgaatgta tgttgaatag tttagtcctt 5880

tccatcattc catgcctcct ttaaccctca tccactctaa ccaaaaacaa aatcaaattt 5940

gttatgtgat tttgatggtt gagaatttga gatacaaaat agccttttgc ctcctttacc 6000

ataacaacca ccgtattgtc aatttgttat agacccatat cgctcattag cctatatggg 6060

aaagtcttag atcaatttac tcattctgtt tccttttgtt cttgctccaa atatgtaggg 6120

agatacaaaa gaaagtgggt aatagagaag gagtaagtgc atataatgat ataattataa 6180

gcaaggcatg tttggcatct atctagaatc tggttttggg ttttaagatt tttcaagtca 6240

tatgatatga aatgtatgat ggacttaaaa taagtcatat tcaaagtaat catgtctata 6300

tcgaaatctg acaacagaac tcgaaaatat tttttctggt tctgacattt ctatttaatt 6360

ggaaacacaa atgataacga agagacacac tgttgataga ccctttaatt tgatggaagt 6420

tacaaaacaa atttacttac ttaatgaact gcgcagggca cacaagaaaa ttgctgctat 6480

cgaggcatgg gagaacagca agaaagcatc tcttgaggca gagctgaaaa agatcgaggt 6540

atagtgccat taaacaacta tctttatctc gagttatttt cgaacaatag aagttagttt 6600

aactgatgag gccagcttac atgaatgcag gaaagcttgg aaaaaaagaa ggcagaatat 6660

gcagaaaaaa tgaagaacaa agtggctatg actcacaagg aagcagagga gaagaaggct 6720

acggttgaag ccaaacgtgc tgaagatatt ctgaaagcgg aagagatggc tgctaagtat 6780

cgcgccactg gaaagccccc caagaaactt cttggtttgt tctaataagg actatgtgaa 6840

ttcagtaatt acttctataa atgtttgatg tgattaacaa ttaagcaagt caattagttt 6900

gtgaaaagga aattatagat aagttatttg tatttttttc ccatttgtat aattgtttta 6960

tggctttatt ggtttggtaa gtgtgtatag tttgggtaga acgatttaag aattggtgca 7020

catttgtttc tatgtggata ttgtttctgt tatgtaagta aattggatat tgtttctgtt 7080

atgtaagtaa attggatatt ggaccacaac ttgtgtctga tgtctatcat ttcctattgt 7140

cttgtcagtc gtttgcaaat tcgcaaatac aaaggtttaa cggaaacctg ttctcctctg 7200

aacagatagt tggaatcgtg tgatgcccac tccaagtgca tatactaaag tcccgctcgg 7260

agaacagttg tccaaatatc ctaaactagc tgaaaattat cctagatatg aattaaactt 7320

tagagcctcc aaagttatca cgttatgtta tatatgtacg tagaagaatt catccgctcc 7380

tgttagtgtt cagctaataa atcagtaagc tgttagttct gcataatgta ccagtactac 7440

tacaaggatt acttaagact gttttcttac taatgaggtc taatatgtga caaagattgt 7500

caagattatg taaatcatgc ctgttagtat aaaacaagct gcactgacta attttattca 7560

ctggttggag aatcgagcaa catgttcatt gctgtatctg aagtgtcacc tgatgcaaca 7620

gaccctgagt ctgtcctctg ctgctgctct gttttgacac ctacgtgttg cggaacttgg 7680

cctgttaccc ctgaacaagc ctctgaggaa ggtgaaacac gatcgatttt caagggccgc 7740

gcattttgga tcttcctaaa ggactctccg acctcggaat tccacataga aaagaagtga 7800

tcagagttgg ttctggaata tcctgagggg ttgtagaggg attctaatga caatcgagcc 7860

tcggtctcca atcggatact ttcccattgg accatatgac gggttgtatg tgactttgat 7920

ggtgtagttt ctgggtaaac gttgatggtt tttcgtttcc tcaagtggct gttccagtgg 7980

ttcttgacat cattgtctgt tcttcctggt agccttgctg ctatagcagc ccacctgtaa 8040

aaatagcatg cataatactt aggacagcta acctttgttt tatcaaccac gtaatttctg 8100

gatttgaggt atcttgctac cttgcataca agaaatgata aacatttata caatggatgc 8160

atctagcaaa gtgtctgtga tatagtgttt gtgttgtttt tctctccttt tgctacttca 8220

caaacgcata aaagacaatt gacggaagag ttataccttt tatagtctaa acgagttttt 8280

atctttaaaa ctaatatgac aacggaaaaa gtatcattta agtctttaag ataataaagt 8340

actagttgtc ttctctcctt ttattaagca cttggtatta ttatatatgc acaatataat 8400

gagatttagc gtaaaagttg tcactttttt taattggcga tgactataat tttaataaaa 8460

tatttcatag tttaggtaag agattatgaa atgataaaaa gtttcctaac tatttctaaa 8520

gagtatattt ttcatgtgat atatggtatt tgtaatcaca taattaggga taataaaaca 8580

attagaaaaa tacctattgc caaggagtcc atgaagttga ataacagtct cgttttcctc 8640

ttcagagaaa ggacctcgtc taatgtcagg tcttaaataa ttggtccatc gaagacggca 8700

actctttcca catcgagata tacctaacat ataacatcaa tatgaaattt tatcacataa 8760

attaacacat agaagtatta tattcaattt ttcatgaatg acatattcaa gcttaatgag 8820

ttaattcacc ttattggaag tatagtcata tacataatca agacaacctt tctactttat 8880

aggtagcaag taaattgtag gactttgagg tgggctgggt taggtccaag caagaagaaa 8940

gtacttccga aaaactgcta aagtattttt ctgtaagaat tactacacaa gaagcaccag 9000

tggtctagtg gtagaatagt accctgccac ggtacagacc cgggttcgat tcccggctgg 9060

tgcataaaat atcagagctg tgatgataat attgcgcctc tatgt 9105

<210> 2
<211> 4649
<212> DNA
<213> Beta vulgaris

<400> 2
atggcaacag aggaagcaaa taaagtggtg actgaaacac cggcaccacc ggtaactccg 60

ccacaagaac cagcagcaac agtttcggaa acccgggaat caccggttgc agtggaagag 120

aaaagtttag tccctgtatc aacccctctt ccttccacca ctgaaaaaac tcctgcttcg 180

gttgaaagtt agttaacaat ttttttttta atcatctatg attttaatta ttattatatg 240

atgatcatta tattatagta tatggctaat tttgctttat tcaagaatat ttagtgattt 300

aagggctttt agaataatta gtgaaattta gttagttttt tcccctgtga tataaagatg 360

ggtcatgggt ggtagttttc tgttttccaa ttatatattt ttactatttt tggtggatgg 420

gtgtttttat ttttccttaa tttggtattt tttctgcata ttatccatgt ttgttaagga 480

gattacttgc ttattttaag aatatcatga gtgttatttg ggaatattag ggacttatct 540

tagggctttt tctggatcat gaggtttgaa gtaaagtggt tgggcacttt tatagtatat 600

tcaaaactat ttttttcgct tattgctcat ttaaacgatt tcaaatgagc aaataacaac 660

tataattgaa gaaattagcc taagctcatg gtatgcctga gtgtatggtg tataatttga 720

ttatcttaga ctttcttgtg cgttgcgcag tgattaaaga gtaaaggcaa ttccagctaa 780

ttttggttat actaagcttt tttactgcta attgatcctt tgttactttg tcctttgatg 840

ataaacctat gtaagcaaag tgcactattc atgaatcatg atactggcct ggccattagc 900

tattatctat gttacttaga cacacggatt gggtgtctac tcggatgtgg gtgtccaagt 960

gtctgactcg gctattttgg aaaaaagtca gtttccggtt taaaatgaag tatacaagta 1020

aagtgtcgtg ttggacacga cacgatacga caccccgttt ttgaggtaaa tgaaggtgtc 1080

ggagtaacac aagctattat cattcatcaa acagaaatta gaagctatta catctttaac 1140

tatacttgga caaccaccta gctatacacg agtcgggaac tcaattagag taaggtgagc 1200

catatgagcc gctaagttgg ctgtctataa tcatgaataa acctaactaa attaaaagaa 1260

ctagtaagag atcaggtatc cttaacaacc attcctgcat aagattcgtc acacttattt 1320

tcattaatca tctttactac ctgcagtcaa tcactttcaa ttataatatc ccacgcatag 1380

cattggatag ccattttcat tccgagcact atagccttcc cttccgtcac attcgtttcc 1440

ctcaagtgtt tcactttttg acacgccata cgaatgatat gcccaccatg atcttgtatt 1500

ctttttacaa ttcccattcc gattcccacc tccttaaaga tcgcagcatc tacattgatt 1560

tccagcacat tatagcttgg acagcaccac accatgatag aacgctgccc accagtctga 1620

tctgcagcag gcagactcat gtgtctgctg tccttcccct ctgtacgagc cagttcaaag 1680

gcagatggag ctgtacacag tcctgtaggg tgacgggcaa tgctgccatg ataaacattg 1740

ttccgtttat tccaaatcat ccatgctatt attgcaactt tacaacgctc ctcctatata 1800

acatccatcc accatgcagc ccagtcacta gcacatcatt gggattctag aatactctcc 1860

aacccgcttc ttcccacagg aatacagggt agttctaact caacgcaagg gtgtttgtta 1920

cataaatctt tataattctt gcatagcatt tccttatgta aatgaacatg ctcaaaatgt 1980

tttattaaac tattttgttt gacatataat agaatttccc ttgaagtacc agtgatgatt 2040

tgcacagtat cagcaggagc acaaaagttg caaaagctta gctatgatga tgctataaac 2100

ttaagagtat atactaggtt attatttata aactaaactt gagtcttaag gagggttgaa 2160

cttggaattt taagctcaag gacactcgag ctcaaattac tacaaggcac aagctgagtt 2220

gagatatccc aagtttaagc tgtctttaac acccctacct atgcacgact gttgctgtat 2280

actgttttct tggtttataa ggagcctaga gggcaatgat gcacatggtt tgaaactttg 2340

ttcttgtgtt ccagtgtatc actacattaa aaaatttcat ctgagctagc cgatgtatgc 2400

tcattatatc ctgtaataaa tacagttatc acttactttt ttctatctga ggttggtttg 2460

ctagattatg actttggact ctcagtatcg caatatacgt ataaatgaaa tcaaagactc 2520

atttttatct cataattttc tctttaaatt tgtattttca ttcagcgtgt gttacattgg 2580

cttaaaattt aaaataattc actttttatt gacctcttct ttgattcact tttattaact 2640

agtaaactgt atctgatgga cagaggttac agaattatct gaagaaaaga ctctagagga 2700

aaagcccata gaggagaagc ctttagaggg ctcagttggt agaggtaata gtttcactca 2760

tcttgttgta aacgagctta gtaagtttga tttcagctta tatctcattg ttgttgtatt 2820

cagatgctca gctagcacga gttcaaactg agaagaggat ttcactgatc aaagcatggg 2880

aagaaagtga aaaatgcaag gccgaaaaca agtaagagta ctttgtatgg atatgattag 2940

atattagtgc tacacccaca ggctatacaa atatatatta ttatgtcatt tagagatcac 3000

atactcaaaa tagcaatggg aatcttaagg gtgcttgaac catgactgaa ttatttggct 3060

taagttgttt tttggtgcta aagaatacga gactcctact cctagcaaga aaagaagaat 3120

ctaataagtc ttaactccac aattgcaagg tcgttaacaa tagaagaact cttgtgactt 3180

aattttagtt aaccaagcga taggtaggcc tcatgccaag ttacaagatt tttcacacat 3240

acgcgtgata tatatagctg tctagcttgc tctgagcctc tgaacaataa gcttggcgag 3300

gttcttaagt ggtttgtcac attgctcgta ccaaccagta tcactcttgt cacctgtcag 3360

tgcatcaatt aggtctccct atatacttgc tggacctgtg ataaccattc tacactctgt 3420

atcaattatt tgtattatac ttgaataggt ctaaaggcca acatttgggt tagtctctaa 3480

aagttgctgg agtaaatttg tgcctatagt tgaatggttc tgtgcttaaa ccttcccttt 3540

gaatattgct aagagttcag tctgtggcac atcacatcgg ccatatattt agctgagttt 3600

agtgctgata caattgctta accgctcctt cccctaccct tcagcaactc ttggactgcc 3660

tcctcctctg catcagtgaa tgtatgttga atagtttagt cctttccatc attccatgcc 3720

tcctttaacc ctcatccact ctaaccaaaa acaaaatcaa atttgttatg tgattttgat 3780

ggttgagaat ttgagataca aaatagcctt ttgcctcctt taccataaca accaccgtat 3840

tgtcaatttg ttatagaccc atatcgctca ttagcctata tgggaaagtc ttagatcaat 3900

ttactcattc tgtttccttt tgttcttgct ccaaatatgt agggagatac aaaagaaagt 3960

gggtaataga gaaggagtaa gtgcatataa tgatataatt ataagcaagg catgtttggc 4020

atctatctag aatctggttt tgggttttaa gatttttcaa gtcatatgat atgaaatgta 4080

tgatggactt aaaataagtc atattcaaag taatcatgtc tatatcgaaa tctgacaaca 4140

gaactcgaaa atattttttc tggttctgac atttctattt aattggaaac acaaatgata 4200

acgaagagac acactgttga tagacccttt aatttgatgg aagttacaaa acaaatttac 4260

ttacttaatg aactgcgcag ggcacacaag aaaattgctg ctatcgaggc atgggagaac 4320

agcaagaaag catctcttga ggcagagctg aaaaagatcg aggtatagtg ccattaaaca 4380

actatcttta tctcgagtta ttttcgaaca atagaagtta gtttaactga tgaggccagc 4440

ttacatgaat gcaggaaagc ttggaaaaaa agaaggcaga atatgcagaa aaaatgaaga 4500

acaaagtggc tatgactcac aaggaagcag aggagaagaa ggctacggtt gaagccaaac 4560

gtgctgaaga tattctgaaa gcggaagaga tggctgctaa gtatcgcgcc actggaaagc 4620

cccccaagaa acttcttggt ttgttctaa 4649

<210> 3
<211> 633
<212> DNA
<213> Artificial Sequence

<220>
<223> cDNA of remorin gene - haplotype 1

<400> 3
atggcaacag aggaagcaaa taaagtggtg actgaaacac cggcaccacc ggtaactccg 60

ccacaagaac cagcagcaac agtttcggaa acccgggaat caccggttgc agtggaagag 120

aaaagtttag tccctgtatc aacccctctt ccttccacca ctgaaaaaac tcctgcttcg 180

gttgaaaagg ttacagaatt atctgaagaa aagactctag aggaaaagcc catagaggag 240

aagcctttag agggctcagt tggtagagat gctcagctag cacgagttca aactgagaag 300

aggatttcac tgatcaaagc atgggaagaa agtgaaaaat gcaaggccga aaacaaggca 360

cacaagaaaa ttgctgctat cgaggcatgg gagaacagca agaaagcatc tcttgaggca 420

gagctgaaaa agatcgagga aagcttggaa aaaaagaagg cagaatatgc agaaaaaatg 480

aagaacaaag tggctatgac tcacaaggaa gcagaggaga agaaggctac ggttgaagcc 540

aaacgtgctg aagatattct gaaagcggaa gagatggctg ctaagtatcg cgccactgga 600

aagcccccca agaaacttct tggtttgttc taa 633

<210> 4
<211> 210
<212> PRT
<213> Beta vulgaris

<400> 4

Met Ala Thr Glu Glu Ala Asn Lys Val Val Thr Glu Thr Pro Ala Pro
1               5                   10                  15

Pro Val Thr Pro Pro Gln Glu Pro Ala Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Thr Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Ile Leu Lys Ala Glu Glu Met
            180                 185                 190

Ala Ala Lys Tyr Arg Ala Thr Gly Lys Pro Pro Lys Lys Leu Leu Gly
        195                 200                 205

Leu Phe
    210

<210>  5
<211>  9944
<212>  DNA
<213>  Beta vulgaris

<400>  5
atagatgacg tggtttaact tttgtactat ttacactcac tcctttgatc atgttttatg      60

atttttacgt ttgaaaaata tatttatgtc ggatattatt agattcatct tgatatatat     120

tttcaaaata ttaacttttt ataattatta tatgtaggga gttagatata ttaatagtta     180

agtttctgcg ttggcatgtg cgtccggaat tgtgtctcaa gtatgttgaa acgaaggaag     240

tatatatgtc attacaaagg tcgcgatgta taaaaaagtg attgcattta catatgggtc     300

actatgtgtt ccacgtacaa tatttgacat gcataacctt ttaatccaca ataacattat     360

tacaatttgc aaaccaggtc aacataaatc ttactctaaa ctattaaaca tggggaaaat     420

caagtaatta acatattcct tttaacttca ccccaaaaga taaacatttt tcttagtggt     480

cgtttggtaa ataggaaatt atgtaggcaa tagaaattcc catgattttc atatttgtgt     540

agttgtttag ttgacagaaa gtgaggaaaa caggggcaat gcaaattcat gagaatttga     600

aattcccaca aaatatacga atttatttac ctactcttcc cttggtcact cacaattgct     660

acataaattc ttatgaatta caactttcct actaccaaac aatttaaact aatttatagg     720

aatttcataa aggaattaat tttctacaaa ttgtaaattc ctatagaaat ttaattccca     780

ttgcaaccaa acaaacacta acacttcgag agaaccaaca atccaactcc cgatcctaaa     840

accataacta caagctattt ttactactct attcctttta atataatcat gattcattat     900

cactagcctt tacaaattac aatattagta acagtggatt attttccaat aaaaaagaat     960

gattaatgtg tgattttgtt ataagttctt aaaactaata tggtttatgc tcgtgtgcaa    1020

tcattatcct atttttgcaa caaaatgcta agagcttttg ggttcaatgg tatcacttcc    1080

tattttctat atggaaaatt aaggatcaaa atttggtgga agcatagttt caatgttgtc    1140

tggggtgggg attggggtac ttcttggaca tctgggtgag agtcttgtgc aagaacaacc    1200

cagaggtaaa ttcttcttac catgtgattt ccaaaaggag gtccaaaaaa atcaacaaaa    1260

taacaaaaaa acaaagatca atgacaacta ctttttattt ttggtgtaaa ggaggatcaa    1320

cgtaataatc ttgtcgagtt cgatcccaag tcttaactct taagtattat tccacgagat 1380

attactaact aggattgttc tctacttctc tccaccatta aaaaaaaaaa ttcgagtcca 1440

ataagttcaa ataatatcta actaaaacta atataatatt taaaaataag tttcaataag 1500

aatcaatcag gtctaattaa ttcaattcaa ataagttcgg attttttttg agatgaaaac 1560

aacaatacct tgacatccac aagaatgaca agtatgaaaa ggtcattttg gctctattat 1620

ttttggtttg attgcttgca ccaactctca actctctctc tagtctctcc tcgttcttat 1680

tcattccagc taatgaagct gctatcgtta tcataaactt ccctctaact cctttctaac 1740

ttcccttcca ctactcctct tcattttctc tctcctccct cctttctttt cttcctcttt 1800

tttctaggaa cttcacaata aaaaaaactt aaatatactt ggaagtactt ttgattgtaa 1860

tacatatggc aacagaggaa gcaaataaag tggtgactga aacaccggca ccaccggtaa 1920

ctccgccgca agaaccgcca gcaacagttt cagaaacccg ggaatcaccg gttgcggtgg 1980

aagagaaaag tttagtccct gtatcaaccc ctcttccttc caccactgaa aaaactcctg 2040

cttcggttga aagttagtta acaatttatt tttgtagtat atactttact tatctataat 2100

tttaattatt atatgatgat cattatatta tagtatatgg ctaattttgc tttattcaag 2160

aatatttagt gatttaaggg cttttaattt tattaaactt ttctcaattc ccctgttctt 2220

tttaatcatt acttgaagtt taggtagagt tcgtttggtt cacctcaaat tctattggaa 2280

ttcggatttt ttaggaagta caaaatagga tagttgtttt gtatgcacga attagggaat 2340

tcatggtaaa cagaatttcc aaggaaatgg aaattctgat gtataggaaa ttaatcacaa 2400

cctcttagta atttgaaatt cctatgtcaa ttacaaattt atatgctcaa ccaaatacca 2460

aaaattattt tctcggatat agacaagaga atttacttct tatgaaatca gacttcccat 2520

gagaattaag tttcaacggt aaccaaacga gtttttagtt ttttcccatg tgatataaag 2580

atgggtcatg ggtggtagtt ttccaattat atatttttac tatttttggt ggatgggtgt 2640

tcttattttt ccttaatttg ttatttattt ctgcttatta tccatgtttg ttaaggagat 2700

tacttgctta ttttaagaat atcatgagtg ttatttggga ataataggga cttatctgag 2760

ggctttttct ggatcatgag gtttgaagta aagtggttgg gcaactttat atcctttaac 2820

ttgggcactt ttatagtata ttcaaaacta ttttttcgct cattgctcat ttaaatgatt 2880

tcaaatgagc aaattaacaa ctataattga agaaattagc ctaagctcat ggtacgcctg 2940

aatgtatggt gtagattttg attatcttag actttcttgt gcgttacaca gtgattaaag 3000

agtaaaggca aagtgctaat tttggttata ctaagttttt aatactattt gatcctttgt 3060

tactttgtcc tttgatgata aaacctatgt aagcaaagtg cactattcat gaatcatgat 3120

actggccatt agctattatc tatgttactt agacacacag attggatgtc tactcggatg 3180

tgggtgtcca aagtgtctga ttcggctatt ttggaaaaaa gtctaagttt ccggtttaaa 3240

atgaagcata caattaaagt gtcatgtcag acacgacacg acacgacacc ccgtttttga 3300

ggtaaatgaa ggtgtcggat taacacaggc tattatcatt catcaaacag aaattagaag 3360

ctattacatc tttaactata cttggacaac cacctagcta tacacgagtc gggaactcaa 3420

ttagagtaag gtgagccata tgagccgcta agttggctgt ctataatcat gaataaacgt 3480

aactaaatta aaagaactag taagagatca ggtatcctta acaaccatat ctgcataaga 3540

ttcatcacac ttgttttcat taatcatctt tactacctgc aggcaattac tttcaattat 3600

aatatcccac gcatagcatt gggtagccat tttcattccg agcactatag ccttcccttc 3660

tgtcacattc gtttcccaca agtgtttcac tttttgacac gccatatgaa tgatatgccc 3720

accatgatct cgtataacaa ttcccattcc gattcccacc tccttaaaga tcgcagcgtc 3780

tacattgatt ttcagcacat tatagcttgg acagcaccac accacactag agtgctgccc 3840

accagtctga tctgcagcag gcagactcat gtgtatgctg tccttcccct ctgtaccagc 3900

cagttcaaag gcagctggtg ctgtacacag ttctggaagg tgacgggcaa tgctgccatg 3960

ataaacattg ttccgttcat tccaaatcat ccatgccagt attgcaactt tacaactctc 4020

ctcctataaa acatccatcc accatgcaac ccagtcacta gcgcatcata ttgggattcc 4080

agaatactct ccaacccgcc tcttcccaaa ggatactggg tatttcttaa tcactatttc 4140

taaatcaacg caagggtgtt tgttacataa atctttacaa ttcttgcata gcatttcctt 4200

atgtaaatga acatgctcaa aatgttttat caaactattt ttgttaaaca tataatagaa 4260

tttcccttga agtaccagtg atgtgcacag tatcagcggg agctcaaaag ttgcaaaagc 4320

ttagctatga tgatgctata aacttaagag tatatatcag gttattattt ataaactaaa 4380

gttgagtctt aaggtgggtt gaacttggaa ttttgtgctc aaagacactc gagctcaaat 4440

tactacaagg cacaagctga gttgtgatat cccaagttta agctgtcttt aacaccccta 4500

cctataacct atgcacgact gttgctattt actgttttct tggttcataa ggagcctaga 4560

gggcaatgat gcacatggtt ggaaactttg ttcttgtgtt tcagtgtatc actacattag 4620

aaaattttca tccgagctag ccaatgtatg ctcattatat cttgtaataa atacagctat 4680

cacttacttt tttctatctg aggttgtttg ctagattatg actttggact agcagtatcg 4740

caaaatgcgt ataaatgaaa tcaaagactc atttttatct cataattttc tctttaaatt 4800

tgtattttca ttcagcgtgt gttaattggc ttcaaattta aaattattca ctttttattg 4860

acctcttttt tttagaggat tcacttttat taactagtac actgtatctg atggacagag 4920

gttacagaat tatctgaaga aaagcctcta gaggaaaagc ctatagagga gaagccttta 4980

gagggctcag ttggtagagg taatagtttc actcatcttg ttgtaaacga gcttaataag 5040

ttcagtttca gcttatatct tattgttgtt gtattcagat gctcagctag cacgagttca 5100

aactgagaag agaatttcac tgatcaaagc atgggaagaa agtgaaaaat gcaaggccga 5160

aaacaagtaa gagtactttg tatggatatg attagatatt agtactacac ccacaggcta 5220

tacaaatata cattatcatg tcatttagag atcacatact caaaatagca atgggaatct 5280

caggggtgct tgaaccatga gtgaattatt tggctaaagt tgttttttgg tgctaaagaa 5340

tatgagactc ctagcaagaa aagaagaatc taataagtct taactccacc attgcaaggt 5400

cgttaacaat agaaaaactc ttgtgactta atttaagtta accaagcgat aggtaggcct 5460

catgccaagt tacaagattt ttcacacata cgcgtgacaa atatagctgt ctagcttgct 5520

cttaacaata agcttggcga ggttcttaag tggtttgtca cattgctcgt accaaccagt 5580

atcactcttg tcacctgtca atgcatcaat tagctctccc tatatacttg ctggacctgg 5640

gataaccatt atatactctg tattaattct ttgtattata cttgaatagg tctaaaggcc 5700

aacatttggg ttagtctcta gaagttgctg gagtaaattt gtgcctatag ttgaatggtt 5760

ccgtgcttta accttccctt tgaatatcgc taagagttca gtctttggag catcacatcg 5820

accatatatt tagctgacta agcgctgata caattgctta agcgctcctt cccctactgt 5880

ttagcaactc ttggactgcc tcatcctttg catcagtgaa cgtatgttga gtagtttaat 5940

cctttccatc attccatgac tcctttaacc ctcatccact ctagccaaaa acaaaatcaa 6000

atttgttgtg tgattttgat ggttgagaat ttgagataca aaatagcctt ttgcctcctt 6060

taccataacc accgtattgt caatttgtta taagacccat atgactcatt agcctatatg 6120

ggaaagtctt agatcaattt actcattctg tttccatttg ttcttgcttc aaattagagg 6180

tggttattgg acagggtagt ccaatggtta aagggttagg gtcaatttta cagggctttt 6240

attggtcagg gttttattgg acagggttat tattggatag ggtcattata gggtcaacaa 6300

agaaacccaa taattttata aaataaaatt agttttgtaa aaacaattac atattcggat 6360

tcgcattcat ttatacttac ttttggctct tcttgttcct tgtttttctt gctcgtttac 6420

cgacccaccc attatcgacc cgctataagc cctctaccca tttggcccga cctgctaccg 6480

accctttatg aggcgaccct cttatgaccc aaacccgctt ccgacccgta ctgaccctga 6540

cccgacccgt ccaataacca ggtctacttc aaatatgtag gaagatacaa aagaaagtgg 6600

gtaataaaga agaaagagta agcgcatata atgatataat tataagcaag gcatgtttgg 6660

catctatcta gaatctggtt ttgggtttta agatttttca agtcatatga tgtgaaatgt 6720

atgatggact taaattaagc catattcaaa gtaatcatgt ctatatcgaa atctgacaac 6780

agaactcgaa aatatttttt ctggtttgga catttctatt taattggaaa cacaaatgat 6840

aacgaagaga cactctattg atagaccctt taatttgatg gaagttacac aaatttactt 6900

gctaaaaatt gcgcagggca cacaagaaaa ttgctgctat cgaggcatgg gagaacagca 6960

agaaagcatc tcttgaggca gagctgaaaa agatcgaggt atagtgccat taaacagcta 7020

tctttatctc aagttatttt cgaacaatag aagtttgttg agctgatgag gccagcttac 7080

atgagtgcag gaaagcttgg aaaaaaagaa ggcagaatat gcagaaaaaa tgaagaacaa 7140

agtggctatg actcacaagg aagcagagga gaagaaggct acggttgaag ccaaacgtgc 7200

tgaagatgtt ctgaaagcgg aagagatggc tgctaagtat cgcgccactg gaaagccccc 7260

caagaaactt cttggtttgt tctaataagg attatgtgaa tttagtaatt acttctataa 7320

atgtttgatg tgattaacaa ttaagcaagt caattagttt gtgataagga aattatagat 7380

aagttatttg tatttttttt tccatttgta taattgtttt atggctttat tggtttggta 7440

ggtgtgtata gtttgggtag aacgatttaa gaattggtgc acatttgttt ccatgtggat 7500

attgtttctg ttatgtaagt aaattggata ttgtttctat tatgtaagta aattggatat 7560

tggaccacaa cttgtgtctg atgtctatca tttcctattg tcttatcagt cgtttgcaaa 7620

ttcgcaaata caaaggttta acgggaaacc tgttctcctc taaacagata gctggaatcg 7680

tgtgatgccc actccaagtg catatactaa agttcccctc ggagaacagt tgtccaaatg 7740

tcctaaatta ggcgaaaagt tatcacgtta tgttatatat gtacgtagga gaattcatcc 7800

gctcctgtta gtgttcagct aataaatcag taagctgtta gttctgcata atgtaccagt 7860

actactacaa ggattactta agactgtttt cttactaatg aggtctaata tgtgacaaag 7920

attgtcaaga ttatgtaaat catgcctgtt agtataaaac aaactgcact gactaatttt 7980

attcactggt tggagaatcg agcaacatgt tcattgctgt atctgaagtg tcacctgatg 8040

caacagaccc tgagtctgtc ctctgctgct gctctgtttt gacacctacg tgttgcggaa 8100

cttggcctgt tacccctgaa caagcctctg aggaaggtga aacacgatcg attttcaagg 8160

gccgcgcatt ttggatcttc ctaaaggact ctccgacctc ggaattccac atagaaaaga 8220

agtgatcaga gttggttctg gaatatcctg aggggttgta gagggattct aatgacaatc 8280

gagcctcggt ctccaatcgg atactttccc attggaccat atgacgggtt gtatgtgact 8340

ttgatggtgt agtttctggg taaacgttga tggtttttcg tttcttcaag tggctgttcc 8400

agtggttctt gacatcattg tctgttcttc ctggtagcct tgctgctata gcagcccacc 8460

tgtaaaaata gcatgcataa tacttagaat agctaacctc tgttttatca accacggaat 8520

ttctagattt gagtagaggc gatcatggac cgggttcggg ccgggcttgg aagttgccca 8580

ttggttgggc cggggcaaaa ttttatgtcg aaagccggct atttcgggcc aaagtgagcg 8640

gcttttcagc ccaaaatcgg gtctggccga aaaatagact aaaaagtcat ttttggcttg 8700

tccaaagcct gttcaaagaa aaaaaaattg gcccggaccg ggcccgacag tggccaaaaa 8760

attctgcaca aaacccgcac aaaaagcggg ccgggtcaaa atgatcatgc tctagatttg 8820

aggtatcttg ctaccttgca tacaagaaat gataaatatc tatacaatgg atgcatctag 8880

caaagtgtgt atataaatag tatttgtcat atagtatttg tgttgttttt ctctcctttt 8940

gctactttac aaacatataa aaaataatga cggcacaggc gaagttaggc ttgattgtgg 9000

aggggagcta attttcttca aagctatgtt ggggcctgta agggccatga ttcttttagg 9060

ccctaacata gcctcgttag ttgccagttt ggttgattgg agagttatac tctttttgta 9120

gtctaagcta gcttttatct ttaaaactaa tttgacaacg agaaaagtat cattttaagt 9180

ctttgagata acaaagtatt agttgtcttc tcttctttta taaaccttgg gtagtattat 9240

atatgcacaa tagaataaga ttcagtgtaa aagttgtcac cttttctaat agaatagtgg 9300

caatgactat aactttttaa aaatacttca taatataggt aagagactat gaaatgataa 9360

aaattgccta actatttcta acaaatatat tttttatgtg atatatggta ttcataatca 9420

cataattagg gataataaac aataagcata ataaacatag cattagagga gtaataacgt 9480

acctattgcc aaggaggcca tgaagttgaa taacaatctt gttttcctct tcggagaaag 9540

gacctcgtct aatgtcaggt cttaaataat tggtccatcg aagacgacaa ctctttccac 9600

atcgagatat acctaacata taatcataac ataaaatttt atcacataaa ttaacacata 9660

gaagtaatat tttcaatttt tcatgaaatg tatcaagctt aatgagttaa ttcaccttat 9720

tggatgtata gtcatataca taatcaagac aacctttcta ggtagcaagt aacttgtggg 9780

actttgaggt gggctgtaaa ctatttttca gtaagcatta ttaaacaaga agcaccagtg 9840

gtctagtggt agaatagtac cctgccacgg tacagacccg ggttcgattc ccggctggtg 9900

cataaaatta tcagagcaat gatgataaca ttgcgcctct atgt 9944

<210> 6
<211> 5420
<212> DNA
<213> Beta vulgaris

<400> 6
atggcaacag aggaagcaaa taaagtggtg actgaaacac cggcaccacc ggtaactccg 60

ccgcaagaac cgccagcaac agtttcagaa acccgggaat caccggttgc ggtggaagag 120

aaaagtttag tccctgtatc aacccctctt ccttccacca ctgaaaaaac tcctgcttcg 180

gttgaaagtt agttaacaat ttatttttgt agtatatact ttacttatct ataattttaa 240

ttattatatg atgatcatta tattatagta tatggctaat tttgctttat tcaagaatat 300

ttagtgattt aagggctttt aattttatta aacttttctc aattcccctg ttctttttaa 360

tcattacttg aagtttaggt agagttcgtt tggttcacct caaattctat tggaattcgg 420

attttttagg aagtacaaaa taggatagtt gttttgtatg cacgaattag ggaattcatg 480

gtaaacagaa tttccaagga aatggaaatt ctgatgtata ggaaattaat cacaacctct 540

tagtaatttg aaattcctat gtcaattaca aatttatatg ctcaaccaaa taccaaaaat 600

tattttctcg gatatagaca agagaattta cttcttatga aatcagactt cccatgagaa 660

ttaagtttca acggtaacca aacgagtttt tagttttttc ccatgtgata taaagatggg 720

tcatgggtgg tagttttcca attatatatt tttactattt ttggtggatg ggtgttctta 780

tttttcctta atttgttatt tatttctgct tattatccat gtttgttaag gagattactt 840

gcttatttta agaatatcat gagtgttatt tgggaataat agggacttat ctgagggctt 900

tttctggatc atgaggtttg aagtaaagtg gttgggcaac tttatatcct ttaacttggg 960

cacttttata gtatattcaa aactattttt tcgctcattg ctcatttaaa tgatttcaaa 1020

tgagcaaatt aacaactata attgaagaaa ttagcctaag ctcatggtac gcctgaatgt 1080

atggtgtaga ttttgattat cttagacttt cttgtgcgtt acacagtgat taaagagtaa 1140

aggcaaagtg ctaattttgg ttatactaag tttttaatac tatttgatcc tttgttactt 1200

tgtcctttga tgataaaacc tatgtaagca aagtgcacta ttcatgaatc atgatactgg 1260

ccattagcta ttatctatgt tacttagaca cacagattgg atgtctactc ggatgtgggt 1320

gtccaaagtg tctgattcgg ctattttgga aaaaagtcta agtttccggt ttaaaatgaa 1380

gcatacaatt aaagtgtcat gtcagacacg acacgacacg acaccccgtt tttgaggtaa 1440

atgaaggtgt cggattaaca caggctatta tcattcatca aacagaaatt agaagctatt 1500

acatctttaa ctatacttgg acaaccacct agctatacac gagtcgggaa ctcaattaga 1560

gtaaggtgag ccatatgagc cgctaagttg gctgtctata atcatgaata aacgtaacta 1620

aattaaaaga actagtaaga gatcaggtat ccttaacaac catatctgca taagattcat 1680

cacacttgtt ttcattaatc atctttacta cctgcaggca attactttca attataatat 1740

cccacgcata gcattgggta gccattttca ttccgagcac tatagccttc ccttctgtca 1800

cattcgtttc ccacaagtgt ttcacttttt gacacgccat atgaatgata tgcccaccat 1860

gatctcgtat aacaattccc attccgattc ccacctcctt aaagatcgca gcgtctacat 1920

tgattttcag cacattatag cttggacagc accacaccac actagagtgc tgcccaccag 1980

tctgatctgc agcaggcaga ctcatgtgta tgctgtcctt cccctctgta ccagccagtt 2040

caaaggcagc tggtgctgta cacagttctg gaaggtgacg ggcaatgctg ccatgataaa 2100

cattgttccg ttcattccaa atcatccatg ccagtattgc aactttacaa ctctcctcct 2160

ataaaacatc catccaccat gcaacccagt cactagcgca tcatattggg attccagaat 2220

actctccaac ccgcctcttc ccaaaggata ctgggtattt cttaatcact atttctaaat 2280

caacgcaagg gtgtttgtta cataaatctt tacaattctt gcatagcatt tccttatgta 2340

aatgaacatg ctcaaaatgt tttatcaaac tatttttgtt aaacatataa tagaatttcc 2400

cttgaagtac cagtgatgtg cacagtatca gcgggagctc aaaagttgca aaagcttagc 2460

tatgatgatg ctataaactt aagagtatat atcaggttat tatttataaa ctaaagttga 2520
gtcttaaggt gggttgaact tggaattttg tgctcaaaga cactcgagct caaattacta 2580
caaggcacaa gctgagttgt gatatcccaa gtttaagctg tctttaacac ccctacctat 2640
aacctatgca cgactgttgc tatttactgt tttcttggtt cataaggagc ctagagggca 2700
atgatgcaca tggttggaaa ctttgttctt gtgtttcagt gtatcactac attagaaaat 2760
tttcatccga gctagccaat gtatgctcat tatatcttgt aataaataca gctatcactt 2820
acttttttct atctgaggtt gtttgctaga ttatgacttt ggactagcag tatcgcaaaa 2880
tgcgtataaa tgaaatcaaa gactcatttt tatctcataa ttttctcttt aaatttgtat 2940
tttcattcag cgtgtgttaa ttggcttcaa atttaaaatt attcactttt tattgacctc 3000
ttttttttag aggattcact tttattaact agtacactgt atctgatgga cagaggttac 3060
agaattatct gaagaaaagc ctctagagga aaagcctata gaggagaagc ctttagaggg 3120
ctcagttggt agaggtaata gtttcactca tcttgttgta aacgagctta ataagttcag 3180
tttcagctta tatcttattg ttgttgtatt cagatgctca gctagcacga gttcaaactg 3240
agaagagaat ttcactgatc aaagcatggg aagaaagtga aaaatgcaag gccgaaaaca 3300
agtaagagta ctttgtatgg atatgattag atattagtac tacacccaca ggctatacaa 3360
atatacatta tcatgtcatt tagagatcac atactcaaaa tagcaatggg aatctcaggg 3420
gtgcttgaac catgagtgaa ttatttggct aaagttgttt tttggtgcta aagaatatga 3480
gactcctagc aagaaaagaa gaatctaata agtcttaact ccaccattgc aaggtcgtta 3540
acaatagaaa aactcttgtg acttaattta agttaaccaa gcgataggta ggcctcatgc 3600
caagttacaa gatttttcac acatacgcgt gacaaatata gctgtctagc ttgctcttaa 3660
caataagctt ggcgaggttc ttaagtggtt tgtcacattg ctcgtaccaa ccagtatcac 3720
tcttgtcacc tgtcaatgca tcaattagct ctccctatat acttgctgga cctgggataa 3780
ccattatata ctctgtatta attctttgta ttatacttga ataggtctaa aggccaacat 3840
ttgggttagt ctctagaagt tgctggagta aatttgtgcc tatagttgaa tggttccgtg 3900
ctttaacctt ccctttgaat atcgctaaga gttcagtctt tggagcatca catcgaccat 3960
atatttagct gactaagcgc tgatacaatt gcttaagcgc tccttcccct actgtttagc 4020
aactcttgga ctgcctcatc ctttgcatca gtgaacgtat gttgagtagt ttaatccttt 4080
ccatcattcc atgactcctt taaccctcat ccactctagc caaaaacaaa atcaaatttg 4140
ttgtgtgatt ttgatggttg agaatttgag atacaaaata gccttttgcc tcctttacca 4200
taaccaccgt attgtcaatt tgttataaga cccatatgac tcattagcct atatgggaaa 4260
gtcttagatc aatttactca ttctgtttcc atttgttctt gcttcaaatt agaggtggtt 4320
attggacagg gtagtccaat ggttaaaggg ttagggtcaa ttttacaggg cttttattgg 4380

tcagggtttt attggacagg gttattattg gatagggtca ttatagggtc aacaaagaaa 4440

cccaataatt ttataaaata aaattagttt tgtaaaaaca attacatatt cggattcgca 4500

ttcatttata cttacttttg gctcttcttg ttccttgttt ttcttgctcg tttaccgacc 4560

cacccattat cgacccgcta taagccctct acccatttgg cccgacctgc taccgaccct 4620

ttatgaggcg accctcttat gacccaaacc cgcttccgac ccgtactgac cctgacccga 4680

cccgtccaat aaccaggtct acttcaaata tgtaggaaga tacaaaagaa agtgggtaat 4740

aaagaagaaa gagtaagcgc atataatgat ataattataa gcaaggcatg tttggcatct 4800

atctagaatc tggttttggg ttttaagatt tttcaagtca tatgatgtga aatgtatgat 4860

ggacttaaat taagccatat tcaaagtaat catgtctata tcgaaatctg acaacagaac 4920

tcgaaaatat tttttctggt ttggacattt ctatttaatt ggaaacacaa atgataacga 4980

agagacactc tattgataga ccctttaatt tgatggaagt tacacaaatt tacttgctaa 5040

aaattgcgca gggcacacaa gaaaattgct gctatcgagg catgggagaa cagcaagaaa 5100

gcatctcttg aggcagagct gaaaaagatc gaggtatagt gccattaaac agctatcttt 5160

atctcaagtt attttcgaac aatagaagtt tgttgagctg atgaggccag cttacatgag 5220

tgcaggaaag cttggaaaaa aagaaggcag aatatgcaga aaaaatgaag aacaaagtgg 5280

ctatgactca caaggaagca gaggagaaga aggctacggt tgaagccaaa cgtgctgaag 5340

atgttctgaa agcggaagag atggctgcta agtatcgcgc cactggaaag ccccccaaga 5400

aacttcttgg tttgttctaa 5420

<210> 7
<211> 633
<212> DNA
<213> Artificial Sequence

<220>
<223> cDNA of remorin gene - haplotype 2

<400> 7
atggcaacag aggaagcaaa taaagtggtg actgaaacac cggcaccacc ggtaactccg 60

ccgcaagaac cgccagcaac agtttcagaa acccgggaat caccggttgc ggtggaagag 120

aaaagtttag tccctgtatc aacccctctt ccttccacca ctgaaaaaac tcctgcttcg 180

gttgaaaagg ttacagaatt atctgaagaa aagcctctag aggaaaagcc tatagaggag 240

aagcctttag agggctcagt tggtagagat gctcagctag cacgagttca aactgagaag 300

agaatttcac tgatcaaagc atgggaagaa agtgaaaaat gcaaggccga aaacaaggca 360

cacaagaaaa ttgctgctat cgaggcatgg gagaacagca agaaagcatc tcttgaggca 420

gagctgaaaa agatcgagga aagcttggaa aaaaagaagg cagaatatgc agaaaaaatg 480

aagaacaaag tggctatgac tcacaaggaa gcagaggaga agaaggctac ggttgaagcc 540

aaacgtgctg aagatgttct gaaagcggaa gagatggctg ctaagtatcg cgccactgga 600

aagcccccca agaaacttct tggtttgttc taa 633

<210> 8
<211> 210
<212> PRT
<213> Beta vulgaris

<400> 8

Met Ala Thr Glu Glu Ala Asn Lys Val Val Thr Glu Thr Pro Ala Pro
1               5                   10                  15

Pro Val Thr Pro Pro Gln Glu Pro Pro Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Pro Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Val Leu Lys Ala Glu Glu Met
            180                 185                 190

Ala Ala Lys Tyr Arg Ala Thr Gly Lys Pro Pro Lys Lys Leu Leu Gly

195                 200                 205

Leu Phe
    210

<210>  9
<211>  8887
<212>  DNA
<213>  Beta vulgaris

<400>  9
atagatgacg tggtttaact tttgtactat ttacactcac tcctttgatc atgttttatg        60

atttttacgt ttgaaatata tatttatgtc ggatattatt agattcatct tgatatatat       120

tttcaaaata ttaacttttt ataattatta tatgtaggga gttagatata ttaatagtta       180

agtttctgcg ttggcaagtg cgtccggaat tgtgtctcaa gtatgttgaa acgaaggaag       240

tatatatgtc attacaaagg tcgcgatgta taaaaaagtg attgcattta catatgggtc       300

actatgtgtt ccacgtacaa tatttgacat gcataacctt ttaatccaca ataacattat       360

tacaatttgc aaaccaggtc aacataaatc ttactctaaa ctattaaaca tggggaaaat       420

caagtaatta acatattcct tttaacttca ccccaaaaga taaacatttt tcttagtggt       480

cgtttggtaa ataggaaatt atgtaggcaa tagaaattcc catgattttc atatttgtgt       540

agttgcttag ttgacagaaa gtgaggaaaa cgggggcaat gcaaattcat gagaatttga       600

aattcccaca aaatatacga atttatttac ctactcttcc cttggtcact cacaattgct       660

acataaattc ttatgaatta caactttcct actaccaaac aatttaaact aatttatagg       720

aatttcataa aggaaataat tttctacaaa ttgtaaattc ctatagaaat ttaattccca       780

ttgcaaccaa acaaacacta acactccgag agaaccaaca atccaactcc cgatcctaaa       840

accataacta caagctattt ttactactct attcctttta atataatcat gattcattat       900

caccagcctt tacaaattac aatattagta acagtggatt attttccaat aaaaaagaat       960

gattaatgtg tgattttgtt ataagttctt aaaactaata tggtttatgc tcgtgtgcaa      1020

tcattatcct atttttgcaa caaaatgcta agagcttttg ggttcaatgg tatcacttcc      1080

tattttctat atggaaaatt aaggatcaaa atttggtgga agcataattt caatgttgtc      1140

tggggtgggg attggggtac ttgttggaca tctgggtgag actcttgtgc aagaacaacc      1200

taaaagtatt cttcttacca tgtggtttcc gaaaggaggc ccaaaaaaat caacaaaata      1260

acaaaaaaca aagatcaatg acaactactt tttatttttg gtgtaaagga ggatcaacgt      1320

aataatcttg tcgagtttga tcccaagtct taactcttaa gtattattcc atgagatatt      1380

actaactagg actgttctct aattctcttc accattaaaa aaaaaatccg agccaaataa      1440

gttcaaataa tatctaacta aaactaatac aatacttaaa aataagtttc gataagattg      1500

aatcaggtct aattaattca attcaaataa gttcggattt tttgagatga aaacaacgct 1560
accttggcat ccacaagaat gacaactatg aaaaggtcat tttggctcta ttatttttgg 1620
tttgattgct tgcaccaact ctcaactctc aactctcaac tctcaactct ctagtctctc 1680
ctcgttctta ttcattccag ctaatgaagc tgctatcgtt atcataaact tccctctaac 1740
tcctttctaa cttcccttcc actactcctc ttcattttct ctctcctccc tccctccttt 1800
cttttcttct tctctttttt ctgggaactt cacaatatat taaaaaagta cttttgattg 1860
taatacatat ggcaacagag gaagcaaata aagtggtgac tgaaacaccg gcaccaccgg 1920
taactccgcc acaagaaccg ccagcaacag tttcagaaac ccgggaatca ccggttgcgg 1980
tggaagagaa aagtttagtc cctgtatcaa cccctcttcc ttccaccact gaaaaaactc 2040
ctgcttcggt tgaaagttag ttaacaattt atttttgtag tatatacttt acttatctat 2100
aattttaatt attatatgat gatcattata ttatagtata tggctaattt tgctttattc 2160
aagaatattt agtgatttaa gggcttttaa ttttattaaa cttttctcaa ttcccctgtt 2220
ctttttaatc attacttgaa gtttaggtag agttcgtttg gttcacctca aattctattg 2280
gaattcggat ttttttagga agtacaagat aggatagttg ttttgtatgc acgaattagg 2340
gaattcatgg taaacagaat ttccaaggaa atggaaattc tgatgtatag gaaattaatc 2400
acaacctctt agtaattaga aattcctatg tcaattacaa atttatatgc tcaaccaaat 2460
accaaaaatc attttctcgg atatagacaa gagaatttac ttcttatgaa atcagacttc 2520
ccatgagaat taagtttcaa cggtaaccaa acgagctttt agttttttcc catgtgatat 2580
aaagatgggt catgggtggt agttttccaa ttatatattt ttttggtgga tgggtgtttt 2640
tatttttcct taatttggta tattttctgc atattatcca tgtttgttaa ggagattact 2700
tgcttatttt aagaatatca tgagtgttta tttgggaata tcagggactt atctgagggc 2760
tttttctgga tcaggaggtt tgaattaaag tggttgggca actttatatc ctttaacttg 2820
ggcacttgta tagtatattc aaaactattt ttttcattca ttgctcattt aaatgatttc 2880
aaatgagcaa attaacaact ataattgaag aaattagcct aagctcatgg tacgcctgag 2940
tatatggtgt agattttgat tatcttagac tttcttgcgc gttacgcagt gattaaagag 3000
taaaggcaat tccagctaaa tagctaattt tggttatgct aagttttttg atactatttg 3060
attctttgtt tctttgtcct ttgatgataa aacctatgta agcaaagtgc actattcatg 3120
aatcatgata ctggccatta gctattatct atgttactta gacacacgga ttgggtgtct 3180
actcggatgt gggtgtccac tgtccaagtg tctgactcgg ctattttgga aaaaagtcta 3240
agtttccggt ttaaaatgaa gtatacaagt aaagtgtcgt gtcggacacg acacgacacg 3300
acaccccgtt tttgaggtaa atgaaggtgt cggagtaaca caagctatta tcattcatca 3360
aacagaaatt agaagctatt acatctttaa ctatacttgg acaaccacct agctatacac 3420

gagtcgggaa ctcaattaga gtaaggtgag ccatatgagc cgctaagttg gctgtctata 3480

atcatgaata aacctaacta aattaaaaga actagtaaga gatcaggtat ccttaacaac 3540

cattcctgca taagattcgt cacacttatt ttcattaatc atctttacta cctgcagtca 3600

atcactttca attataatat cccacgcata gcattggata gccattttca ttccgagcac 3660

tatagccttc ccttccgtca cattcgtttc cctcaagtgt ttcacttttt gacacgccat 3720

acgaatgata tgcccaccat gatcttgtat aacaattccc attccgattc ccacctcctt 3780

aaagatcgca gcatctacat tgatttccag cacattatag cttggacagc accacaccat 3840

gatagaacgc tgcccaccag tctgatctgc agcaggcaga ctcatgtgtc tgctgtcctt 3900

cccctctgta cgagccagtt caaaggcaga tggggctgta cacagtcctg tagggtgacg 3960

ggcaatgctg ccatgataaa cattgttccg tttattccaa atcatccatg ctattattgc 4020

aactttacaa cgctcctcct atataacatc catccaccat gcagcccagt cactagcaca 4080

tcattgggat tctagaatac tctccaaccc gcttcttccc acaggaatac agggtagttc 4140

taactcaacg caagggtgtt tgttacataa atctttataa ttcttgcata gcatttcctt 4200

atgtaaatga acatgctcaa aatgttttat taaactattt tgtttaacat ataatagaat 4260

ttcccttgaa gtaccagtga tgatttgcac agtatcagcg ggagcacaaa agttgcaaaa 4320

gcttagctat gatgatgcta taaacttaag agtatatact aggttattat ttataaacta 4380

aacttgagtc ttaaggtggg ttgaacttgg aattttaagc tcaaggacac tcgagctcaa 4440

attactacaa ggcacaagct gagtcgagat atcccaagtt taagctgtct ttaacacccc 4500

tacctatgca cgactgttgc tgtatactgt tttcttggtt tataaggagc ctagagggca 4560

atgatgcaca tggtttgaaa ctttgttctt gtgtttcagt gtatcactac attaaaaaaa 4620

tttcatctga gctagccgat gtatgctaat tataacctgt aataaataca gttatcactt 4680

acttttttct atctgaggtt ggtttgctag attatgactt tggactatca gtatcgcaat 4740

atacgtataa atgaaatcaa agactcattt ttatctcata attttctctt taaatttgta 4800

ttttcattca gcgtgtgtta cattggctta aaataattca ctttttattg acctcttctt 4860

tgattcactt ttattaacta gtaaactgta tctgatggac agaggttaca gaattatctg 4920

aagaaaagcc tctagaggaa aagcccatag aggagaagcc tttagagggc tcagttggta 4980

gaggtaatag tttcactcat cttgttgtaa acgagcttag taagtttgat ttcagcttat 5040

atctcattgt tgttgtattc agatgctcag ctagcacgag ttcaaactga gaagaggatt 5100

tcactgatca aagcatggga agaaagtgaa aaatgcaagg ccgaaaacaa gtaagagtac 5160

tttgtatgga tatgattaga tattagtgct acacccacag gctatacaaa tatatattat 5220

tatgtcattt agagatcaca tactcaaaat agcaatggga atcttaaggg tgcttgaacc 5280

atgactgaat tatttggctt aagttgtttt ttggtgctaa agaatacgag actcctagca 5340

agaaaagaag aatataataa gtcttaactc cacaattgca aggtcgttaa caatagaaga 5400

actcttgtga cttaatttta gttaaccaag cgataggtag gcctcatgcc aagttacaag 5460

atatttcaca catacgcgtg ataaatatag ctgtctagct tgctctgaac aataagcttg 5520

gcgaggttct taagtggttt gtcacattgc tcgtaccaac cagtatcact cttgtcacct 5580

gtcagtgcat caattaggtc tccctatata cttgctggac ctgtgataac cattctacac 5640

tctgtatcaa ttatttgtat tatacttgaa taggtctaaa ggccaacatt tgggttagtc 5700

tctaaaagtt gctggagtaa atttgtgcct atagttgaat ggttccgtgc ttaaaccttc 5760

cctttgaata ttgctaagag ttcagtctgt ggcacatcac atcggccata tatttagctg 5820

agttaagtgc tgatacaatt gcttaaccgc tccttcccct acccttcagc aactcttgga 5880

ctgcctcctc ctctgcatca gtgaatgtat gttgaatagt ttaaccctca tccactctaa 5940

ccaaaaacaa aatcaaattt gttatgtgat tttgatggtt gagaatttga gatacaaaat 6000

agccttttgc ctcctttacc ataacaacca ccgtattgtc aatttgttat agacccatat 6060

cgctcattag cctatatggg aaagtcttag atcaatttac tcattctgtt tccttttgtt 6120

cttgctccaa atatgtaggg agatacaaaa gaaagtgggt aatagaggag taagtgcata 6180

taatgatata attataagca aggcatgttt ggcatctatc tagaatctgg ttttgggttt 6240

taagattttt caagtcatga tatgaaatgt atgatggact taaaataagt catattcaaa 6300

gtaatcatgt ctatatcaaa atctgacaac agaactcgaa aatatttttt ctggttttga 6360

catttctatt taattggaaa cacaaatgat aacgaagaga cacactgttg atagaccctt 6420

taatttgatg gaagttacaa aacaaattta cttacttaat gaactgcgca gggcacacaa 6480

gaaaattgct gctatcgagg catgggagaa cagcaagaaa gcatctcttg aggcagagct 6540

gaaaaagatc gaggtatagt gccattaaac aactatcttt atctcgagtt attttcgaac 6600

aatagaagtt cgttgaactg atgaggccag cttacatgaa tgcaggaaag cttggaaaaa 6660

aagaaggcag aatatgcaga aaaaatgaag aacaaagtgg ctatgactca caaggaagca 6720

gaggagaaga aggctacggt tgaagccaaa cgtgctgaag atgttctgaa agcggaagag 6780

atggctgcta agtatcgcgc cactggaaag ccccccaaga aacttcttgg tttgttctaa 6840

taaggattat gtgaatttag taattacttc tataaatgtt tgatgtgatt aacaattaag 6900

caactcaatt agtttgtgaa aaggaaatta tagataagtt atttgtattt ttttttcatt 6960

tgtatgattg ttttatggct ttattggttt ggtaggtgtg tatagtttgg gtagaacgat 7020

ttaagaattg gtgcacattt gtttccatgt ggatattgtt tctgttatgt aagtaaattg 7080

gatattgttt ctattatgta agtaaattgg atattggacc acaacttgtg tctcatgtct 7140

atcatttcct attgtcttgt cagtcgtttg caaattcgca aatacaaagg tttaacggga 7200

aacctgttct cctctgaaca gatagttgga atcgtgtgat gcccactcca agcgcatata 7260

ctaaagttcc catcggagaa caattgtcca aatatcctaa attagctgaa aatcatctta 7320

gatatgaatt aaactttaga gcctccgaag ttatcatgtt gtgttatata tgtacgtaga 7380

agaattcatc cgctcctgtt agtgttcagc taatgaatca gtaagctgtt agaagaagtt 7440

atcatgttgt gttatataag gattacttaa gactgttttc ttactaatga ggtctaatat 7500

gtgacaaaga ttgtcaagat tatgtaaatc atgcctgtta gtataaaaca aactgcactg 7560

actaacttta ttcactggtt ggagaatcga gcaacatgtt cattgctgta tctgaagtat 7620

cacctgatgc aacagaccct gagtctgtcc tctgctgctg ctctgttttg acacctacgt 7680

gttgcggaac ttggcctgtt acccctgaac aagcctctga ggaaggtgaa acacgatcga 7740

ttttcaaggg ccgctggttt tggatcttcc taaaggactc tccgacctcg gaattccaca 7800

tagaaaagaa gtgatcagag ttggttctgg aataacccga ggggttgtag agggattcta 7860

atgacaaccg agcctcggtc tccagtcgga tactttccca ttggaccata tgacgggttg 7920

tatgtgactt ggattgtgta gtttctgagt catcgttgat ggtttttcgt ttcttcaagt 7980

ggctgttcca gtggttcttg acatcattgt ctgttcttcc tggtagcctt gctgctatag 8040

cagcccacct gtaaaaatag catgcataat acttaggaca gctaacctct gttttatcaa 8100

ccacggaatt tctagatttg aggtatcttg ctaccttgca tagaagaaat gataaacatc 8160

tatacaatgg atgcatctag caaagtgtct gtgatatagt gtttgtgttg tttttctctc 8220

cttttgctac ttcacaaacg cataaaagac aattgacggg agagttatac cttttatagt 8280

ctaaacgatt ttttttatct ttaaaactaa tttgacaacg ggaaaagtat catttaagtc 8340

tttaagataa taaagtacta gttgccttct ctccttttat taagcaccgg gtattattat 8400

atatgcacaa tataatgaat agtgtaaaag ttgtcacctt ttctaattgg tgatgactat 8460

aatttttaaa aaatatttca tagtttaggt aagagattat gaaatgataa aaagtttcct 8520

aactatttct aaagagtata tttttcatgt gatatatggt atttgtaatc acatagttag 8580

agataataaa acaattagag gaatacctat tgccaaggag tccatgaagt tgaataacag 8640

tctcgttttc ctcttcagag aaaggacctc gtctaatgtc aggtcttaaa taattggtcc 8700

atcgaagacg gcaactcttt ccacatcgag atatacctaa catataacat caatatgaaa 8760

ttttatcaca taaattaaca catagaagta ttatattcaa tttttcatga atgacatatt 8820

caagcttaat gagttaattc accttattgg aagtatagtc atatacataa tcaagacaac 8880

ctttcta 8887

<210> 10
<211> 4972
<212> DNA

<213>  Beta vulgaris

<400>  10
atggcaacag aggaagcaaa taaagtggtg actgaaacac cggcaccacc ggtaactccg      60

ccacaagaac cgccagcaac agtttcagaa acccgggaat caccggttgc ggtggaagag     120

aaaagtttag tccctgtatc aacccctctt ccttccacca ctgaaaaaac tcctgcttcg     180

gttgaaagtt agttaacaat ttatttttgt agtatatact ttacttatct ataattttaa     240

ttattatatg atgatcatta tattatagta tatggctaat tttgctttat tcaagaatat     300

ttagtgattt aagggctttt aattttatta aacttttctc aattcccctg ttctttttaa     360

tcattacttg aagtttaggt agagttcgtt tggttcacct caaattctat tggaattcgg     420

atttttttag gaagtacaag ataggatagt tgttttgtat gcacgaatta gggaattcat     480

ggtaaacaga atttccaagg aaatggaaat tctgatgtat aggaaattaa tcacaacctc     540

ttagtaatta gaaattccta tgtcaattac aaatttatat gctcaaccaa ataccaaaaa     600

tcattttctc ggatatagac aagagaattt acttcttatg aaatcagact tcccatgaga     660

attaagtttc aacggtaacc aaacgagctt ttagtttttt cccatgtgat ataaagatgg     720

gtcatgggtg gtagttttcc aattatatat ttttttggtg gatgggtgtt tttatttttc     780

cttaatttgg tatattttct gcatattatc catgtttgtt aaggagatta cttgcttatt     840

ttaagaatat catgagtgtt tatttgggaa tatcagggac ttatctgagg gctttttctg     900

gatcaggagg tttgaattaa agtggttggg caactttata tcctttaact tgggcacttg     960

tatagtatat tcaaaactat ttttttcatt cattgctcat ttaaatgatt tcaaatgagc    1020

aaattaacaa ctataattga agaaattagc ctaagctcat ggtacgcctg agtatatggt    1080

gtagattttg attatcttag actttcttgc gcgttacgca gtgattaaag agtaaaggca    1140

attccagcta aatagctaat tttggttatg ctaagttttt tgatactatt tgattctttg    1200

tttctttgtc ctttgatgat aaaacctatg taagcaaagt gcactattca tgaatcatga    1260

tactggccat tagctattat ctatgttact tagacacacg gattgggtgt ctactcggat    1320

gtgggtgtcc actgtccaag tgtctgactc ggctattttg gaaaaaagtc taagtttccg    1380

gtttaaaatg aagtatacaa gtaaagtgtc gtgtcggaca cgacacgaca cgacaccccg    1440

tttttgaggt aaatgaaggt gtcggagtaa cacaagctat tatcattcat caaacagaaa    1500

ttagaagcta ttacatcttt aactatactt ggacaaccac ctagctatac acgagtcggg    1560

aactcaatta gagtaaggtg agccatatga gccgctaagt tggctgtcta taatcatgaa    1620

taaacctaac taaattaaaa gaactagtaa gagatcaggt atccttaaca accattcctg    1680

cataagattc gtcacactta ttttcattaa tcatctttac tacctgcagt caatcacttt    1740

caattataat atcccacgca tagcattgga tagccatttt cattccgagc actatagcct    1800

tcccttccgt cacattcgtt tccctcaagt gtttcacttt ttgacacgcc atacgaatga 1860

tatgcccacc atgatcttgt ataacaattc ccattccgat tcccacctcc ttaaagatcg 1920

cagcatctac attgatttcc agcacattat agcttggaca gcaccacacc atgatagaac 1980

gctgcccacc agtctgatct gcagcaggca gactcatgtg tctgctgtcc ttcccctctg 2040

tacgagccag ttcaaaggca gatggggctg tacacagtcc tgtagggtga cgggcaatgc 2100

tgccatgata aacattgttc cgtttattcc aaatcatcca tgctattatt gcaactttac 2160

aacgctcctc ctatataaca tccatccacc atgcagccca gtcactagca catcattggg 2220

attctagaat actctccaac ccgcttcttc ccacaggaat acagggtagt tctaactcaa 2280

cgcaagggtg tttgttacat aaatctttat aattcttgca tagcatttcc ttatgtaaat 2340

gaacatgctc aaaatgtttt attaaactat tttgtttaac atataataga atttcccttg 2400

aagtaccagt gatgatttgc acagtatcag cgggagcaca aaagttgcaa aagcttagct 2460

atgatgatgc tataaactta agagtatata ctaggttatt atttataaac taaacttgag 2520

tcttaaggtg ggttgaactt ggaattttaa gctcaaggac actcgagctc aaattactac 2580

aaggcacaag ctgagtcgag atatcccaag tttaagctgt ctttaacacc cctacctatg 2640

cacgactgtt gctgtatact gttttcttgg tttataagga gcctagaggg caatgatgca 2700

catggtttga aactttgttc ttgtgtttca gtgtatcact acattaaaaa aatttcatct 2760

gagctagccg atgtatgcta attataacct gtaataaata cagttatcac ttactttttt 2820

ctatctgagg ttggtttgct agattatgac tttggactat cagtatcgca atatacgtat 2880

aaatgaaatc aaagactcat ttttatctca taattttctc tttaaatttg tattttcatt 2940

cagcgtgtgt tacattggct taaaataatt cactttttat tgacctcttc tttgattcac 3000

ttttattaac tagtaaactg tatctgatgg acagaggtta cagaattatc tgaagaaaag 3060

cctctagagg aaaagcccat agaggagaag cctttagagg gctcagttgg tagaggtaat 3120

agtttcactc atcttgttgt aaacgagctt agtaagtttg atttcagctt atatctcatt 3180

gttgttgtat tcagatgctc agctagcacg agttcaaact gagaagagga tttcactgat 3240

caaagcatgg gaagaaagtg aaaaatgcaa ggccgaaaac aagtaagagt actttgtatg 3300

gatatgatta gatattagtg ctacacccac aggctataca aatatatatt attatgtcat 3360

ttagagatca catactcaaa atagcaatgg gaatcttaag ggtgcttgaa ccatgactga 3420

attatttggc ttaagttgtt ttttggtgct aaagaatacg agactcctag caagaaaaga 3480

agaatataat aagtcttaac tccacaattg caaggtcgtt aacaatagaa gaactcttgt 3540

gacttaattt tagttaacca agcgataggt aggcctcatg ccaagttaca agatatttca 3600

cacatacgcg tgataaatat agctgtctag cttgctctga acaataagct tggcgaggtt 3660

cttaagtggt ttgtcacatt gctcgtacca accagtatca ctcttgtcac ctgtcagtgc 3720

atcaattagg tctccctata tacttgctgg acctgtgata accattctac actctgtatc 3780

aattatttgt attatacttg aataggtcta aaggccaaca tttgggttag tctctaaaag 3840

ttgctggagt aaatttgtgc ctatagttga atggttccgt gcttaaacct tccctttgaa 3900

tattgctaag agttcagtct gtggcacatc acatcggcca tatatttagc tgagttaagt 3960

gctgatacaa ttgcttaacc gctccttccc ctacccttca gcaactcttg gactgcctcc 4020

tcctctgcat cagtgaatgt atgttgaata gtttaaccct catccactct aaccaaaaac 4080

aaaatcaaat ttgttatgtg attttgatgg ttgagaattt gagatacaaa atagcctttt 4140

gcctccttta ccataacaac caccgtattg tcaatttgtt atagacccat atcgctcatt 4200

agcctatatg ggaaagtctt agatcaattt actcattctg tttccttttg ttcttgctcc 4260

aaatatgtag ggagatacaa aagaaagtgg gtaatagagg agtaagtgca tataatgata 4320

taattataag caaggcatgt ttggcatcta tctagaatct ggttttgggt tttaagattt 4380

ttcaagtcat gatatgaaat gtatgatgga cttaaaataa gtcatattca aagtaatcat 4440

gtctatatca aaatctgaca acagaactcg aaaatatttt ttctggtttt gacatttcta 4500

tttaattgga aacacaaatg ataacgaaga gacacactgt tgatagaccc tttaatttga 4560

tggaagttac aaaacaaatt tacttactta atgaactgcg cagggcacac aagaaaattg 4620

ctgctatcga ggcatgggag aacagcaaga aagcatctct tgaggcagag ctgaaaaaga 4680

tcgaggtata gtgccattaa acaactatct ttatctcgag ttattttcga acaatagaag 4740

ttcgttgaac tgatgaggcc agcttacatg aatgcaggaa agcttggaaa aaaagaaggc 4800

agaatatgca gaaaaaatga agaacaaagt ggctatgact cacaaggaag cagaggagaa 4860

gaaggctacg gttgaagcca aacgtgctga agatgttctg aaagcggaag agatggctgc 4920

taagtatcgc gccactggaa agccccccaa gaaacttctt ggtttgttct aa 4972

<210> 11
<211> 633
<212> DNA
<213> Artificial Sequence

<220>
<223> cDNA of remorin gene - haplotype 3

<400> 11
atggcaacag aggaagcaaa taaagtggtg actgaaacac cggcaccacc ggtaactccg 60

ccacaagaac cgccagcaac agtttcagaa acccgggaat caccggttgc ggtggaagag 120

aaaagtttag tccctgtatc aacccctctt ccttccacca ctgaaaaaac tcctgcttcg 180

gttgaaaagg ttacagaatt atctgaagaa aagcctctag aggaaaagcc catagaggag 240

aagcctttag agggctcagt tggtagagat gctcagctag cacgagttca aactgagaag 300

aggatttcac tgatcaaagc atgggaagaa agtgaaaaat gcaaggccga aaacaaggca 360

cacaagaaaa ttgctgctat cgaggcatgg gagaacagca agaaagcatc tcttgaggca 420

gagctgaaaa agatcgagga aagcttggaa aaaaagaagg cagaatatgc agaaaaaatg 480

aagaacaaag tggctatgac tcacaaggaa gcagaggaga agaaggctac ggttgaagcc 540

aaacgtgctg aagatgttct gaaagcggaa gagatggctg ctaagtatcg cgccactgga 600

aagcccccca agaaacttct tggtttgttc taa 633

<210> 12
<211> 210
<212> PRT
<213> Beta vulgaris

<400> 12

Met Ala Thr Glu Glu Ala Asn Lys Val Val Thr Glu Thr Pro Ala Pro
1               5                   10                  15

Pro Val Thr Pro Pro Gln Glu Pro Pro Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Pro Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Val Leu Lys Ala Glu Glu Met
            180                 185                 190

Ala Ala Lys Tyr Arg Ala Thr Gly Lys Pro Pro Lys Lys Leu Leu Gly
        195                 200                 205

Leu Phe
    210

<210>  13
<211>  9805
<212>  DNA
<213>  Beta vulgaris

<400>  13
atagatgacg tggtttaact tttgtactat ttacactcac tcctttgatc atgttttatg      60

atttttacgt ttgaaaaata tatttatgtc ggatattatt agattcatct tgatatatat     120

tttcaaaata ttaacttttt ataattatta tatgtaggga gttagatata ttaatagtta     180

agtttctgcg ttggcaagtg cgtccggaat tgtgtctcaa gtatgttgaa acgaaggaag     240

tatatatgtc attacaaagg tcgcgatgta taaaaaagtg attgcattta catatgggtc     300

actatgtgtt ccacgtacaa tatttgacat gcataacctt ttaatccaca ataacattat     360

tacaatttgc aaaccaggtc aacataaatc ttactctaaa ctattaaaca tggggaaaat     420

caagtaatta acatattcct tttaacttca ccccaaaaga taaacatttt tcttagtggt     480

cgtttggtaa ataggaaatt atgtaggcaa tagaaattcc tatgattttc atatttgtat     540

agttgtttag ttgacagaaa gtgaggaaaa cgggggtaat gcaaattcat gagaatttga     600

aattcccaca aaatatacga atttatttac ctactcttcc cttggtcact cacaattgct     660

acataaattc ttatgaatta caactttcct actaccaaac aatttaaact aatttatagg     720

aatttcataa aggaattaat tttctacaaa ttgtaaattc ctatagaaat ttaattccca     780

ttgcaaccaa acaaacacta acactccgag agaaccaaca atccaactcc cgatcctaaa     840

accataacta caagctattt ttactactct attcctttta atataatcat gattcattat     900

caccagcctt tacaaattac aatattagta acagtggatt attttccaat aaaaaagaat     960

gattaatgtg tgattttgtt ataagttctt aaaactaata tggtttatgc tcgtgtgcaa    1020

tcattatcct atttttgcaa caaaatgcta agagcttttg ggttcaatgg tatcacttcc    1080

tattttctat atggaaaatt aaggatcaaa atttggtgga agcataattt caatgttgtc    1140

tggggtgggg attggggtac ttgttggata tctgggtgag actcttgtgc aagaacaacc    1200

caaaagtaaa ttcttcttac catgtggttt ccgaaaggag gcccaaaaaa atcaacaaaa    1260

taacaaaaaa caaagatcaa tgacaactac tttttatttt tggtgtaaag gaggatcaac    1320

gtaataatct tgtcgagttt gatcccaagt cttaactctt aagtattatt ccacgagata 1380

ttactaacta ggactgttct ctaattctct tcaccattaa aaaaaaaatc caagccaaat 1440

aagttcaaat aatatctaac taaaactaat actccctccg tcccataata taagtcctga 1500

ttatgttttt acgcgggaat taaggtaaag tagaatcttc ttttatttaa ataaaaataa 1560

tcatttttat tgggaaacac ttgcattgtg tttttctttt taataaaata taaaaactga 1620

cattaatatt gtatggttta atgtgtagag tagttcaatg gggtaaaaga ttccattttg 1680

tcattaatga tggtgcattg aaagctgaaa ttatgaggac attttgggaa tgaaagggga 1740

cttatattat gggacggagg gagtacaata cttaaaaata agtttcgata agattgaatc 1800

aggtctaatt aattcaattc aattcaaata agttcggatt ttttgagatg aaaacaacgc 1860

taccttggca tccacaagaa tgacaactat gaaaaggtca ttttggctct attatttttg 1920

gtttgattgc ttgcaccaac tctcaactct ctctctagtc tctcctcgtt cttattcatt 1980

ccagctaatg aagctgctat cgttatcata aacttccctc taactccttt ctaacttccc 2040

ttccactact cttcttcatt ttctctctcc tccctccttt cttttcttcc tcttttttta 2100

ggaacttcac aataaaaaaa acttaaatat acttggaagt acttttgatt gtaatacata 2160

tggcaacaga ggaagcaaat aaagtggtga ttgaaacacc ggcaccaccg gtaactccgc 2220

cgcaagaacc gccggcaaca gtttcggaaa cccgggaatc accagttgcg gtggaagaga 2280

aaagtttagt ccctgtatca acccctcttc cttccaccac tgaaaaaact cctgcttcgg 2340

ttgaaagtta gttaacaatt tatttttgta gtatatactt tacttatcta taattttaat 2400

tattatatga tgatcattat attatagtat atggctaatt ttgctttatt caagaatatt 2460

tagtgattta agggctttaa attttattaa acttttctca attcccctgt tctttttaat 2520

cattacttga agtttaggta gagttcgttt ggttcacctc aaattctatt ggaattcgga 2580

ttttttagga agtacaaaat aggatagttg ttttgtatgc acgaattagg gaattcatgg 2640

taaacagaat ttccaaggaa atggaaattc tgatgtatag gaaattaatc acaacctctt 2700

agtaatttga aattcctatg tcagttacaa atttatatgc tcaaccaaat accaaaaatc 2760

attttctcgg atatagacaa gagaatttac ttcttatgaa atcagacttc ccatgagaat 2820

taagtttcaa cggtaaccaa acgagctttt agtttttacc catgtgatat aaagatgggt 2880

catgggtggt agttttccaa ttatatattt ttactatttt tggtggatgg gtgttcttat 2940

ttttccttaa tttgttattt atttctgctt attatccatg tttgttaagg agattacttg 3000

cttattttaa gaatatcatg agtgttattt gggaataata gggacttatc tgagggcttt 3060

ttctggatca tgaggtttga agtaaagtgg ttgggcaact ttatatcctt taacttgggc 3120

acttttatag tatattcaaa actatttttt cgctcattgc tcatttaaat gatttcaaat 3180

gagcaaatta acaactataa ttgaagaaat tagcctaagc tcatggtacg cctgaatgta 3240

tggtgtagat tttgattatc ttagactttc ttgtgcgtta cagagtgatt aaagagtaaa 3300

ggcaaagtgc taattttggt tatactaagt ttttaatact atttgatcct ttgttacttt 3360

gtcctttgat gataaaacct atgtaagcaa agtgcactat tcatgaatca tgatactggc 3420

cattagctat tatctatgtt acttagacac acagattggg tgtctactcg gatgtgggtg 3480

tccaaagtgt ctgactcggc tattttggaa aaaagtctaa gtttccggtt taaaatgaag 3540

tatacaatta aagtgtcatg tcggacacga caccccgttt ttgaggtaaa tgaaggtgtc 3600

ggagtaacac aggctattat cattcatcaa acagaaatta gaagctatta catctttaac 3660

tatacttgga caaccaccta gctatacacg agtcgggaac tcaattagag taaggtgagc 3720

catatgagcc gctaagttgg ctgtctataa tcatgaataa acctaactaa attaaaagaa 3780

ctagtaagag atcaggtatc cttaacaacc atatctgcat aagattcatc acacttgttt 3840

tcattaatca tctttactac ctgcaggcaa ttactttcaa ttataatatc ccacgcatag 3900

cattgggtag ccattttcat tccgagcact atagccttcc cttctgtcac attcgtttcc 3960

cacaagtgtt tcactttttg acacgccata tgaatgatat gcccaccatg atctcgtata 4020

acaattccca ttccgattcc cacctcctta aagatcgcag cgtctacatt gattttcagc 4080

acattatagc ttggacagca ccacaccaca ctagagtgct gcccaccagt ctgatctgca 4140

gcaggcagac tcatgtgtat gctgtccttc ccctctgtac cagccagttc aaaggcagct 4200

ggtgctgtac acagttctgg aaggtgacgg gcaatgctgc catgataaac attgttccgt 4260

tcattccaaa tcatccatgc cagtattgca actttacaac tctcctccta taaaacatcc 4320

atccaccatg caacccagtc actagcgcat catattggga ttccagaata ctctccaacc 4380

cgcctcttcc caaaggatac tgggtatttc ttaatcacta tttctaaatc aacgcaaggg 4440

tgtttgttaa ataaatcttt acaattcttg catagcattt ccttatgtaa atgaacatgc 4500

tcaaaatgtt ttatcaaact atttttgtta aacatataat agaatttccc ttgaagtacc 4560

agtgatgtgc acagtatcag cgggagctca aaagttgcaa aagcttagct atgatgatgc 4620

tataaactta agagtatata ccaggttatt atttataaac taaagttgag tcttaaggtg 4680

ggttgaactt ggaattttgt gctcaaagac actcgagctc aaattactac aaggcacaag 4740

ctgagttgtg atatcccaag tttaagctgt ctttaacacc cctacctata acctatgcac 4800

gactgttgct atttactgtt ttcttggttc ataaggagcc tagagggcaa tgatgcacat 4860

ggttggaaac tttgttcttg tgtttcagtg tatcactaca ttaaaaaaat ttcatccgag 4920

ctagccaatg tatgctcatt atatcttgta ataaatacag ctatcactta cttttttcta 4980

tctgaggttg tttgctagat tatgactttg gactagcagt atcgcaaaat gcgtataaat 5040

gaaatcaaag actcattttt atctcataat tttctcttta aatttgtatt ttcattcagc 5100

gtgtgttaat tggcttcaaa tttaaaatta ttcacttttt attgacctct tttttttaga 5160

ggattcactt ttattaacta gtacactgta tctgatggac agaggttaca gaattatctg 5220

aagaaaagcc tctagaggaa aagcctatag aggagaagcc tttagagggc tcagttggta 5280

gaggtaatag tttcactcat cttgttgtaa acgagcttaa taagttcagt ttcagcttat 5340

atcttattgt tgttgtattc agatgctcag ctagcacgag ttcaaactga gaagagaatt 5400

tcactgatca aagcatggga agaaagtgaa aaatgcaagg ccgaaaacaa gtaagagtac 5460

tttgtatgga tatgattaga tattagtact acacccacag gctatacaaa tatacattat 5520

catgtcattt agagatcaca tactcaaaat agcaatggga atctcagggg tgcttgaacc 5580

atgagtgaat tatttggctt aagttgtttt ttggtgctaa agaatatgag actcctagca 5640

agaaaagaag aatctaataa gtcttaactc caccattgca aggtcgttaa caatagaaaa 5700

actcttgtga cttaatttaa gttaaccaag cgataggtag gcctcatgcc aagttacaag 5760

atttttcaca catacgcgtg acaaatatag ctgtctagct tgctcttaac aataagcttg 5820

gcgaggttct taagtggttt gtcacattgc tcgtaccaac cagtatcact cttgtcacct 5880

gtcaatgcat caattagctc tccctatata cttgctggac ctgggataac cattatatac 5940

tctgtattaa ttctttgtat tatacatgaa taggtctaaa ggccaacatt tgggttagtc 6000

tctagaagtt gctggagtaa atttgtgcct atagttgaat ggttccgtgc ttaaaccttc 6060

ctttgaatat cgctaagagt tcagtctttg gagcatcaca tcgaccatat atttagctga 6120

gtaagcgctg atacaattgc ttaagcgctc cttcccctac tgttcagcaa ctcttggact 6180

gcctcatcct ttgcatcagt gaacgtatgt tgagtagttt aatcctttcc atcattccat 6240

gactccttta accctcatcc actctagcca aaaacaaaat caaatttgtt gtgtgatttt 6300

gatggttgag aatttgagat acaaaatagc cttttgcctc ctttaccata accaccgtat 6360

tgtcaatttg ttataagacc catatgactc attagcctat atgggaaagt cttagatcaa 6420

tttactcatt ctgtttccat ttgttcttgc ttcaaattag aggtggttat tggacagggt 6480

agtccaatgg ttaaagggtt agggtcaatt ttacagggct tttattggtc agggttttat 6540

tggacagggt cattattgga tagggtcatt atagggtcaa caaagaaacc caataatttt 6600

aaaaaataaa attagttttg taaaaacaat tacatattcg gattcgcatt catttatact 6660

tacttttggc tctttttgtt ccttgttttt cttgctcgtt taccgaccca cccattatcg 6720

acccgctata agccctctac ccatttggcc cgacctgcta ccgacccttt atgaggcgac 6780

cctcttatga cccaaacccg cttccgaccc gtactgaccc tgacccgacc cgttcaataa 6840

ccaggtctac ttcaaatatg taggaagata caaaagaaag tgggtaataa agaagaaaga 6900

gtaagtgcat ataatgatat aattataagc aaggcatgtt tggcatctat ctagaatctg 6960

gttttgggtt ttaagatttt tcaagtcata tgatgtgaaa tgtatgatgg acttaaatta 7020

agccatattc aaagtaatca tgtctatatc gaaatctgac aacagaactc gaaaatattt 7080

tttctggttt ggacatttct atttaattgg aaacacaaat gataacgaag agacactcta 7140

ttgatagacc ctttaatttg atggaagtta cacaaattta cttgctaaaa attgcgcagg 7200

gcacacaaga aaattgctgc tatcgaggca tgggagaaca gcaagaaagc atctcttgag 7260

gcagagctga aaaagatcga ggtatagtgc cattaaacag ctatctttat ctcaagttat 7320

tttcgaacaa tagaagtttg ttgagctgat gaggccagct tacatgagtg caggaaagct 7380

tggaaaagaa gaaggcagaa tatgcagaaa aaatgaagaa caaagtggct atgactcaca 7440

aggaagcaga ggagaagaag gctacggttg aagccaaacg tgctgaagat gttctgaaag 7500

cggaagagat ggctgctaag tatcgcgcca ctggaaagcc ccccaagaaa cttcttggtt 7560

tgttctaata aggattatgt gaatttagta attacttcta taaatgtttg atgtgattaa 7620

caattaagca agtcaattag tttgtgataa ggaaattata gataagttat ttgtattttt 7680

tttccatttg tataattgtt ttatggcttt attggtttgg taggtgtgta tagtttgggt 7740

agaacgatat aagaattggt gcacatttgt ttccatgtgg atattgtttc tgttatgtaa 7800

gtaaattgga tattgtttct attatgtaag taaattggat attggaccac aacttgtgtc 7860

tgatgtctat catttcctat tgtcttatca gtcgtttgca aattcgcaaa tacaaaggtt 7920

taacgggaaa cctgttctcc tctaaacaga tagctggaat cgtgtgatgc ccactccaag 7980

tgcatatact aaagttcccc tcggagaaca gttgtccaaa tgtcctaaat taggtgaaaa 8040

gttatcacgt tatgttatat atgtacgtag aagaattcat ccgctcctgt tagtgttcag 8100

ctaataaatc agtaagctgt tagttctgca taatgtacca gtactactac aaggattact 8160

taagactgtt ttcttactaa tgaggtctaa tatgtgacaa agattgtcaa gattatgtaa 8220

atcatgcctg ttagtataaa acaaactgca ctgactaatt ttattcactg gttggagaat 8280

cgagcaacat cttcattgct gtatctgaag tgtcacctga tgcaacagac cctgagtctg 8340

tcctctgctg ctgctctgtt ttgacaccta cgtgttgcgg aacttggcct gttacccctg 8400

aacaagcctc tgaggaaggt gaaacacgat cgattttcaa gggccgcgca ttttggatct 8460

tcctaaagga ctctccgacc tcggaattcc acatagaaaa gaagtgatca gagttggttc 8520

tggaatatcc tgaggggttg tagagggatt ctaatgacaa tcgagcctcg gtctccaatc 8580

ggatactttc ccattggacc atatgacggg ttgtatgtga ctttgatggt gtagtttctg 8640

ggtaaacgtt gatggttttt cgtttcttca agtggctgtt ccagtggttc ttgacatcat 8700

tgtctgttct tcctggtagc cttgctgcta tagcagccca cctgtaaaaa tagcatgcat 8760

aatacttagg acagctaacc tctgctttat caaccacgta atttctggat ttgaggtatc 8820

ttgctacctt gcatacaaga aatgataaac atctatacaa tggatgcatc tagcaaagtg 8880

tctgtgatat agtgtttgtg ttgtttttct ctccttttgc tacttcacaa acgcataaaa 8940

gacaattgac ggaagagtta taccttttat agtctaaacg agtttttatc tttaaaacta 9000

atatggcaac gggaaaagta tcatttaagt ctttaagata ataaagtact agttgtcttc 9060

tctcctttta ttaagcactg ggtattatta tatatgcaca atataatgag atttagcgta 9120

aaagttgtca ctttttttaa ttggcgatga ctataatttt tttaaaatat tttatagttt 9180

aggtaagaga ttatgaaatg ataaaaagtt tcctaactat ttctaaagag tatatttttc 9240

atgtgatata tggtatttgt aatcacataa ttagggataa taaaacaatt agaaaaatac 9300

ctattgccaa ggagtccatg aagttgaata acagactcgt tttcctcttc agagaaagga 9360

cctcgtctaa tgtcaggtct taaataattg gtccatcgaa gacggcaact ctttccacat 9420

cgagatatac ctaacatata tcatcaatat gaaactttat cacataaatt aacacataga 9480

agtattatat tcaatttttc atgaatgaca tattcaagct taatgagtta attcacctta 9540

ttggaagtat agtcatatac ataatcaaga caacctttct actttatagg tagcaagtaa 9600

attgtaggac tttgaggtgg gctgggttag gtccaagcaa gaagaaagta cttccgaaaa 9660

actggtaaag tatttttctg taagtattac tacacaagaa gcaccagtgg tctagtggta 9720

gaatagtacc ctgccacggt acagacccgg gttcgattcc cggctggtgc ataaattatc 9780

agagctgtga tgataatatt gcgcc 9805

<210> 14
<211> 5409
<212> DNA
<213> Beta vulgaris

<400> 14
   atggcaacag aggaagcaaa taaagtggtg attgaaacac cggcaccacc ggtaactccg 60
   ccgcaagaac cgccggcaac agtttcggaa acccgggaat caccagttgc ggtggaagag 120
   aaaagtttag tccctgtatc aacccctctt ccttccacca ctgaaaaaac tcctgcttcg 180
   gttgaaagtt agttaacaat ttatttttgt agtatatact ttacttatct ataattttaa 240
   ttattatatg atgatcatta tattatagta tatggctaat tttgctttat tcaagaatat 300
   ttagtgattt aagggcttta aattttatta aacttttctc aattcccctg ttctttttaa 360
   tcattacttg aagtttaggt agagttcgtt tggttcacct caaattctat tggaattcgg 420
   attttttagg aagtacaaaa taggatagtt gttttgtatg cacgaattag ggaattcatg 480
   gtaaacagaa tttccaagga aatggaaatt ctgatgtata ggaaattaat cacaacctct 540
   tagtaatttg aaattcctat gtcagttaca aatttatatg ctcaaccaaa taccaaaaat 600
   cattttctcg gatatagaca agagaattta cttcttatga aatcagactt cccatgagaa 660
   ttaagtttca acggtaacca aacgagcttt tagtttttac ccatgtgata taaagatggg 720
   tcatgggtgg tagttttcca attatatatt tttactattt ttggtggatg ggtgttctta 780
   tttttcctta atttgttatt tatttctgct tattatccat gtttgttaag gagattactt 840
   gcttatttta agaatatcat gagtgttatt tgggaataat agggacttat ctgagggctt 900
   tttctggatc atgaggtttg aagtaaagtg gttgggcaac tttatatcct ttaacttggg 960
   cacttttata gtatattcaa aactattttt tcgctcattg ctcatttaaa tgatttcaaa 1020
   tgagcaaatt aacaactata attgaagaaa ttagcctaag ctcatggtac gcctgaatgt 1080
   atggtgtaga ttttgattat cttagacttt cttgtgcgtt acagagtgat taaagagtaa 1140
   aggcaaagtg ctaatttggg ttatactaag tttttaatac tatttgatcc tttgttactt 1200
   tgtcctttga tgataaaacc tatgtaagca aagtgcacta ttcatgaatc atgatactgg 1260
   ccattagcta ttatctatgt tacttagaca cacagattgg gtgtctactc ggatgtgggt 1320
   gtccaaagtg tctgactcgg ctattttgga aaaaagtcta agtttccggt ttaaaatgaa 1380
   gtatacaatt aaagtgtcat gtcggacacg acaccccgtt tttgaggtaa atgaaggtgt 1440
   cggagtaaca caggctatta tcattcatca aacagaaatt agaagctatt acatctttaa 1500
   ctatacttgg acaaccacct agctatacac gagtcgggaa ctcaattaga gtaaggtgag 1560
   ccatatgagc cgctaagttg gctgtctata atcatgaata aacctaacta aattaaaaga 1620
   actagtaaga gatcaggtat ccttaacaac catatctgca taagattcat cacacttgtt 1680
   ttcattaatc atctttacta cctgcaggca attactttca attataatat cccacgcata 1740
   gcattgggta gccattttca ttccgagcac tatagccttc ccttctgtca cattcgtttc 1800
   ccacaagtgt ttcacttttt gacacgccat atgaatgata tgcccaccat gatctcgtat 1860
   aacaattccc attccgattc ccacctcctt aaagatcgca gcgtctacat tgattttcag 1920
   cacattatag cttggacagc accacaccac actagagtgc tgcccaccag tctgatctgc 1980
   agcaggcaga ctcatgtgta tgctgtcctt cccctctgta ccagccagtt caaaggcagc 2040
   tggtgctgta cacagttctg gaaggtgacg ggcaatgctg ccatgataaa cattgttccg 2100
   ttcattccaa atcatccatg ccagtattgc aactttacaa ctctcctcct ataaaacatc 2160
   catccaccat gcaacccagt cactagcgca tcatattggg attccagaat actctccaac 2220
   ccgcctcttc ccaaaggata ctgggtattt cttaatcact atttctaaat caacgcaagg 2280
   gtgtttgtta aataaatctt tacaattctt gcatagcatt tccttatgta aatgaacatg 2340
   ctcaaaatgt tttatcaaac tatttttgtt aaacatataa tagaatttcc cttgaagtac 2400
   cagtgatgtg cacagtatca gcgggagctc aaaagttgca aaagcttagc tatgatgatg 2460
   ctataaactt aagagtatat accaggttat tatttataaa ctaaagttga gtcttaaggt 2520
   gggttgaact tggaattttg tgctcaaaga cactcgagct caaattacta caaggcacaa 2580
   gctgagttgt gatatcccaa gtttaagctg tctttaacac ccctacctat aacctatgca 2640
   cgactgttgc tatttactgt tttcttggtt cataaggagc ctagagggca atgatgcaca 2700
   tggttggaaa ctttgttctt gtgtttcagt gtatcactac attaaaaaaa tttcatccga 2760
   gctagccaat gtatgctcat tatatcttgt aataaataca gctatcactt acttttttct 2820
   atctgaggtt gtttgctaga ttatgacttt ggactagcag tatcgcaaaa tgcgtataaa 2880
   tgaaatcaaa gactcatttt tatctcataa ttttctcttt aaatttgtat tttcattcag 2940
   cgtgtgttaa ttggcttcaa atttaaaatt attcactttt tattgacctc ttttttttag 3000
   aggattcact tttattaact agtacactgt atctgatgga cagaggttac agaattatct 3060
   gaagaaaagc ctctagagga aaagcctata gaggagaagc ctttagaggg ctcagttggt 3120
   agaggtaata gtttcactca tcttgttgta aacgagctta ataagttcag tttcagctta 3180
   tatcttattg ttgttgtatt cagatgctca gctagcacga gttcaaactg agaagagaat 3240
   ttcactgatc aaagcatggg aagaaagtga aaaatgcaag gccgaaaaca agtaagagta 3300
   ctttgtatgg atatgattag atattagtac tacacccaca ggctatacaa atatacatta 3360
   tcatgtcatt tagagatcac atactcaaaa tagcaatggg aatctcaggg gtgcttgaac 3420
   catgagtgaa ttatttggct taagttgttt tttggtgcta aagaatatga gactcctagc 3480
   aagaaaagaa gaatctaata agtcttaact ccaccattgc aaggtcgtta acaatagaaa 3540
   aactcttgtg acttaattta agttaaccaa gcgataggta ggcctcatgc caagttacaa 3600
   gatttttcac acatacgcgt gacaaatata gctgtctagc ttgctcttaa caataagctt 3660
   ggcgaggttc ttaagtggtt tgtcacattg ctcgtaccaa ccagtatcac tcttgtcacc 3720
   tgtcaatgca tcaattagct ctccctatat acttgctgga cctgggataa ccattatata 3780
   ctctgtatta attctttgta ttatacatga ataggtctaa aggccaacat ttgggttagt 3840
   ctctagaagt tgctggagta aatttgtgcc tatagttgaa tggttccgtg cttaaacctt 3900
   cctttgaata tcgctaagag ttcagtcttt ggagcatcac atcgaccata tatttagctg 3960
   agtaagcgct gatacaattg cttaagcgct ccttccccta ctgttcagca actcttggac 4020
   tgcctcatcc tttgcatcag tgaacgtatg ttgagtagtt taatcctttc catcattcca 4080
   tgactccttt aaccctcatc cactctagcc aaaaacaaaa tcaaatttgt tgtgtgattt 4140
   tgatggttga gaatttgaga tacaaaatag ccttttgcct cctttaccat aaccaccgta 4200
   ttgtcaattt gttataagac ccatatgact cattagccta tatgggaaag tcttagatca 4260
   atttactcat tctgtttcca tttgttcttg cttcaaatta gaggtggtta ttggacaggg 4320
   tagtccaatg gttaaagggt tagggtcaat tttacagggc ttttattggt cagggtttta 4380
   ttggacaggg tcattattgg atagggtcat tatagggtca acaaagaaac ccaataattt 4440
   taaaaaataa aattagtttt gtaaaaacaa ttacatattc ggattcgcat tcatttatac 4500
   ttacttttgg ctctttttgt tccttgtttt tcttgctcgt ttaccgaccc acccattatc 4560
   gacccgctat aagccctcta cccatttggc ccgacctgct accgaccctt tatgaggcga 4620
   ccctcttatg acccaaaccc gcttccgacc cgtactgacc ctgacccgac ccgttcaata 4680
   accaggtcta cttcaaatat gtaggaagat acaaaagaaa gtgggtaata aagaagaaag 4740
   agtaagtgca tataatgata taattataag caaggcatgt ttggcatcta tctagaatct 4800
   ggttttgggt tttaagattt ttcaagtcat atgatgtgaa atgtatgatg gacttaaatt 4860
   aagccatatt caaagtaatc atgtctatat cgaaatctga caacagaact cgaaaatatt 4920
   ttttctggtt tggacatttc tatttaattg gaaacacaaa tgataacgaa gagacactct 4980
   attgatagac cctttaattt gatggaagtt acacaaattt acttgctaaa aattgcgcag 5040
   ggcacacaag aaaattgctg ctatcgaggc atgggagaac agcaagaaag catctcttga 5100
   ggcagagctg aaaaagatcg aggtatagtg ccattaaaca gctatcttta tctcaagtta 5160
   ttttcgaaca atagaagttt gttgagctga tgaggccagc ttacatgagt gcaggaaagc 5220
   ttggaaaaga agaaggcaga atatgcagaa aaaatgaaga acaaagtggc tatgactcac 5280
   aaggaagcag aggagaagaa ggctacggtt gaagccaaac gtgctgaaga tgttctgaaa 5340
   gcggaagaga tggctgctaa gtatcgcgcc actggaaagc cccccaagaa acttcttggt 5400
   ttgttctaa 5409

<210> 15
<211> 633
<212> DNA
<213> Artificial Sequence

<220>
<223> cDNA of remorin gene - haplotype 4

<400> 15
atggcaacag aggaagcaaa taaagtggtg attgaaacac cggcaccacc ggtaactccg 60

ccgcaagaac cgccggcaac agtttcggaa acccgggaat caccagttgc ggtggaagag 120

aaaagtttag tccctgtatc aacccctctt ccttccacca ctgaaaaaac tcctgcttcg 180

gttgaaaagg ttacagaatt atctgaagaa aagcctctag aggaaaagcc tatagaggag 240

aagcctttag agggctcagt tggtagagat gctcagctag cacgagttca aactgagaag 300

agaatttcac tgatcaaagc atgggaagaa agtgaaaaat gcaaggccga aaacaaggca 360

cacaagaaaa ttgctgctat cgaggcatgg gagaacagca agaaagcatc tcttgaggca 420

gagctgaaaa agatcgagga aagcttggaa aagaagaagg cagaatatgc agaaaaaatg 480

aagaacaaag tggctatgac tcacaaggaa gcagaggaga agaaggctac ggttgaagcc 540

aaacgtgctg aagatgttct gaaagcggaa gagatggctg ctaagtatcg cgccactgga 600

aagcccccca agaaacttct tggtttgttc taa 633

<210>  16
<211>  210
<212>  PRT
<213>  Beta vulgaris

<400>  16

Met Ala Thr Glu Glu Ala Asn Lys Val Val Ile Glu Thr Pro Ala Pro
1               5                   10                  15

Pro Val Thr Pro Pro Gln Glu Pro Pro Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Pro Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Val Leu Lys Ala Glu Glu Met
            180                 185                 190

Ala Ala Lys Tyr Arg Ala Thr Gly Lys Pro Pro Lys Lys Leu Leu Gly
        195                 200                 205

Leu Phe
    210

<210> 17
<211> 9388
<212> DNA
<213> Beta vulgaris

<220>
<221> misc_feature
<222> (1336)..(1372)
<223> n is a, c, g, or t

<220>
<221> misc_feature
<222> (6213)..(6235)
<223> n is a, c, g, or t

<400> 17
atagatgacg tggtttaact tttgtactat ttacactcac tcctttgatc atgttttatg 60

atttttacgt ttgaaaaata tatttatgtc ggatattatt agattcatct tgatatatat 120

tttcaaaata ttaacttttt ataattatta tatgtaggga gttagatata ttaatagtta 180

agtttctgcg ttggcaagtg cgtccggaat tgtgtctcaa gtatgttgaa acgaaggaag 240

tatatatgtc attacaaagg tcacgatgta taaaaaagtg attgcattta catatgggtc 300

actatgtgtt ccacgtacaa tatttgacat gcataacctt ttaatccaca ataacattat 360

tacaatttgc aaaccaggtc aacataaatc ttactctaaa ctattaaaca tggggaaaat 420

caagtaatta acatattcct tttaacttca ccccaaaaga taaacatttt tcttagtggt 480

cgtttggtaa ataggaaatt atgtaggcaa tagaaattcc catgattttc atatttgtgt 540

agttgtttag ttgacagata gtgaggaaaa cgggggcaat gcaaattcat gagaatttga 600

aattcccaca aaatatacga atttatttac ttactcttcc cttggtcact cacaattgct 660

acataaattc ttatgaatta caactttcct actaccaaac aatttaaact aatttatagg 720

aatttcataa aggaattaat tttctacaaa ttgtaaattc ctatagaaat ttaattccca 780

ttgcaaccaa acaaacacta acactccgag agaaccaaca atccaactcc cgatcctaaa 840

accataacta caagctattt ttactactct attcctttta atataatcat gattcattat 900

cactagcctt tacaaattac aatattagta acagtggatt attttccaat aaaaaagaat 960

gattaatgtg tgattttgtt ataagttctt aaaactaata tggtttatgc tcgtgtgcaa 1020

tcattatcct atttttgcaa caaaatgcta agagtttttg ggttcaatgg tatcacttcc 1080

tattttctat atggaaaatt aaggatcaaa atttggtgga agcataattt caatgttgtc 1140

tggggtgggg attgggtaga ggtgatcatt ttggcccggc ccgtttttgg gccgggtttt 1200

gggcagaatt tttcggcccg cgggccgggc cgggcccggt cgtttttttt ttgtttttgg 1260

gcgggctttg ggcaagctaa aaacgacttt ttagtctatt ttttggcccg gcccgttttg 1320

gcccgcaaaa gcccgnnnnn nnnnnnnnnn nnnnnnnnnn nnnnnnnnnn nnggattcgg 1380

gtacttgttg gacatctggg tgagagtctt gtgcaagaac aacccaaaag taaattcttc 1440

ttaccatgtg gtttccgaaa ggaggcccaa aaaaatcaac aaaataacaa aaaacaaaga 1500

tcaatgacaa ctacttttta tttttggtgt aaaggaggat caacgtaata atcttgtcga 1560

gtttgatccc aagtcttaac tcttaagtat tattccacga gatattacta actaggactg 1620

ttctctaatt ctcttcacca ttaagttcaa ataatatcta actaaaacta atacaatact 1680

taaaaataag tttcgataag attgaatctg gtctaattaa ttcaattcaa ataagttcgg 1740

attttttgag atgaaaacaa cgctaccttg gcatccacaa gaatgacaac tatgaaaagg 1800

tcattttggc tctattattt ttggtttgat tgcttgcacc aactctcaac tctctagtct 1860

ctcctcgttc ttattcattc cagctaatga agctgctatc gttatcataa acttccctct 1920

aactcctttc taacttccct tccactactc ttcttcattt tctctctcct ccctcctttc 1980

ttttcttcct cttttttcta ggaacttcac aattaaaaaa acttaaatat acttggaagt 2040

acttttgatt gtaatacata tggcaacaga ggaagcaaat aaagtggtga ctgaaacacc 2100

ggcaccaccg gtaactccgc cgcaagaacc gccagcaaca gtttcagaaa cccgggaatc 2160

accggttgcg gtggaagaga aaagtttagt ccctgtatca acccctcttc cttccaccac 2220

tgaaaaaact cctgcttcgg ttgaaagtta gttaacaatt ttttttttaa ttatctataa 2280

ttttaattat tattatatga tgatcattat attatagtat atggctaatt ttgctttatt 2340

caagaatatt tagtgattta agggctttta gaataattag tgagatttag ttagtttttt 2400

cccctgtgat ataaagatgg gtcatgggtg gtagtttctt gttttccaat tatatatttt 2460

tactattttt ggtggatggg tgtttttatt tttccttaat ttggtatttt ttctgcatat 2520

tatccatgtt tgttaaggag attacttgct tattttaaga atatcatgag tgttatttgg 2580

gaatattagg gacttatctt agggcttttt ctggatcatg aggtttgaag taaagtggtt 2640

gggcactttt atagtatatt caaaactatt tttttcgctt attgctcatt taaacgattt 2700

caaatgagca aataacaact ataattgaag aaattagcct aagctcatgg tatgcctgag 2760

tgtatggtgt ataatttgat tatcttagac tttcttgtgc gttgcgcagt gattaaagag 2820

taaaggcaat tccagctaat tttggttata ctaagctttt ttaatgctaa ttgatccttt 2880

gttactttgt ccgttgatga taaacctatg taagcaaagt gcactattca tgaatcatga 2940

tactggcctg gccattagct attatctatg ttacttagac acacggattg ggtgtctact 3000

cggatgtggg tgtccaaagt gtctgactcg gctattttgg aaaaaagtct aagtttccgg 3060

tttaaaatga agtatacaat taaagtgtca tgtcggacac gacacgacac gacaccccgt 3120

ttttgaggta aatgaaggtg tcggagtaac acatgctatt atcattcatc aaacagaaat 3180

tagaagctat tacatcttta actatacttg gacaaccacc tagctataca cgagtcggga 3240

actcaattag agtaaggtga gccatatgag ccgctaagtt ggctgtctat aatcatgaat 3300

aaacctaact aaattaaaag aactagtaag agatcaggta tccttaacaa ccatatctgc 3360

ataagattca tcacacttgt tttcattaat catctttact acctgcaggc aattactttc 3420

aattataata tcccacgcat agcattgggt agccattttc attccgagca ctatagcctt 3480

cccttctgtc acattcgttt cccacaagtg tttcactttt tgacacgcca tatgaaaaat 3540

atgcccacca tgatctcgta taacaattcc cattccgatt cccacctcct taaagatcgc 3600

agcgtctaca ttgattttca gcacattata gcttggacag caccacacca cactagagtg 3660

ctgcccacca gtctgatctg cagcaggcag actcatgtgt atgctgtcct tcccctctgt 3720

accagccagt tcaaaggcag ctggtgctgt acacagttct ggaaggtgac gggcaatgct 3780

gccatgataa acattgttcc gttcattcca aaccatccat gccagtattg caactttaca 3840

actctcctcc tataaaacat ccatccacca tgcaacccag tcactagcgc atcatattgg 3900

gattccagaa tactctccaa cccgcctctt ccgaaaggat actgggtatt tcttaatcac 3960

tatttctaaa tcaacgcaag ggtgtttgtt acataaatct ttacaattct tgcatagcat 4020

ttccttatgt aaatgaacat gctcaaaatg ttttatcaaa ctatttttgt taaacatata 4080

atagaatttc ccttgaagta ccaatgatgt gcacagtatc agcgggagct caaaagttgc 4140

aaaagcttag ctatgatgat gctataaact taagagtata taccaggtta ttatttataa 4200

actaaagttg agtcttaagg tgggttgaac ttggaatttt gtgctcaaag acactcgagc 4260

tcaaattact acaaggcaca agctgagttg tgatatccca agtttaagct gtctttaaca 4320

cccctaccta taacctatgc acgactgttg ctatttactg ttttcttggt tcataaggag 4380

cctagagggc aatgatgcac atggttggaa actttgttct tgtgtttcag tgtatcacta 4440

cattaaaaaa atttcatccg agctagccaa tgtatgctca ttatatcttg taataaatac 4500

agctatcact tacttttttc tatctgagat tgtttgctag attatgactt tggactagca 4560

gtatcgcaaa atgcgtataa atgaaatcaa agactcattt ttatctcata atttctcttt 4620

taaatttgta ttttcattca gcgtgtgtta attggcttca aatttaaaat tattcacttt 4680

ttattgacct ctttttttta gaggattcac ttttattaac tagtacactg tatctgatgg 4740

acagaggtta cagaattatc tgaagaaaag cctctagagg aaaagcctat agaggagaag 4800

cctttagagg gctcagttgg tagaggtaat agtttcactc atcttgttgt aaacgagctt 4860

aataagttca gtttcagctt atatcttatt gttgttgtat tcagatgctc agctagcacg 4920

agttcaaact gagaagagaa tttcactgat caaagcatgg gaagaaagtg aaaaatgcaa 4980

ggccgaaaac aagtaagagt actttgtatg gatatgatta gatattagta ctacacccac 5040

aggctataca aatatacatt atcatgtcat ttagagatca catactcaaa atagcaatgg 5100

gaatctcagg ggtgcttgaa ccatgagtga attatttggc ttaagttgtt ttttggtgct 5160
aaagaatatg agactcctag caagaaaaga agaatctaat aagtcttaac tccaccattg 5220
caaggtcgtt aacaatagaa aaactcttgt gacttaattt aagttaacca agcgataggt 5280
aggcctcatg ccaagttaca agatttttca cacatacgcg tgacaaatat agctgtctag 5340
cttgctctta acaataagct tggcgaggtt cttaagtggt ttgtcacatt gctcgtacca 5400
accagtatca ctcttgtcac ctgtcaatgc atcaattagc tctccctata tacttgctgg 5460
acctgggata accattatat actctgtatt aattctttgt attatacttg aataggtcta 5520
aaggccaaca tttgggttag tctctagaag ttgctggagt aaatttgtgc ctatagttga 5580
atggttccgt gcttaaacct tccctttgaa tatcgctaag agttcagtct ttggagcatc 5640
acatcgacca tatatttagc tgagtaagcg ctgatacaat tgcttaagcg ctccttcccc 5700
tactgttcag caactcttgg actgcctcat cctttgcatc agtgaacgta tgttgagtag 5760
tttaatcctt tccatcattc catgactcct ttaaccctca tccactctag ccaaaaacaa 5820
aatcaaattt gttgtgtgat tttgatggtt gagaatttga gatacaaaat agccttttgc 5880
ctcctttacc ataaccaccg tattgtcaat ttgttataag acccatatga ctcattagcc 5940
tatatgggaa agtcttagat caatttactc attctgtttc catttgttct tgcttcaaat 6000
tagaggtggt tattggacag ggtagtccaa tggttaaagg gttagggtca attttacagg 6060
gcttttattg gtcagggttt tattggacag ggtcattatt ggatagggtc attatagggt 6120
caacaaagaa acccaataat tttaaaaaat aaaattagtt ttgtaaaaac aattacatat 6180
tcggattcgc attcatttat acttactttt ggnnnnnnnn nnnnnnnnnn nnnnncgacc 6240
cacccattat cgacccgcta taagccctct acccatttgg cccgacctgc taccgaccct 6300
ttatgaggcg accctcttat gacccaaacc cgcttctgac ccgtattgac cctgacccga 6360
cccgttcaat aaccaggtct acttcaaata tgtaggaaga tacaaaagaa agtgggtaat 6420
aaagaagaaa gagtaagtgt atataatgat ataattataa gcaaggcatg tttggcatct 6480
atctagaatc tggttttggg ttttaagatt tttcaagtca tatgatgtga aatgtatgat 6540
ggacttaaat taagccatat tcaaagtaat catgtctata tcgaaatctg acaacagaac 6600
tcgaaaatat tttttctggt ttggacattt ctatttaatt ggaaacacaa atgataacaa 6660
agagacactc tattgataga ccctttaatt tgatggaagt tacacaaatt tacttgctaa 6720
aaattgcgca gggcacacaa gaaaattgct gctatcgagg catgggagaa cagcaagaaa 6780
gcatctcttg aggcagagct gaagaagatc gaggtatagt gccattaaac agctatcttt 6840
atctcaagtt attttcgaac aatagaagtt tgttgagctg atgaggccag cttacatgag 6900
tgcaggaaag cttggaaaaa aagaaggcag aatatgcaga aaaaatgaag aacaaagtgg 6960
ctatgactca caaggaagca gaggagaaga aggctacggt tgaagccaaa cgtgctgaag 7020

atgttctgaa agcggaagag atggctgcta agtatcgcgc cactggaaag ccccccaaga 7080

aacttcttgg tttgttctaa taaggattat gtgaatttag taattacttc tataaatgtt 7140

tgatgtgatt aacaattaag caagtcaatt agtttgtgat aaggaaatta tagataagtt 7200

atttgtattt tttttccatt tgtataattg ttttatggct ttattggttt ggtaggtgtg 7260

tatagtgtgg gtagaacgat ttaagaattg gtgcacatta ttttccatgt ggatattgtt 7320

tctgttatgt aagtaaattg gatattgttt ctgtaagtaa attggatatt ggaccataac 7380

ttgtgtctga tgtctatcat ttcctattgt cttgtcagtc gtttgcaaat tcgcaaatgc 7440

aaaggtttaa cggaaacctg ttctcctctg aatagatagt tggaatcgtg tgatgcccac 7500

tccaagtgca tatactaaag tccccctcgg agaacagttg tccaaatatc ctaaactagc 7560

tgaaaattat cttagatatg aattaaactt tagagcctcc agagtcatca cgttatgtta 7620

tatatgtacg tagaagaatt catccgctcc tgttagtgtt cagctaataa attagtaaac 7680

tgttagttct gcataatgta ccagtactac aagaaggatt atacttaaga ctgttatctt 7740

actaatgagg tctaatatgt gacaaagatt gtcaagatta tgtaaatcat gcctgttagt 7800

ataaaacaaa ctgcactgat taattttatt cgctggttgg agaatcgagc aacatgttca 7860

ttgctgtatc tgaagtgtca tctgatgcaa cagaccctga gtctgtcctc tgctgctgct 7920

ctgttttgac acctacgtgt tgcggaactt ggcctgttac ccctgaacaa gcctctgagg 7980

aaggtgaaac acgatcgatt ttcaagggca gctggttttg gatcttccta aaggactctc 8040

cgacctctga attccacata gaaaagaagt gatcagagtt ggttctggaa tatcctgagg 8100

ggttgtagag ggattctaat gacaaccgag cctcggtctc caatcggata ctttcccatt 8160

ggaccatatg acgggttgta tgtgactttg attgtgtagt ttctgagtca tcgttgatgg 8220

tttttcgttt cttcaagtgg ctgttccagt ggttcttgac atcattgtct gttcttcctg 8280

gtagccttgc tgctatagca gcccacctgt aaaaatagca tgcataatac ttaggacagc 8340

taacctctgt tttatcaacc acggaatttc tagatttgag gtatcttgct accttgcata 8400

caagaaatga taaacatcta tacaatggat gcatctagca aagtgtctgt gatatagtgt 8460

ttgtgttgtt tttctctcct tttgctactt cacaaatgca taaaagataa ttgacgggag 8520

agttatacct tttatggtct aaacgatttt ttatctttaa aattaatttg acgacgggaa 8580

aagtatcatt taagtcttta agataataaa gtactagttg tcttctctcc ttttattaag 8640

tactgggtat tattatatat gcacaatata atgagattca gcgtaaaagt tgtcaccttt 8700

tctaattggg gatgactata attttttaaa aatatttcat agtttaggta agagattatg 8760

aaatgataaa aagtttccta actatttcta aagagtatat ttttcatgtg atatatggta 8820

tttgtaatca cattagggat aataaaacaa ttagaaaaat acctattgcc aaggagtcca 8880

tgaagttgaa taacagtctc gttttcctct tcagagaaag gacctcgtct aatgtcaggt 8940

cttaaataat tggtccatcg aagacgacaa ctttttccac atcgagatat acctaacata 9000

taacatcaat atgaaatttt atcacacaaa ttaacacata tatagaagta atattttcaa 9060

tttttcatga atgaaatatt caagcttaat gagttaattc accttattgg atgtatagtc 9120

atatacataa tcaagacaac ctttctaggt agcaagtaaa ttgtatgact ttgaggtggg 9180

atggccggat gggttaggtc catgggcttt tgaataagca agaagtgagt acttcgggaa 9240

cgtacttatt tttctgtaaa cccttttaaa caaaaagcac cagtggtcta gtggtagaat 9300

agtaccctgc cacggtacag acccgggttc gattcccggc tggtgcataa aatatcagag 9360

caatgatgat aacattgcgc ctctatgt 9388

<210> 18
<211> 5041
<212> DNA
<213> Beta vulgaris

<220>
<221> misc_feature
<222> (4154)..(4176)
<223> n is a, c, g, or t

<400> 18
atggcaacag aggaagcaaa taaagtggtg actgaaacac cggcaccacc ggtaactccg 60

ccgcaagaac cgccagcaac agtttcagaa acccgggaat caccggttgc ggtggaagag 120

aaaagtttag tccctgtatc aacccctctt ccttccacca ctgaaaaaac tcctgcttcg 180

gttgaaagtt agttaacaat ttttttttta attatctata attttaatta ttattatatg 240

atgatcatta tattatagta tatggctaat tttgctttat tcaagaatat ttagtgattt 300

aagggctttt agaataatta gtgagattta gttagttttt tcccctgtga tataaagatg 360

ggtcatgggt ggtagttttc tgttttccaa ttatatattt ttactatttt tggtggatgg 420

gtgtttttat ttttccttaa tttggtattt tttctgcata ttatccatgt ttgttaagga 480

gattacttgc ttattttaag aatatcatga gtgttatttg ggaatattag ggacttatct 540

tagggctttt tctggatcat gaggtttgaa gtaaagtggt tgggcacttt tatagtatat 600

tcaaaactat ttttttcgct tattgctcat ttaaacgatt tcaaatgagc aaataacaac 660

tataattgaa gaaattagcc taagctcatg gtatgcctga gtgtatggtg tataatttga 720

ttatcttaga ctttcttgtg cgttgcgcag tgattaaaga gtaaaggcaa ttccagctaa 780

ttttggttat actaagcttt tttaatgcta attgatcctt tgttactttg tccgttgatg 840

ataaacctat gtaagcaaag tgcactattc atgaatcatg atactggcct ggccattagc 900

tattatctat gttacttaga cacacggatt gggtgtctac tcggatgtgg gtgtccaaag 960

tgtctgactc ggctattttg gaaaaaagtc taagtttccg gtttaaaatg aagtatacaa 1020

ttaaagtgtc atgtcggaca cgacacgaca cgacaccccg tttttgaggt aaatgaaggt 1080

gtcggagtaa cacatgctat tatcattcat caaacagaaa ttagaagcta ttacatcttt 1140

aactatactt ggacaaccac ctagctatac acgagtcggg aactcaatta gagtaaggtg 1200

agccatatga gccgctaagt tggctgtcta taatcatgaa taaacctaac taaattaaaa 1260

gaactagtaa gagatcaggt atccttaaca accatatctg cataagattc atcacacttg 1320

ttttcattaa tcatctttac tacctgcagg caattacttt caattataat atcccacgca 1380

tagcattggg tagccatttt cattccgagc actatagcct tcccttctgt cacattcgtt 1440

tcccacaagt gtttcacttt ttgacacgcc atatgaaaaa tatgcccacc atgatctcgt 1500

ataacaattc ccattccgat tcccacctcc ttaaagatcg cagcgtctac attgattttc 1560

agcacattat agcttggaca gcaccacacc acactagagt gctgcccacc agtctgatct 1620

gcagcaggca gactcatgtg tatgctgtcc ttcccctctg taccagccag ttcaaaggca 1680

gctggtgctg tacacagttc tggaaggtga cgggcaatgc tgccatgata aacattgttc 1740

cgttcattcc aaaccatcca tgccagtatt gcaactttac aactctcctc ctataaaaca 1800

tccatccacc atgcaaccca gtcactagcg catcatattg ggattccaga atactctcca 1860

acccgcctct tccgaaagga tactgggtat ttcttaatca ctatttctaa atcaacgcaa 1920

gggtgtttgt tacataaatc tttacaattc ttgcatagca tttccttatg taaatgaaca 1980

tgctcaaaat gttttatcaa actatttttg ttaaacatat aatagaattt cccttgaagt 2040

accaatgatg tgcacagtat cagcgggagc tcaaaagttg caaaagctta gctatgatga 2100

tgctataaac ttaagagtat ataccaggtt attatttata aactaaagtt gagtcttaag 2160

gtgggttgaa cttggaattt tgtgctcaaa gacactcgag ctcaaattac tacaaggcac 2220

aagctgagtt gtgatatccc aagtttaagc tgtctttaac acccctacct ataacctatg 2280

cacgactgtt gctatttact gttttcttgg ttcataagga gcctagaggg caatgatgca 2340

catggttgga aactttgttc ttgtgtttca gtgtatcact acattaaaaa aatttcatcc 2400

gagctagcca atgtatgctc attatatctt gtaataaata cagctatcac ttactttttt 2460

ctatctgaga ttgtttgcta gattatgact ttggactagc agtatcgcaa aatgcgtata 2520

aatgaaatca aagactcatt tttatctcat aattttctct ttaaatttgt attttcattc 2580

agcgtgtgtt aattggcttc aaatttaaaa ttattcactt tttattgacc tctttttttt 2640

agaggattca cttttattaa ctagtacact gtatctgatg gacagaggtt acagaattat 2700

ctgaagaaaa gcctctagag gaaaagccta tagaggagaa gcctttagag ggctcagttg 2760

gtagaggtaa tagtttcact catcttgttg taaacgagct taataagttc agtttcagct 2820

tatatcttat tgttgttgta ttcagatgct cagctagcac gagttcaaac tgagaagaga 2880

atttcactga tcaaagcatg ggaagaaagt gaaaaatgca aggccgaaaa caagtaagag 2940

tactttgtat ggatatgatt agatattagt actacaccca caggctatac aaatatacat 3000

tatcatgtca tttagagatc acatactcaa aatagcaatg ggaatctcag gggtgcttga 3060

accatgagtg aattatttgg cttaagttgt tttttggtgc taaagaatat gagactccta 3120

gcaagaaaag aagaatctaa taagtcttaa ctccaccatt gcaaggtcgt taacaataga 3180

aaaactcttg tgacttaatt taagttaacc aagcgatagg taggcctcat gccaagttac 3240

aagatttttc acacatacgc gtgacaaata tagctgtcta gcttgctctt aacaataagc 3300

ttggcgaggt tcttaagtgg tttgtcacat tgctcgtacc aaccagtatc actcttgtca 3360

cctgtcaatg catcaattag ctctccctat atacttgctg gacctgggat aaccattata 3420

tactctgtat taattctttg tattatactt gaataggtct aaaggccaac atttgggtta 3480

gtctctagaa gttgctggag taaatttgtg cctatagttg aatggttccg tgcttaaacc 3540

ttccctttga atatcgctaa gagttcagtc tttggagcat cacatcgacc atatatttag 3600

ctgagtaagc gctgatacaa ttgcttaagc gctccttccc ctactgttca gcaactcttg 3660

gactgcctca tcctttgcat cagtgaacgt atgttgagta gtttaatcct ttccatcatt 3720

ccatgactcc tttaaccctc atccactcta gccaaaaaca aaatcaaatt tgttgtgtga 3780

ttttgatggt tgagaatttg agatacaaaa tagccttttg cctcctttac cataaccacc 3840

gtattgtcaa tttgttataa gacccatatg actcattagc ctatatggga aagtcttaga 3900

tcaatttact cattctgttt ccatttgttc ttgcttcaaa ttagaggtgg ttattggaca 3960

gggtagtcca atggttaaag ggttagggtc aattttacag ggcttttatt ggtcagggtt 4020

ttattggaca gggtcattat tggatagggt cattataggg tcaacaaaga aacccaataa 4080

ttttaaaaaa taaaattagt tttgtaaaaa caattacata ttcggattcg cattcattta 4140

tacttacttt tggnnnnnnn nnnnnnnnnn nnnnnncgac ccacccatta tcgacccgct 4200

ataagccctc tacccatttg gcccgacctg ctaccgaccc tttatgaggc gaccctctta 4260

tgacccaaac ccgcttctga cccgtattga ccctgacccg acccgttcaa taaccaggtc 4320

tacttcaaat atgtaggaag atacaaaaga aagtgggtaa taaagaagaa agagtaagtg 4380

tatataatga tataattata agcaaggcat gtttggcatc tatctagaat ctggttttgg 4440

gttttaagat ttttcaagtc atatgatgtg aaatgtatga tggacttaaa ttaagccata 4500

ttcaaagtaa tcatgtctat atcgaaatct gacaacagaa ctcgaaaata ttttttctgg 4560

tttggacatt tctatttaat tggaaacaca aatgataaca aagagacact ctattgatag 4620

accctttaat ttgatggaag ttacacaaat ttacttgcta aaaattgcgc agggcacaca 4680

agaaaattgc tgctatcgag gcatgggaga acagcaagaa agcatctctt gaggcagagc 4740

tgaagaagat cgaggtatag tgccattaaa cagctatctt tatctcaagt tattttcgaa     4800

caatagaagt ttgttgagct gatgaggcca gcttacatga gtgcaggaaa gcttggaaaa     4860

aaagaaggca gaatatgcag aaaaaatgaa gaacaaagtg gctatgactc acaaggaagc     4920

agaggagaag aaggctacgg ttgaagccaa acgtgctgaa gatgttctga aagcggaaga     4980

gatggctgct aagtatcgcg ccactggaaa gccccccaag aaacttcttg gtttgttcta     5040

a                                                                     5041

<210>  19
<211>  633
<212>  DNA
<213>  Artificial Sequence

<220>
<223>  cDNA of remorin gene - haplotype 5

<400>  19
atggcaacag aggaagcaaa taaagtggtg actgaaacac cggcaccacc ggtaactccg       60

ccgcaagaac cgccagcaac agtttcagaa acccgggaat caccggttgc ggtggaagag      120

aaaagtttag tccctgtatc aacccctctt ccttccacca ctgaaaaaac tcctgcttcg      180

gttgaaaagg ttacagaatt atctgaagaa aagcctctag aggaaaagcc tatagaggag      240

aagcctttag agggctcagt tggtagagat gctcagctag cacgagttca aactgagaag      300

agaatttcac tgatcaaagc atgggaagaa agtgaaaaat gcaaggccga aaacaaggca      360

cacaagaaaa ttgctgctat cgaggcatgg gagaacagca agaaagcatc tcttgaggca      420

gagctgaaga agatcgagga aagcttggaa aaaaagaagg cagaatatgc agaaaaaatg      480

aagaacaaag tggctatgac tcacaaggaa gcagaggaga agaaggctac ggttgaagcc      540

aaacgtgctg aagatgttct gaaagcggaa gagatggctg ctaagtatcg cgccactgga      600

aagcccccca agaaacttct tggtttgttc taa                                   633

<210>  20
<211>  210
<212>  PRT
<213>  Beta vulgaris

<400>  20

Met Ala Thr Glu Glu Ala Asn Lys Val Val Thr Glu Thr Pro Ala Pro
1               5                   10                  15

Pro Val Thr Pro Pro Gln Glu Pro Pro Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Pro Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Val Leu Lys Ala Glu Glu Met
            180                 185                 190

Ala Ala Lys Tyr Arg Ala Thr Gly Lys Pro Pro Lys Lys Leu Leu Gly
        195                 200                 205

Leu Phe
    210

<210>  21
<211>  5009
<212>  DNA
<213>  Beta vulgaris

<400>  21
   caaatcttct ggcatcaatg gcggtgttgc cgttcatcaa tttaacatca atggaggtaa      60
   gagtcatgtt ttttcaacaa tataaaactt atacttcctc tgttctgttt taaatgaaac     120
   gtttgttttc tcacgcaacc caacccactt ttttaataat aaatattttt agttgtgtgc     180
   acgtaaaaaa tataaaaaag ttataatttg atagtatctt gtttgagatt gtgattatta     240
   agagagtcaa gtctcacaat attcgaaagt ctacgtaatc cacctcaaat tgacgaagaa     300
   aacaagcagg aaaggattaa gtaagttcgt ggaaccacta gaattgattt tcaaatatag     360
   ctctacctaa tatatggcct acttttaatt ttaaataagg agaaggtaat gtgattagaa 420
   acaaattggt cttaaattat tcattaagct taataatgta taaacataat caagtgctat 480
   cttcttttca gggccgtctt gaagattttg gggcccggtt ctattatgaa aattgggccc 540
   ctaaatttat agaaaataaa gatggaaggt tagagttcta aagatagaaa gttgaaaatc 600
   taaatataaa tcattgacaa atttattaag ggtgagaaac aagggtgttt tcttcaaata 660
   tgaagcaaaa ttttcaaaat aatacttcct ccgtttctaa ataagtgcaa catttgcata 720
   atgtttacta ttcacagttt aaactttaat tagctttggt gatttacatt ttaggaaaaa 780
   acatagtcat gtgggatctt gttagattcg tctgaatgtg aattttttta atatcaactt 840
   tttataattt ttacttattg ataattgaag atattaatgg ttaaaataat gcattggcaa 900
   acgtgaaaac aagaagtgtt gcacttattt agaaacggag gaagtatatt ttgggccttt 960
   ataattttgg agaccctgcg ctgttgggct ccttgcacac cttcatctac ccctctgctt 1020
   cttttgatac aatttttcag cgacatgatt gtcgattgat gcatatatta ttgtatactc 1080
   gatccatatt gtttaagatg aattgtttgt ctttgatggt ctccaatgca tattttgtat 1140
   acttaggaat tctaattatg tactattagt agacattgag atgaatacat aattgccata 1200
   atgaagtatg attattttag ttatatactt tctccattcc aaatatataa atgtaacact 1260
   tgtgtacttt atgcgtacta atgcataaca acgtgcactc tcatgtgttt aattatatac 1320
   tttttgagag aagtgttaca ttggggacca tgggactgtg tataatttga ccgcaaaatc 1380
   gaagtgtcgc atttgattga aaatggagag agtagtatat agatggaaca cagcagagac 1440
   tgctggtcat ctttggccaa caaacccaaa attgatatta atcccttatt caggtcattt 1500
   catctttttg acacaaaatg gatgttgtag gcactgcgct atctgctgcc caatctctgt 1560
   ttgcagccct gcaaagttct gagctcaaag agatcctctc gatctttggc tacaaatccc 1620
   gacttgatga cctccaacgc actgtctcta ccatcaacgc tgtattccgt gatgctgaga 1680
   ccaaacagga gctcactcat gaagcacagc attggctcga ggaactcaag gatgctgtct 1740
   ttgaagcaga tgatctgttc gacgagtttg tcactcttgc cgagcagaag caacttgtag 1800
   aggctggtgg cagtctttcc aaaaagatgc gccaattctt ttctgattcc aacccccttg 1860
   gcattgctta taggatgtca cgaggggtta agaagatcaa gaagaagttg gatgctatcg 1920
   cttacaatca tcaatttagc tttaagattg atcttgagcc tatgaaagag agaaggctag 1980
   agactggttc tgtcgtgaac gcaggtgata tcattggaag agaggacgac ttggagaaga 2040
   ttgtaggttt gttgcttgat tctaacatcc aacgtgatgt gtctttcctt actattgtgg 2100
   gaatgggagg gttgggtaaa actgctcttg cccaactcgt gtacaatgat ccaagggtca 2160
   gaactgcttt tccattgaga tgttggaatt gtgtgtctga tcaagatcaa aagcaactag 2220
   atgtgaaaga aattttgggt aagattctgg ctacagctac tggtaagaat catgagggtt 2280
   caaccatgga tcaggtgcaa acccaactac gagaacaact atgtggcaag agatacttgc 2340
   ttgttttgga tgatgtatgg aatgagaatc ctaatcaatt gcgtgatctg gtagaattct 2400
   tcatgggagg tcgaagcaga aattggattg tggtaactac gcgttcgcac gagacagcga 2460
   gaattataag agatggtcca ttgcacaagc tccaaggttt gtctgaggaa aactcttggc 2520
   gtttatttgt caggtggacc ttcggatcag tgcaagcaaa attccctaat gactttatca 2580
   tgattgcacg agatatagtt gacaaatgtg ctcgaaaccc tctggctata agagtggtag 2640
   gaagtctttt gtgtggtcaa gacaagagta agtggctttc atttcatgag atcgatttag 2700
   gcaacattag aaagagccat aatgatatca tgccaatact gaacctaagt taccatcatc 2760
   ttgaacctcc aattaagaga tgctttagtt attgtgcagt gtttccaaag gatttcctta 2820
   tagggaagca gacgctgata aacctctgga tggcacaagg ttatattgtt ccgttagaca 2880
   aagatcaaag catagatgat gctagtgagg aatacatatc aattttgttg cggagatgtt 2940
   ttttcgaaaa tgtcggagca gaaaaagatg gtgttattaa gatccatgat ctcatgcatg 3000
   atattgctca aaatgtcatg gggaaggagc tttgtacgac taaaaacatt agtggcagct 3060
   tggataaaag tgttcgccat ctatctcttg ccagaactag ttttgcaaga tactctttca 3120
   atgcaactca tattcgctcc tatttctgtg ctggctactg gtgtcaggat gctgagataa 3180
   accagttttc agttgaggca ttagtaccaa actgtttgta cctaagggca atggacctcg 3240
   cttggtcgaa gataaaaagt ttaccagact cgattggtgg attgttgcat ttgaggtact 3300
   tagatctttc gtataacgaa gatctggaag tacttccaaa ctcaattgct aaactatata 3360
   atctacaaac cttacaattg aagggttgca agagattgga agggttacca aaacatttga 3420
   gcaggctggt taagcttcaa actttggata tacatggttg caacaatgta acttatatgc 3480
   ccaaaggcat gggtaagttg acttgccttc acactctcag taagtttata gtgggtggag 3540
   aagggagttg ttcaagttgg aagcaatgtt ttgatgggtt ggaagatcta aaggctctca 3600
   ataacctaaa gggtcatctg gaaatccaaa tcaggtggcc caaaaatact acagatgctg 3660
   tcaaagaaga tgttacgagg gaaggattat acctgaatca taaggaacat ctcaatcaca 3720
   ttgtggttga ttttagatgt gaggagggtg gtggaagaat ggatgatgag gaagcaagaa 3780
   gattgatgga agagttgcgg ccacatcctt atcttgaaaa tttggctgtg aaagcatatt 3840
   atggtgtgaa aatgcctggt tgggcaaccc ttctcccaaa tcttacagag ctttttcttt 3900
   ctgattgtgg ggaactggag aaccttccat gcctgggaaa cttggatcat ctaaaagtcc 3960
   tccgactttc gcatttggca aaattggagt acattgaaga agatagctca tcagctaatt 4020
   tcaggtgtag gcctggacca gaaagtgcag gactatcatt atacttcccc tcccttgaac 4080
   gccttgagtt gaagcgtttg tgtaagttaa aaggatggag gagaggggaa gggttaggag 4140
   atgatcacca gccttttaat gaaagcagca gcaatacaca agtccaatta caattatgtc 4200
   ttcctcaatt gaagtcattg agaatagaaa gatgcccatt gctgacattt atgccgctgt 4260
   gtcccaagac agaaaaactg catttagttg tatttaatga acgactccgg atagtgcatg 4320
   ctaagagaga tgagaatttc tatgctccat tacattcatc atcatctgat cctgaaaacc 4380
   cgaggaacac tattcccatt cccatgttta gagaggtata cataaacaat gtggcgtggc 4440
   taaattcgct gcctatggag gcttttaggt gtctcactca tatgacaata aaaaacgacg 4500
   aggtagagag tttgggagaa gttggagagg tgtttcggag ctgctcatct tctttgcgat 4560
   ccttgaatat cacaggttgc tccaacttaa gaagtgtttc tggagggctg gagcatctca 4620
   ctgctttgga gatgttagaa atatacgaca cccataagct gagtctatca gaagacccag 4680
   aaggtgttgt gccatggaaa tcccttcatc actccctcag ctacttgcaa ctgatgaatc 4740
   tcccacagct ggtcaacctg cctgattcga tgcagttctt ggctgccctc cgaactcttt 4800
   caatagtgca ttgcactaaa ctgcaatcag tgccagattg gatgcccaga ctcacttctc 4860
   tcaggaagct tatggtttca ttctgttccg cacatctgga gagaagatgc caaaatccaa 4920
   ctggggtgga ctggcctaac attcaacaca tcccctccat tgatgtcacc tctagccttc 4980
   ctaagttttt agtgttgccg tatgaatag 5009

<210> 22
<211> 1163
<212> PRT
<213> Beta vulgaris

<400> 22

Met Asp Val Val Gly Thr Ala Leu Ser Ala Ala Gln Ser Leu Phe Ala
1               5                   10                  15

Ala Leu Gln Ser Ser Glu Leu Lys Glu Ile Leu Ser Ile Phe Gly Tyr
            20                  25                  30

Lys Ser Arg Leu Asp Asp Leu Gln Arg Thr Val Ser Thr Ile Asn Ala
        35                  40                  45

Val Phe Arg Asp Ala Glu Thr Lys Gln Glu Leu Thr His Glu Ala Gln
    50                  55                  60

His Trp Leu Glu Glu Leu Lys Asp Ala Val Phe Glu Ala Asp Asp Leu
65                  70                  75                  80

Phe Asp Glu Phe Val Thr Leu Ala Glu Gln Lys Gln Leu Val Glu Ala
                85                  90                  95

Gly Gly Ser Leu Ser Lys Lys Met Arg Gln Phe Phe Ser Asp Ser Asn

            100                 105                 110

Pro Leu Gly Ile Ala Tyr Arg Met Ser Arg Gly Val Lys Lys Ile Lys
        115                 120                 125

Lys Lys Leu Asp Ala Ile Ala Tyr Asn His Gln Phe Ser Phe Lys Ile
    130                 135                 140

Asp Leu Glu Pro Met Lys Glu Arg Arg Leu Glu Thr Gly Ser Val Val
145                 150                 155                 160

Asn Ala Gly Asp Ile Ile Gly Arg Glu Asp Asp Leu Glu Lys Ile Val
                165                 170                 175

Gly Leu Leu Leu Asp Ser Asn Ile Gln Arg Asp Val Ser Phe Leu Thr
            180                 185                 190

Ile Val Gly Met Gly Gly Leu Gly Lys Thr Ala Leu Ala Gln Leu Val
        195                 200                 205

Tyr Asn Asp Pro Arg Val Arg Thr Ala Phe Pro Leu Arg Cys Trp Asn
    210                 215                 220

Cys Val Ser Asp Gln Asp Gln Lys Gln Leu Asp Val Lys Glu Ile Leu
225                 230                 235                 240

Gly Lys Ile Leu Ala Thr Ala Thr Gly Lys Asn His Glu Gly Ser Thr
                245                 250                 255

Met Asp Gln Val Gln Thr Gln Leu Arg Glu Gln Leu Cys Gly Lys Arg
            260                 265                 270

Tyr Leu Leu Val Leu Asp Asp Val Trp Asn Glu Asn Pro Asn Gln Leu
        275                 280                 285

Arg Asp Leu Val Glu Phe Phe Met Gly Gly Arg Ser Arg Asn Trp Ile
    290                 295                 300

Val Val Thr Thr Arg Ser His Glu Thr Ala Arg Ile Ile Arg Asp Gly
305                 310                 315                 320

Pro Leu His Lys Leu Gln Gly Leu Ser Glu Glu Asn Ser Trp Arg Leu
                325                 330                 335

Phe Val Arg Trp Thr Phe Gly Ser Val Gln Ala Lys Phe Pro Asn Asp
            340                 345                 350

Phe Ile Met Ile Ala Arg Asp Ile Val Asp Lys Cys Ala Arg Asn Pro
        355                 360                 365

Leu Ala Ile Arg Val Val Gly Ser Leu Leu Cys Gly Gln Asp Lys Ser
    370                 375                 380

Lys Trp Leu Ser Phe His Glu Ile Asp Leu Gly Asn Ile Arg Lys Ser
385                 390                 395                 400

His Asn Asp Ile Met Pro Ile Leu Asn Leu Ser Tyr His His Leu Glu
                405                 410                 415

Pro Pro Ile Lys Arg Cys Phe Ser Tyr Cys Ala Val Phe Pro Lys Asp
            420                 425                 430

Phe Leu Ile Gly Lys Gln Thr Leu Ile Asn Leu Trp Met Ala Gln Gly
        435                 440                 445

Tyr Ile Val Pro Leu Asp Lys Asp Gln Ser Ile Asp Asp Ala Ser Glu
    450                 455                 460

Glu Tyr Ile Ser Ile Leu Leu Arg Arg Cys Phe Phe Glu Asn Val Gly
465                 470                 475                 480

Ala Glu Lys Asp Gly Val Ile Lys Ile His Asp Leu Met His Asp Ile
                485                 490                 495

Ala Gln Asn Val Met Gly Lys Glu Leu Cys Thr Thr Lys Asn Ile Ser
            500                 505                 510

Gly Ser Leu Asp Lys Ser Val Arg His Leu Ser Leu Ala Arg Thr Ser
        515                 520                 525

Phe Ala Arg Tyr Ser Phe Asn Ala Thr His Ile Arg Ser Tyr Phe Cys
    530                 535                 540

Ala Gly Tyr Trp Cys Gln Asp Ala Glu Ile Asn Gln Phe Ser Val Glu
545                 550                 555                 560

Ala Leu Val Pro Asn Cys Leu Tyr Leu Arg Ala Met Asp Leu Ala Trp
                565                 570                 575

Ser Lys Ile Lys Ser Leu Pro Asp Ser Ile Gly Gly Leu Leu His Leu
            580                 585                 590

Arg Tyr Leu Asp Leu Ser Tyr Asn Glu Asp Leu Glu Val Leu Pro Asn
        595                 600                 605

Ser Ile Ala Lys Leu Tyr Asn Leu Gln Thr Leu Gln Leu Lys Gly Cys
    610                 615                 620

Lys Arg Leu Glu Gly Leu Pro Lys His Leu Ser Arg Leu Val Lys Leu
625                 630                 635                 640

Gln Thr Leu Asp Ile His Gly Cys Asn Asn Val Thr Tyr Met Pro Lys
                645                 650                 655

Gly Met Gly Lys Leu Thr Cys Leu His Thr Leu Ser Lys Phe Ile Val
            660                 665                 670

Gly Gly Glu Gly Ser Cys Ser Ser Trp Lys Gln Cys Phe Asp Gly Leu
        675                 680                 685

Glu Asp Leu Lys Ala Leu Asn Asn Leu Lys Gly His Leu Glu Ile Gln
    690                 695                 700

Ile Arg Trp Pro Lys Asn Thr Thr Asp Ala Val Lys Glu Asp Val Thr
705                 710                 715                 720

Arg Glu Gly Leu Tyr Leu Asn His Lys Glu His Leu Asn His Ile Val
                725                 730                 735

Val Asp Phe Arg Cys Glu Glu Gly Gly Gly Arg Met Asp Asp Glu Glu
            740                 745                 750

Ala Arg Arg Leu Met Glu Glu Leu Arg Pro His Pro Tyr Leu Glu Asn
        755                 760                 765

Leu Ala Val Lys Ala Tyr Tyr Gly Val Lys Met Pro Gly Trp Ala Thr
    770                 775                 780

Leu Leu Pro Asn Leu Thr Glu Leu Phe Leu Ser Asp Cys Gly Glu Leu
785                 790                 795                 800

Glu Asn Leu Pro Cys Leu Gly Asn Leu Asp His Leu Lys Val Leu Arg
                805                 810                 815

Leu Ser His Leu Ala Lys Leu Glu Tyr Ile Glu Glu Asp Ser Ser Ser
            820                 825                 830

Ala Asn Phe Arg Cys Arg Pro Gly Pro Glu Ser Ala Gly Leu Ser Leu
        835                 840                 845

Tyr Phe Pro Ser Leu Glu Arg Leu Glu Leu Lys Arg Leu Cys Lys Leu
    850                 855                 860

Lys Gly Trp Arg Arg Gly Glu Gly Leu Gly Asp Asp His Gln Pro Phe
865                 870                 875                 880

Asn Glu Ser Ser Ser Asn Thr Gln Val Gln Leu Gln Leu Cys Leu Pro
                885                 890                 895

Gln Leu Lys Ser Leu Arg Ile Glu Arg Cys Pro Leu Leu Thr Phe Met
            900                 905                 910

Pro Leu Cys Pro Lys Thr Glu Lys Leu His Leu Val Val Phe Asn Glu
        915                 920                 925

Arg Leu Arg Ile Val His Ala Lys Arg Asp Glu Asn Phe Tyr Ala Pro
    930                 935                 940

Leu His Ser Ser Ser Ser Asp Pro Glu Asn Pro Arg Asn Thr Ile Pro
945                 950                 955                 960

Ile Pro Met Phe Arg Glu Val Tyr Ile Asn Asn Val Ala Trp Leu Asn
                965                 970                 975

Ser Leu Pro Met Glu Ala Phe Arg Cys Leu Thr His Met Thr Ile Lys
            980                 985                 990

Asn Asp Glu Val Glu Ser Leu Gly  Glu Val Gly Glu Val  Phe Arg Ser
        995                 1000                 1005

Cys Ser  Ser Ser Leu Arg Ser  Leu Asn Ile Thr Gly  Cys Ser Asn
    1010                 1015                 1020

Leu Arg  Ser Val Ser Gly Gly  Leu Glu His Leu Thr  Ala Leu Glu
    1025                 1030                 1035

Met Leu  Glu Ile Tyr Asp Thr  His Lys Leu Ser Leu  Ser Glu Asp
    1040                 1045                 1050

Pro Glu  Gly Val Val Pro Trp  Lys Ser Leu His His  Ser Leu Ser
    1055                 1060                 1065

Tyr Leu  Gln Leu Met Asn Leu  Pro Gln Leu Val Asn  Leu Pro Asp
    1070                 1075                 1080

Ser Met  Gln Phe Leu Ala Ala  Leu Arg Thr Leu Ser  Ile Val His
    1085                 1090                 1095

Cys Thr  Lys Leu Gln Ser Val  Pro Asp Trp Met Pro  Arg Leu Thr

1100                    1105                    1110

Ser Leu  Arg Lys Leu Met Val  Ser Phe Cys Ser Ala  His Leu Glu
    1115                    1120                    1125

Arg Arg  Cys Gln Asn Pro Thr  Gly Val Asp Trp Pro  Asn Ile Gln
    1130                    1135                    1140

His Ile  Pro Ser Ile Asp Val  Thr Ser Ser Leu Pro  Lys Phe Leu
    1145                    1150                    1155

Val Leu  Pro Tyr Glu
    1160

<210>  23
<211>  1177
<212>  PRT
<213>  Beta vulgaris

<400>  23

Met Glu Arg Val Val Tyr Arg Trp Asn Thr Ala Glu Thr Ala Gly His
1               5                   10                  15

Leu Trp Pro Thr Asn Pro Lys Leu Ile Leu Ile Pro Tyr Ser Ala Leu
            20                  25                  30

Gln Ser Ser Glu Leu Lys Glu Ile Leu Ser Ile Phe Gly Tyr Lys Ser
        35                  40                  45

Arg Leu Asp Asp Leu Gln Arg Thr Val Ser Thr Ile Asn Ala Val Phe
    50                  55                  60

Arg Asp Ala Glu Thr Lys Gln Glu Leu Thr His Glu Ala Gln His Trp
65                  70                  75                  80

Leu Glu Glu Leu Lys Asp Ala Val Phe Glu Ala Asp Asp Leu Phe Asp
                85                  90                  95

Glu Phe Val Thr Leu Ala Glu Gln Lys Gln Leu Val Glu Ala Gly Gly
            100                 105                 110

Ser Leu Ser Lys Lys Met Arg Gln Phe Phe Ser Asp Ser Asn Pro Leu
        115                 120                 125

Gly Ile Ala Tyr Arg Met Ser Arg Gly Val Lys Lys Ile Lys Lys Lys
    130                 135                 140

Leu Asp Ala Ile Ala Tyr Asn His Gln Phe Ser Phe Lys Ile Asp Leu

145                 150                 155                 160

Glu Pro Met Lys Glu Arg Arg Leu Glu Thr Gly Ser Val Val Asn Ala
                165                 170                 175

Gly Asp Ile Ile Gly Arg Glu Asp Asp Leu Glu Lys Ile Val Gly Leu
            180                 185                 190

Leu Leu Asp Ser Asn Ile Gln Arg Asp Val Ser Phe Leu Thr Ile Val
        195                 200                 205

Gly Met Gly Gly Leu Gly Lys Thr Ala Leu Ala Gln Leu Val Tyr Asn
    210                 215                 220

Asp Pro Arg Val Arg Thr Ala Phe Pro Leu Arg Cys Trp Asn Cys Val
225                 230                 235                 240

Ser Asp Gln Asp Gln Lys Gln Leu Asp Val Lys Glu Ile Leu Gly Lys
                245                 250                 255

Ile Leu Ala Thr Ala Thr Gly Lys Asn His Glu Gly Ser Thr Met Asp
            260                 265                 270

Gln Val Gln Thr Gln Leu Arg Glu Gln Leu Cys Gly Lys Arg Tyr Leu
        275                 280                 285

Leu Val Leu Asp Asp Val Trp Asn Glu Asn Pro Asn Gln Leu Arg Asp
    290                 295                 300

Leu Val Glu Phe Phe Met Gly Gly Arg Ser Arg Asn Trp Ile Val Val
305                 310                 315                 320

Thr Thr Arg Ser His Glu Thr Ala Arg Ile Ile Arg Asp Gly Pro Leu
                325                 330                 335

His Lys Leu Gln Gly Leu Ser Glu Glu Asn Ser Trp Arg Leu Phe Val
            340                 345                 350

Arg Trp Thr Phe Gly Ser Val Gln Ala Lys Phe Pro Asn Asp Phe Ile
        355                 360                 365

Met Ile Ala Arg Asp Ile Val Asp Lys Cys Ala Arg Asn Pro Leu Ala
    370                 375                 380

Ile Arg Val Val Gly Ser Leu Leu Cys Gly Gln Asp Lys Ser Lys Trp
385                 390                 395                 400

Leu Ser Phe His Glu Ile Asp Leu Gly Asn Ile Arg Lys Ser His Asn
                405                 410                 415

Asp Ile Met Pro Ile Leu Asn Leu Ser Tyr His His Leu Glu Pro Pro
            420                 425                 430

Ile Lys Arg Cys Phe Ser Tyr Cys Ala Val Phe Pro Lys Asp Phe Leu
        435                 440                 445

Ile Gly Lys Gln Thr Leu Ile Asn Leu Trp Met Ala Gln Gly Tyr Ile
    450                 455                 460

Val Pro Leu Asp Lys Asp Gln Ser Ile Asp Asp Ala Ser Glu Glu Tyr
465                 470                 475                 480

Ile Ser Ile Leu Leu Arg Arg Cys Phe Phe Glu Asn Val Gly Ala Glu
                485                 490                 495

Lys Asp Gly Val Ile Lys Ile His Asp Leu Met His Asp Ile Ala Gln
            500                 505                 510

Asn Val Met Gly Lys Glu Leu Cys Thr Thr Lys Asn Ile Ser Gly Ser
        515                 520                 525

Leu Asp Lys Ser Val Arg His Leu Ser Leu Ala Arg Thr Ser Phe Ala
    530                 535                 540

Arg Tyr Ser Phe Asn Ala Thr His Ile Arg Ser Tyr Phe Cys Ala Gly
545                 550                 555                 560

Tyr Trp Cys Gln Asp Ala Glu Ile Asn Gln Phe Ser Val Glu Ala Leu
                565                 570                 575

Val Pro Asn Cys Leu Tyr Leu Arg Ala Met Asp Leu Ala Trp Ser Lys
            580                 585                 590

Ile Lys Ser Leu Pro Asp Ser Ile Gly Gly Leu Leu His Leu Arg Tyr
        595                 600                 605

Leu Asp Leu Ser Tyr Asn Glu Asp Leu Glu Val Leu Pro Asn Ser Ile
    610                 615                 620

Ala Lys Leu Tyr Asn Leu Gln Thr Leu Gln Leu Lys Gly Cys Lys Arg
625                 630                 635                 640

Leu Glu Gly Leu Pro Lys His Leu Ser Arg Leu Val Lys Leu Gln Thr
                645                 650                 655

Leu Asp Ile His Gly Cys Asn Asn Val Thr Tyr Met Pro Lys Gly Met
            660                 665                 670

Gly Lys Leu Thr Cys Leu His Thr Leu Ser Lys Phe Ile Val Gly Gly
        675                 680                 685

Glu Gly Ser Cys Ser Ser Trp Lys Gln Cys Phe Asp Gly Leu Glu Asp
    690                 695                 700

Leu Lys Ala Leu Asn Asn Leu Lys Gly His Leu Glu Ile Gln Ile Arg
705                 710                 715                 720

Trp Pro Lys Asn Thr Thr Asp Ala Val Lys Glu Asp Val Thr Arg Glu
                725                 730                 735

Gly Leu Tyr Leu Asn His Lys Glu His Leu Asn His Ile Val Val Asp
            740                 745                 750

Phe Arg Cys Glu Glu Gly Gly Gly Arg Met Asp Asp Glu Glu Ala Arg
        755                 760                 765

Arg Leu Met Glu Glu Leu Arg Pro His Pro Tyr Leu Glu Asn Leu Ala
    770                 775                 780

Val Lys Ala Tyr Tyr Gly Val Lys Met Pro Gly Trp Ala Thr Leu Leu
785                 790                 795                 800

Pro Asn Leu Thr Glu Leu Phe Leu Ser Asp Cys Gly Glu Leu Glu Asn
                805                 810                 815

Leu Pro Cys Leu Gly Asn Leu Asp His Leu Lys Val Leu Arg Leu Ser
            820                 825                 830

His Leu Ala Lys Leu Glu Tyr Ile Glu Glu Asp Ser Ser Ser Ala Asn
        835                 840                 845

Phe Arg Cys Arg Pro Gly Pro Glu Ser Ala Gly Leu Ser Leu Tyr Phe
    850                 855                 860

Pro Ser Leu Glu Arg Leu Glu Leu Lys Arg Leu Cys Lys Leu Lys Gly
865                 870                 875                 880

Trp Arg Arg Gly Glu Gly Leu Gly Asp Asp His Gln Pro Phe Asn Glu
                885                 890                 895

Ser Ser Ser Asn Thr Gln Val Gln Leu Gln Leu Cys Leu Pro Gln Leu
            900                 905                 910

Lys Ser Leu Arg Ile Glu Arg Cys Pro Leu Leu Thr Phe Met Pro Leu
      915                 920                 925

Cys Pro Lys Thr Glu Lys Leu His Leu Val Val Phe Asn Glu Arg Leu
    930                 935                 940

Arg Ile Val His Ala Lys Arg Asp Glu Asn Phe Tyr Ala Pro Leu His
945                 950                 955                 960

Ser Ser Ser Ser Asp Pro Glu Asn Pro Arg Asn Thr Ile Pro Ile Pro
                965                 970                 975

Met Phe Arg Glu Val Tyr Ile Asn Asn Val Ala Trp Leu Asn Ser Leu
            980                 985                 990

Pro Met Glu Ala Phe Arg Cys Leu  Thr His Met Thr Ile  Lys Asn Asp
      995                 1000                1005

Glu Val  Glu Ser Leu Gly Glu  Val Gly Glu Val Phe  Arg Ser Cys
    1010                1015                1020

Ser Ser  Ser Leu Arg Ser Leu  Asn Ile Thr Gly Cys  Ser Asn Leu
    1025                1030                1035

Arg Ser  Val Ser Gly Gly Leu  Glu His Leu Thr Ala  Leu Glu Met
    1040                1045                1050

Leu Glu  Ile Tyr Asp Thr His  Lys Leu Ser Leu Ser  Glu Asp Pro
    1055                1060                1065

Glu Gly  Val Val Pro Trp Lys  Ser Leu His His Ser  Leu Ser Tyr
    1070                1075                1080

Leu Gln  Leu Met Asn Leu Pro  Gln Leu Val Asn Leu  Pro Asp Ser
    1085                1090                1095

Met Gln  Phe Leu Ala Ala Leu  Arg Thr Leu Ser Ile  Val His Cys
    1100                1105                1110

Thr Lys  Leu Gln Ser Val Pro  Asp Trp Met Pro Arg  Leu Thr Ser
    1115                1120                1125

Leu Arg  Lys Leu Met Val Ser  Phe Cys Ser Ala His  Leu Glu Arg
    1130                1135                1140

Arg Cys  Gln Asn Pro Thr Gly  Val Asp Trp Pro Asn  Ile Gln His

1145                 1150                 1155

Ile Pro Ser Ile Asp Val Thr Ser Ser Leu Pro Lys Phe Leu Val
    1160                 1165                 1170

Leu Pro Tyr Glu
    1175

<210>  24
<211>  48
<212>  DNA
<213>  Beta vulgaris

<400>  24
gaaggtgacc aagttcatgc tgtttaatgg cactatacct cgatcttt                48

<210>  25
<211>  45
<212>  DNA
<213>  Beta vulgaris

<400>  25
gaaggtcgga gtcaacggat ttaatggcac tatacctcga tcttc                   45

<210>  26
<211>  141
<212>  DNA
<213>  Beta vulgaris

<220>
<221>  misc_feature
<222>  (39)..(48)
<223>  n is a, c, g, or t

<400>  26
ggatgatgta tggaatgaga atcctaatca attgcgtdnn nnnnnnnnat tcttcatggg    60

aggtcraags rgaaattgga ttstggtaac tacgcgttcg caygagacar cgagaattat   120

aagagatggt ccattgcaca a                                             141

<210>  27
<211>  141
<212>  DNA
<213>  Beta vulgaris

<400>  27
atttcctywt cacaaactaa ttgasttgct taattgttaa tcacatcaaa catttataga    60

agtaawtact raattcacat artccttatt agaacaaacc aagaagtttc ttggggggct   120

ttccagtggc gcgatactta g                                             141

<210>  28
<211>  141

<212>  DNA
<213>  Beta vulgaris

<400>  28
aaaccaagaa gtttcttggg gggctttcca gtggcgcgat acttagcagc catctcttcc      60

gctttcagaa yatcttcagc acgtttggct tcaacygtag ccttcttctc ytctgcytcc     120

ttgtgagtca tagccacttt g                                               141


<210>  29
<211>  141
<212>  DNA
<213>  Beta vulgaris

<400>  29
ccgaagcagg agttttttca gtggtggaag gaagaggggt tgatacaggg actaaacttt      60

tctcttccac ygcaacygrt gattcccggg tttcygaaac ygttgcygsy ggttcttgyg     120

gcggagttac cggyggtgcc g                                               141


<210>  30
<211>  141
<212>  DNA
<213>  Beta vulgaris


<220>
<221>  misc_feature
<222>  (104)..(104)
<223>  n is a, c, g, or t

<400>  30
ttgagaacct atgttcatct ygagagaata aattgggggc caggagttag atataatttg      60

gtcatagaga ywtgatcacg cctaactcct ccaatcatgt actnagccaa tctttacgaa     120

tgtcgaatgt gtaattgatc t                                               141


<210>  31
<211>  141
<212>  DNA
<213>  Beta vulgaris

<400>  31
tggttacgct cctgtcagtt gtttccaagt tctcacaraa ggactggtta tcaagctatg      60

agacattgac ygactatgtg gtaaggacta catatatatt atactctctt cataggttag     120

gtgcacttat ttattgtgat y                                               141


<210>  32
<211>  141
<212>  DNA
<213>  Beta vulgaris

<400>  32
aattacggtt taagtcctaa ttacaagatc tcatatggaa gatctgcttc tcttgtttcg      60

acatcatcag wtcagttcgg tcctatccga gctgtgcaag ataatgtggg acctagaaga 120

ctggtagaca tcattcggac t 141

<210> 33
<211> 141
<212> DNA
<213> Beta vulgaris

<400> 33
tggaaagaga agtaatgttg attaatatta tctctagtat ctgctcaagt accgcaagct 60

ratgctggtg ygggtgcaat ttcatccaag mctgcgaaat ctagtcaacg tgaaactcag 120

ccggckaggg ttscagaaaa t 141

<210> 34
<211> 141
<212> DNA
<213> Beta vulgaris

<220>
<221> misc_feature
<222> (31)..(31)
<223> n is a, c, g, or t

<400> 34
aagtcaaaaa gtttggagaa gcaaaaaacg naaaaaaaar agtagaagaa ggtaaagcta 60

cagagtggag raggaggaga gagaaagaga gagacagaga gaagcttcga ttcagagttc 120

ctagatagtt tgtggaaccc c 141

<210> 35
<211> 141
<212> DNA
<213> Beta vulgaris

<400> 35
tgtcgtcttc atcgggtgat tttgggtctt ccatgatcaa ttgagaagga tttttaggtw 60

takttcgatt rttggagttt acgcgggtaa ttagatggag taggagatta gccaaatttg 120

tagtttcgca catgaattag g 141

<210> 36
<211> 141
<212> DNA
<213> Beta vulgaris

<400> 36
tagctttaga aaaagctggg cctagattgg aggaaattaa ccagaggttg ccttcacttg 60

aggctgctgt yagacctatc agggctaatg aggatgccct tgtgtctgtt gtgggccaca 120

ttgaccgsgc tgtcggccct g 141

<210> 37
<211> 141
<212> DNA
<213> Beta vulgaris

<400> 37
cattttccga gaaagcggtg tatatysgta ttactatcgt tgccttttgt aacttgtctt 60

tttgttaata mgggaaataa aggcaatgaa ctctttgttc tcagcagaac ggttatgtga 120

atagcccaac tcttgcttct a 141


<210> 38
<211> 141
<212> DNA
<213> Beta vulgaris

<400> 38
atatattatt acctgtgtca ctcaaattta ttggttgatg tcaattattc tttgttttat 60

atwattcaty ytttttaata taataacttt atataaatct aataatgtgg accaataata 120

ttaagtggga catatataat g 141


<210> 39
<211> 141
<212> DNA
<213> Beta vulgaris

<400> 39
agcgtgagct gaggcaatgs atgcaacgtt gccaacagcg accaggtggt cagtacgarc 60

agaaggagtg kgtccaagag tgtcgagagg aatacggtgg gagaggaaga tgggtcgacg 120

aaggtaatta tgatgagttg a 141


<210> 40
<211> 141
<212> DNA
<213> Beta vulgaris


<220>
<221> misc_feature
<222> (84)..(84)
<223> n is a, c, g, or t

<400> 40
ttgcattctt cagcatgctt tgcattacat cacaaatcag cccaatctta kswwymcwwy 60

gtccaacaac maycactcca aswncataca cagatttctt aaaaacatca tgcaaggcaa 120

cactttaccc tgagctwtgc t 141


<210> 41
<211> 1155
<212> DNA
<213> Artificial Sequence

<220>
<223>  cDNA of TGB1 derived from Beet necrotic yellow vein virus

<400>  41
atggtccaag tacagcgtag aacgggcggc gacaagggcg caaagggcaa tcgtgcttcc      60

agcgcccccg tccgctcccg ccggatgact caggatgact ggtcacgtac ccatcccgac     120

gatattttct cagttattga gaaaacacta gtagaggatg ggtataaatg gaacggggta     180

aaacccggac attgcgattg gggcaaattg aaggaatctg gtgctattga taattttagg     240

ggtacattag aaggcgagtt aggtaaaaat tgtgatttga cttgtaatgc tgctgccgtt     300

aaacttgaca cattgcaaaa ggtgaaaatg tcatcagatt ggactgccag agttggtatt     360

gttttgggtg ctcctggtgt tgggaaatct acctcgatta agaacttatt agacaaattt     420

ggagcaaaac ataaaatggt gttatgctta ccttttagtc agttgttaga aggagtgttt     480

gctggtcggt tggacacttt tctggttgat gatttgttct gtaggtccgt gggatatgga     540

aaatacaaca ccatgcttgt agatgaggtc actcgtgtgc atatgtgtga gattttggta     600

cttgccggac atttaggtgt taagaacgtg atatgttttg gtgatccggc gcaagggtta     660

aattataagg ccggttctgc cgtgaactat aattttccga ttattgctga atgttatgct     720

agtagacggt tcggtaaggc gactgccgat ctcattaatt ccagcaatgg tggtggtaaa     780

cctgtagtcg gtaataacga ggtaaaggat agttggactt ttgaagaact atgtgggaag     840

atactagata tgtctactgt tttggtagct acacgcgaaa cccaaaagtt tctattagaa     900

gataatattg agtctattct ttactcggac gcccacgggc aaacatacga cgttgtcact     960

atcattttgg aagacgagtt tgatgatgct gccatttgcg acccaaatgt tagggctgtc    1020

ttgttaacta gagctcgtaa gggtggtatg attaagatgg gtcctaacat tgctgccagg    1080

ttcaaaaacg gtgattttaa ttcacgtgga gttagtaagt cttgcaccgg agatactttt    1140

tgcgaagata gataa                                                     1155

<210>  42
<211>  384
<212>  PRT
<213>  Beet necrotic yellow vein mosaic virus

<400>  42

Met Val Gln Val Gln Arg Arg Thr Gly Gly Asp Lys Gly Ala Lys Gly
1               5                   10                  15

Asn Arg Ala Ser Ser Ala Pro Val Arg Ser Arg Arg Met Thr Gln Asp
            20                  25                  30

Asp Trp Ser Arg Thr His Pro Asp Asp Ile Phe Ser Val Ile Glu Lys
        35                  40                  45

Thr Leu Val Glu Asp Gly Tyr Lys Trp Asn Gly Val Lys Pro Gly His
    50                  55                  60

Cys Asp Trp Gly Lys Leu Lys Glu Ser Gly Ala Ile Asp Asn Phe Arg
65                  70                  75                  80

Gly Thr Leu Glu Gly Glu Leu Gly Lys Asn Cys Asp Leu Thr Cys Asn
                85                  90                  95

Ala Ala Ala Val Lys Leu Asp Thr Leu Gln Lys Val Lys Met Ser Ser
            100                 105                 110

Asp Trp Thr Ala Arg Val Gly Ile Val Leu Gly Ala Pro Gly Val Gly
        115                 120                 125

Lys Ser Thr Ser Ile Lys Asn Leu Leu Asp Lys Phe Gly Ala Lys His
    130                 135                 140

Lys Met Val Leu Cys Leu Pro Phe Ser Gln Leu Leu Glu Gly Val Phe
145                 150                 155                 160

Ala Gly Arg Leu Asp Thr Phe Leu Val Asp Asp Leu Phe Cys Arg Ser
                165                 170                 175

Val Gly Tyr Gly Lys Tyr Asn Thr Met Leu Val Asp Glu Val Thr Arg
            180                 185                 190

Val His Met Cys Glu Ile Leu Val Leu Ala Gly His Leu Gly Val Lys
        195                 200                 205

Asn Val Ile Cys Phe Gly Asp Pro Ala Gln Gly Leu Asn Tyr Lys Ala
    210                 215                 220

Gly Ser Ala Val Asn Tyr Asn Phe Pro Ile Ile Ala Glu Cys Tyr Ala
225                 230                 235                 240

Ser Arg Arg Phe Gly Lys Ala Thr Ala Asp Leu Ile Asn Ser Ser Asn
                245                 250                 255

Gly Gly Gly Lys Pro Val Val Gly Asn Asn Glu Val Lys Asp Ser Trp
            260                 265                 270

Thr Phe Glu Glu Leu Cys Gly Lys Ile Leu Asp Met Ser Thr Val Leu
        275                 280                 285

Val Ala Thr Arg Glu Thr Gln Lys Phe Leu Leu Glu Asp Asn Ile Glu

290                 295                 300

Ser Ile Leu Tyr Ser Asp Ala His Gly Gln Thr Tyr Asp Val Val Thr
305                 310                 315                 320

Ile Ile Leu Glu Asp Glu Phe Asp Asp Ala Ala Ile Cys Asp Pro Asn
                325                 330                 335

Val Arg Ala Val Leu Leu Thr Arg Ala Arg Lys Gly Gly Met Ile Lys
            340                 345                 350

Met Gly Pro Asn Ile Ala Ala Arg Phe Lys Asn Gly Asp Phe Asn Ser
        355                 360                 365

Arg Gly Val Ser Lys Ser Cys Thr Gly Asp Thr Phe Cys Glu Asp Arg
    370                 375                 380

<210>  43
<211>  382
<212>  PRT
<213>  Beet soil-borne virus

<400>  43

Met Ala Pro Glu Gln His Lys Gln Asn Ala Ser Glu Thr Ala Ser Gly
1               5                   10                  15

Arg Arg Asn Ser Ser Val Arg Ser Arg Gly Met Ser Lys Asp Asp Trp
            20                  25                  30

Ser Val Thr His Pro Asp Asp Val Phe Ser Ile Ile Glu Lys Thr Leu
        35                  40                  45

Val Glu Asp Gly Tyr Lys Trp His Gly Val Lys Pro Gly His Cys Asp
    50                  55                  60

Trp Asp Lys Leu Glu Gln Ser Gly Ala Ile Lys Asn Phe Lys Gly Thr
65                  70                  75                  80

Leu Glu Gly Glu Val Asp Ser Ser Cys Ser Leu Thr Cys Asn Ala Ala
                85                  90                  95

Ala Ile Lys Leu Asp Ile Val Glu Arg Leu Asp Val Ser Ser Asp Trp
            100                 105                 110

Ser Ala Arg Val Gly Ile Val Leu Gly Ala Pro Gly Val Gly Lys Ser
        115                 120                 125

Thr Ser Ile Lys His Ile Leu Asp Thr Tyr Gly Ser Arg Tyr Lys Met

    130                 135                 140

Val Leu Cys Leu Pro Val Lys Gln Leu Leu Asp Gly Val Phe Ser Gly
145                 150                 155                 160

Arg Met Asp Thr Phe Leu Ile Asp Asp Ile Phe Ser Arg Ser Val Asp
                165                 170                 175

Tyr Gly Lys Tyr His Thr Met Leu Val Asp Glu Ile Thr Arg Val His
            180                 185                 190

Met Cys Glu Val Leu Val Leu Ala Gly Tyr Leu Gly Ile Lys Asn Val
        195                 200                 205

Ile Cys Phe Gly Asp Pro Ala Gln Gly Ile Asn Phe Lys Ala Gly Ser
    210                 215                 220

Ala Val Asn Tyr Asn Phe Pro Val Ile Ala Glu Cys Tyr Ser Ser Arg
225                 230                 235                 240

Arg Phe Gly Val Ala Thr Ala Asp Leu Ile Asn Ser Cys Asn Gly Gly
                245                 250                 255

Gly Lys Ser Val Val Gly Asn Asn Asp Val Lys Asp Asn Trp Thr Phe
            260                 265                 270

Glu Glu Leu Cys Gly Lys Ile Glu Glu Met Ser Thr Val Leu Val Ala
        275                 280                 285

Thr His Ala Thr Lys Glu Phe Leu Ala Asp Asp Gly Ile Glu Ala Val
    290                 295                 300

Tyr Tyr Glu Asp Ala Gln Gly Met Thr Tyr Asp Val Val Thr Ile Val
305                 310                 315                 320

Leu Lys Asp Glu Phe Asp Asp Asp Ala Ile Cys Asp Ser Asn Val Arg
                325                 330                 335

Ala Val Leu Leu Thr Arg Ala Arg Lys Gly Gly Leu Leu Lys Val Asp
            340                 345                 350

Pro Asn Ile Ala Ala Arg Phe Lys Asn Gly Asp Phe Asn Ser Arg Gly
        355                 360                 365

Val Ser Lys Ala Cys Thr Gly Asp Thr Phe Cys Glu Asp Arg
    370                 375                 380

<210>  44
<211>  633
<212>  DNA
<213>  Artificial Sequence

<220>
<223>  consensus of cDNA derived from haplotypes 2 to 5

<400>  44
atggcaacag aggaagcaaa taaagtggtg aytgaaacac cggcaccacc ggtaactccg      60

ccrcaagaac cgccrgcaac agtttcrgaa acccgggaat caccrgttgc ggtggaagag     120

aaaagtttag tccctgtatc aacccctctt ccttccacca ctgaaaaaac tcctgcttcg     180

gttgaaaagg ttacagaatt atctgaagaa aagcctctag aggaaaagcc yatagaggag     240

aagcctttag agggctcagt tggtagagat gctcagctag cacgagttca aactgagaag     300

agratttcac tgatcaaagc atgggaagaa agtgaaaaat gcaaggccga aaacaaggca     360

cacaagaaaa ttgctgctat cgaggcatgg gagaacagca agaaagcatc tcttgaggca     420

gagctgaara agatcgagga aagcttggaa aaraagaagg cagaatatgc agaaaaaatg     480

aagaacaaag tggctatgac tcacaaggaa gcagaggaga agaaggctac ggttgaagcc     540

aaacgtgctg aagatgttct gaaagcggaa gagatggctg ctaagtatcg cgccactgga     600

aagcccccca agaaacttct tggtttgttc taa                                  633

<210>  45
<211>  210
<212>  PRT
<213>  Artificial Sequence

<220>
<223>  consensus of protein derived from haplotypes 2 to 5

<220>
<221>  MISC_FEATURE
<222>  (11)..(11)
<223>  Xaa is a threonine (T) or an isoleucine (I)

<400>  45

Met Ala Thr Glu Glu Ala Asn Lys Val Val Xaa Glu Thr Pro Ala Pro
1               5                   10                  15

Pro Val Thr Pro Pro Gln Glu Pro Pro Ala Thr Val Ser Glu Thr Arg
            20                  25                  30

Glu Ser Pro Val Ala Val Glu Glu Lys Ser Leu Val Pro Val Ser Thr
        35                  40                  45

Pro Leu Pro Ser Thr Thr Glu Lys Thr Pro Ala Ser Val Glu Lys Val
    50                  55                  60

Thr Glu Leu Ser Glu Glu Lys Pro Leu Glu Glu Lys Pro Ile Glu Glu
65                  70                  75                  80

Lys Pro Leu Glu Gly Ser Val Gly Arg Asp Ala Gln Leu Ala Arg Val
                85                  90                  95

Gln Thr Glu Lys Arg Ile Ser Leu Ile Lys Ala Trp Glu Glu Ser Glu
            100                 105                 110

Lys Cys Lys Ala Glu Asn Lys Ala His Lys Lys Ile Ala Ala Ile Glu
        115                 120                 125

Ala Trp Glu Asn Ser Lys Lys Ala Ser Leu Glu Ala Glu Leu Lys Lys
    130                 135                 140

Ile Glu Glu Ser Leu Glu Lys Lys Lys Ala Glu Tyr Ala Glu Lys Met
145                 150                 155                 160

Lys Asn Lys Val Ala Met Thr His Lys Glu Ala Glu Glu Lys Lys Ala
                165                 170                 175

Thr Val Glu Ala Lys Arg Ala Glu Asp Val Leu Lys Ala Glu Glu Met
            180                 185                 190

Ala Ala Lys Tyr Arg Ala Thr Gly Lys Pro Pro Lys Lys Leu Leu Gly
        195                 200                 205

Leu Phe
    210

## Description

### FIELD OF THE INVENTION

The present invention relates to a nucleic acid molecule which encodes a polypeptide, which is involved in Rz2 resistance gene mediated resistance against *Beet necrotic yellow vein virus* (BNYVV) in a plant in which the polypeptide is expressed as well as to a nucleic acid molecule which encodes a non-functional allele of the polypeptide, which interferes with said resistance against BNYVV in a plant in which the polypeptide is expressed. The present invention further relates to the corresponding polypeptides encoded by the nucleic acid molecules according to the invention. Furthermore, the invention relates to a plant, plant cell, plant organ, plant tissue, plant part, or a seed or descendant of a plant, which comprises any one of the nucleic acid molecules or portions thereof as an endogenous gene or as a transgene. Furthermore, the present invention also encompasses methods for conferring, restoring or increasing the resistance to BNYVV in a plant of the species *Beta vulgaris* comprising the Rz2 resistance gene, as well as methods for producing or identifying and possibly selecting a BNYVV-resistant plant. The present invention also encompasses methods for cultivating plants of the species *Beta vulgaris* counteracting an infestation of the cultivated plants with BNYVV as well as to oligonucleotide probes and primers for specific hybridization with any one of the nucleic acid molecules according to the invention.

### BACKGROUND OF THE INVENTION

Rhizomania is the most economically important sugar beet disease worldwide, which can cause yield losses of 50% or more. The disease, also called "root madness", is caused by the *Beet necrotic yellow vein virus* (BNYVV) and transmitted by the soil-borne fungus *Polymyxa betae.* BNYVV infection manifests itself in an increased proliferation of the thin roots and lateral roots and in the formation of a strongly reduced root body with reduced sugar content. Infected plants show a reduced water absorption and are therefore more sensitive to drought stress. When the infection spreads to the entire plant, the leaf veins become yellow, with necrotic lesions and yellow spots on the leaves. Since curative control of the disease is not possible, as with other viral diseases, damage can only be prevented by cultivating resistant varieties. Three major genes are currently under investigation against rhizomania: Rz1 (also known as "Holly"), Rz2 and Rz3.

The Rz2 resistance gene, which was identified and described in international patent application WO 2014/202044 A1 originates from the *Beta maritima* accession WB42 and is known to provide an elevated resistance to rhizomania in comparison to Rz1 when expressed in several varieties of sugar beet. Furthermore, it is known that Rz3 from *Beta maritima* accession WB41 has been identified, which is most probably the same or a very similar allelic variant of Rz2.

However, in the course of development of new sugar beet varieties, it has been observed that certain genetic backgrounds lead to a weakening or loss of resistance, although the resistant allele of Rz2 or Rz3 is present. So far, nothing is known about the molecular genetic background leading to the observed effect. However, in order to make the resistance effect mediated by Rz2 or Rz3, respectively, usable in all genetic backgrounds, there is a high demand among plant breeders of the genus *Beta* to prevent the observed reduction or loss of resistance.

In particular, the aim is to identify the mechanism behind the observed reduction or loss of resistance and to use it in order to establish sugar beet lines which show a stable resistance to rhizomania based on Rz2 and Rz3, respectively. According to the invention, this aim is achieved via the embodiments characterized in the claims and in the specification.

### SUMMARY OF THE INVENTION

The present invention relates to a nucleic acid molecule which encodes a polypeptide, which is involved in resistance against *Beet necrotic yellow vein virus* (BNYVV) mediated by the Rz2 resistance gene in a plant as well as to a nucleic acid molecule which encodes a non-functional allele of the polypeptide, which interferes with said resistance against BNYVV in which the polypeptide is expressed. The polypeptide which is encoded by any one of the nucleic acid molecules is thereby produced in the plant.

Furthermore, the invention relates to a plant, plant cell, plant organ, plant tissue, plant part, or a seed or descendant of a plant, which endogenously or transgenically comprises any one of the nucleic acid molecules or portions thereof. According to a specific optional embodiment, those plants and their components that have been obtained via an essentially biological process are exempted.

Methods for conferring, restoring or increasing the resistance to BNYVV in a plant of the species *Beta vulgaris* comprising the Rz2 resistance gene, as well as methods for producing or identifying and possibly selecting a BNYVV-resistant plant, are likewise encompassed by the present invention. The present invention also encompasses methods for cultivating plants of the species *Beta vulgaris* counteracting an infestation of the cultivated plants with BNYVV as well as oligonucleotide probes and primers for specific hybridization with any one of the nucleic acid molecules according to the invention.

The present invention therefore relates to the embodiments that are listed in the following points [1] through [29] and illustrated in the Examples and Figures.
[1] A nucleic acid molecule encoding a polypeptide which is involved in resistance against *Beet necrotic yellow vein virus* (BNYVV) mediated by the Rz2 resistance gene in a plant in which the polypeptide is expressed, wherein the nucleic acid molecule comprises a nucleotide sequence which is selected from the group consisting of:
   (a) a nucleotide sequence that encodes a polypeptide having the amino acid sequence according to SEQ ID No. 45;
   (b) a nucleotide sequence that comprises the coding DNA sequence according to SEQ ID No. 44
   (c) a nucleotide sequence that hybridizes with a complementary sequence of the nucleotide sequence according to (a) or (b) under stringent conditions;
   (d) a nucleotide sequence that encodes a polypeptide which, via substitution, deletion, and/or addition of one or more amino acids of the amino acid sequence, differs from a polypeptide that is encoded by the nucleotide sequence according to (a) or (b);
   (e) a nucleotide sequence that encodes a polypeptide which has an amino acid sequence that is at least 70% identical to the amino acid sequence according to SEQ ID No. 45; and
   (f) a nucleotide sequence that comprises the coding DNA sequence which is at least 70% identical to the DNA sequence according to SEQ ID No. 44,
   wherein the amino acid sequence of the encoded polypeptide has a proline (P) at position 25 with reference to SEQ ID No. 45, a proline (P) at position 72 with reference to SEQ ID No. 45 and/or a valine (V) at position 186 with reference to SEQ ID No. 45, or wherein the nucleic acid of the coding DNA sequence has a cytosine (c) at position 73 with reference to SEQ ID No. 44, a cytosine (c) at position 214 with reference to SEQ ID No. 44, and/or a guanine (g) at position 556 with reference to SEQ ID No. 44.
[2] The nucleic acid molecule according to [1], wherein the polypeptide has the amino acid sequence of SEQ ID No. 8, 12, 16, or 20, or the nucleotide sequence that encodes said polypeptide is SEQ ID No. 5, 6, 7, 9, 10, 11, 13, 14, 15, 17, 18 or 19, or the coding DNA sequence is a nucleotide sequence according to SEQ ID No. 7, 11, 15, or 19.
[3] A nucleic acid molecule which specifically hybridizes to the nucleic acid molecule according to [1] or [2] and encodes a non-functional allele of the polypeptide, which interferes with resistance against BNYVV mediated by the Rz2 resistance gene in a plant in which the polypeptide is expressed, wherein the nucleic acid molecule preferably comprises a nucleotide sequence which is selected from
   (a) a nucleotide sequence that encodes a polypeptide having an amino acid sequence according to SEQ ID No. 4 or a polypeptide having an amino acid sequence that is at least 70% identical to SEQ ID No. 4, wherein the polypeptide comprises an amino acid sequence having at least one amino acid substitution due to one or more mutations in the nucleotide sequence, preferably wherein proline (P) at position 25 with reference to SEQ ID No. 45, proline (P) at position 72 with reference to SEQ ID No. 45, and/or valine (V) at position 186 with reference to SEQ ID No. 45 is substituted with another amino acid; and/or
   (b) a nucleotide sequence that comprises the coding DNA sequence according to SEQ ID No. 3, that comprises a coding DNA sequence which is at least 70% identical to the DNA sequence according to SEQ ID No. 3, or that hybridizes with a complementary sequence of SEQ ID No. 3 under stringent conditions, wherein the nucleotide sequence comprises at least one nucleic acid substitution due to at least one mutation leading to an amino acid substitution, preferably wherein one or more nucleotides are substituted at positions 73-75 with reference to SEQ ID No. 44, at positions 214-216 with reference to SEQ ID No. 44 and/or at positions 556-558 with reference to SEQ ID No. 44.
[4] The nucleic acid molecule according to [3], characterized in that the encoded polypeptide comprises an amino acid sequence having
   (i) alanine (A) instead of proline (P) at position 25 with reference to SEQ ID No. 45,
   (ii) threonine (T) instead of proline (P) at position 72 with reference to SEQ ID No. 45, and/or
   (iii) isoleucine (I) instead of valine (V) at position 186 with reference to SEQ ID No. 45.
[5] The nucleic acid molecule according to [3] or [4], characterized in that the nucleic acid molecule comprises a DNA sequence having
   (i) guanine (g) instead of cytosine (c) at position 73 with reference to SEQ ID No. 44,
   (ii) adenine (a) instead of cytosine (c) at position 214 with reference to SEQ ID No. 44, and/or
   (iii) adenine (a) instead of guanine (g) at position 556 with reference to SEQ ID No. 44.
[6] The nucleic acid molecule according to any one of [3] to [5], characterized in that the nucleic acid molecule comprises a coding DNA sequence having in addition
   (i) adenine (a) instead of guanine (g) at that position 72 with reference to SEQ ID No. 44, and/or
   (ii) adenine (a) instead of guanine (g) at position 111 with reference to SEQ ID No. 44.
[7] A polypeptide which is encoded by the nucleic acid molecule according to [1] or [2] or any one of [3] to [6].
[8] A vector or expression cassette which comprises the nucleic acid molecule according to [1] or [2] or any one of [3] to [6], preferably wherein the nucleic acid molecule according to [1] or [2] or any one of [3] to [6] is operably linked to a heterologous regulatory element, preferably wherein the regulatory element is a promoter.
[9] A cell which comprises the nucleic acid molecule according to [1] or [2] or any one of [3] to [6], a polypeptide according to [7], or a vector or expression cassette according to [8].
[10] A plant or a portion thereof comprising the nucleic acid molecule according to [1] or [2] endogenously or transgenically or the vector or the expression cassette which comprises the nucleic acid molecule according to [1] or [2] as defined in [8] as transgene.
[11] A plant or a portion thereof according to [10], characterized by comprising additionally the Rz2 resistance gene mediating BNYVV resistance endogenously or transgenically.
[12] Seed or a descendant of the plant according to [10] or [11], wherein the seed or the descendant endogenously or transgenically comprises the nucleic acid molecule according to [1] or [2], or the vector or the expression cassette according to [8].
[13] Seed according to [12], which has been technically treated, whereby the technical treatment is selected from the group consisting of:
   (a) Polishing,
   (b) Dressing, preferably pelleting,
   (c) Incrustation, and
   (d) Colouring.
[14] Oligonucleotide or a pair of oligonucleotides of at least 15, 16, 17, 18, 19, or 20, preferably at least 21, 22, 23, 24, or 25, particularly preferably at least 30, 35, 40, 45, or 50, and particularly preferably at least 100, 200, 300, or 500 nucleotides in length, wherein the oligonucleotide or the pair of oligonucleotides
   (i) specifically hybridizes to a nucleotide sequence as defined in any one of [3] to [6], but not to a nucleotide sequence as defined in [1] or [2];
   (ii) specifically hybridizes to a nucleotide sequence as defined in [1] or [2], but not to the nucleotide sequence as defined in any one of [3] to [6];
   (iii) specifically hybridizes to a region in the *Beta vulgaris* genome that, in *Beta vulgaris,* cosegregates with the nucleic acid molecule according to any one of [3] to [6] and/or with the nucleic acid molecule according to any one of [3] to [6] together with the Rz2 gene; and/or
   (iv) specifically hybridizes to a region in the *Beta vulgaris* genome that, in *Beta vulgaris,* cosegregates with the nucleic acid molecule according to [1] or [2] and/or with the nucleic acid molecule according to [1] or [2] together with the Rz2 gene.
[15] The oligonucleotide or pair of oligonucleotides according to [14], wherein the oligonucleotide or pair of oligonucleotides is selected from the group consisting of SEQ ID NOs: 24 to 40.
[16] A method for conferring, restoring or increasing the resistance to BNYVV in a plant of the species *Beta vulgaris* comprising the Rz2 resistance gene mediating BNYVV resistance endogenously or transgenically, including the following steps:
   (i) integration or introgression of the nucleic acid molecule according to [1] or [2] by means of homology-directed repair or homologous recombination - preferably, promoted by site-directed nuclease - into the genome of at least one cell of a plant of the species *Beta vulgaris,* and optional regeneration of a plant from the plant cell; or
   (ii) increasing the expression of the nucleic acid molecule according to [1] or [2] in the plant - preferably, via modification of the native promoter or via fusion of the nucleic acid molecule with a heterologous promoter that exhibits a higher activity in comparison to the native promoter - in particular, after BNYVV infection; or
   (iii) increase in the activity and/or stability of the functional allele of the polypeptide which is encoded by the nucleic acid molecule according to [1] or [2] as defined in [7] via modification of the nucleotide sequence according to [1] or [2];
   (iv) transformation of a plant cell with the nucleic acid molecule according to [1] or [2], or the vector or the expression cassette which comprises the nucleic acid molecule according to [1] or [2] as defined in [8,] and optionally regeneration of a transgenic plant from the transformed plant cell; or
   (v) mutagenesis of a plant or plant cells comprising the nucleic acid molecule according to any one of [3] to [6], and screening/selecting for plants or plant cells, respectively, in which a functional allele of the polypeptide encoded by the nucleic acid molecule according to [1] or [2] has been restored and/or the expression of the non-functional polypeptide has been abolished.
[17] A method for producing a BNYVV-resistant plant comprising the nucleic acid molecule according to [1] or [2] as defined in [10], including the following steps:
   (I) provision of a plant of the genus *Beta* or a plant cell of the genus *Beta* comprising the Rz2 resistance gene mediating BNYVV resistance endogenously or transgenically; and
   (IIa) transformation of the plant cell of (I) with the nucleic acid molecule according to [1] or [2], or the vector or the expression cassette which comprises the nucleic acid molecule according to [1] or [2] as defined in [8]; and
   (IIb) regeneration of a transgenic plant from the transformed plant cell; or
   (IIIa) introduction of a site-directed nuclease or nickase and optionally a repair matrix, or a site-directed base editor into the plant cell, wherein the site-directed nuclease is able to generate at least one single-strand break of the DNA or at least one double-strand break of the DNA at a predetermined location in the genome of the cell and the repair matrix comprises the nucleic acid molecule according to [1] or [2], or wherein the site-directed base editor is able to generate at least one single-strand break of the DNA at a predetermined location in the genome of the cell and to convert at least one nucleobase; and
   (IIIb) optionally, cultivation of the cell from (IIIa) under conditions that allow modification of the genome at the predetermined location, selected from
      a) a replacement of at least one nucleotide;
      b) a deletion of at least one nucleotide;
      c) an insertion of at least one nucleotide;
      d) any combination of a) - c),
      optionally by homology-directed repair or homologous recombination,
      wherein the nucleic acid molecule is integrated into the genome of the plant; and
   (IIIc) regeneration of a plant from the cell modified in (IIIb); or
   (IVa) crossing of the plant of (I) with the plant according to any one of [10] to [12]; and
   (IVb) identifying and selecting a plant comprising the Rz2 resistance gene mediating BNYVV endogenously or transgenically and the nucleic acid molecule according to [1] or [2] endogenously or transgenically, wherein the Rz2 resistance gene and/or the nucleic acid molecule according to [1] or [2] is present homozygously in the genome of the plant.
[18] The method according to [17], characterized in that the predetermined location is in the nucleic acid molecule according to any one of [3] to [6] or a position which is at most 10.000 base pairs upstream or downstream away from the nucleic acid molecule according to any one of [3] to [6].
[19] A method for identifying a plant of the species *Beta vulgaris,* a portion thereof or a seed thereof comprising the nucleic acid molecule according to [1] or [2], wherein the plant is capable of exhibiting resistance against BNYVV mediated by Rz2, if present in the genome of the plant, comprising:
   (i) detection of the presence and/or expression of the nucleic acid molecule according to [1] or [2], or the presence of the polypeptide which is encoded by the nucleic acid molecule according to [1] or [2] as defined in [7] in the plant, the portion thereof or the seed thereof; and/or
   (ii) detection of at least one marker locus in the nucleotide sequence of the nucleic acid molecule according to [1] or [2] or in one or more cosegregating regions thereof; and
   (iii) identification and optionally selection of the plant of the species *Beta vulgaris,* the portion thereof or the seed thereof comprising the nucleic acid molecule according to [1] or [2], which is capable of exhibiting resistance against BNYVV mediated by Rz2, if present in the genome of the plant, based on the detection of (i) and/or (ii).
[20] A method for identifying a plant of the species *Beta vulgaris,* a portion thereof or a seed thereof comprising the nucleic acid molecule according to any one of [3] to [6], comprising:
   (i) detection of the presence and/or expression of the nucleic acid molecule according to any one of [3] to [6], or the presence of the polypeptide which is encoded by the nucleic acid molecule according to any one of [3] to [6] as defined in [7] in the plant, the portion thereof or the seed thereof; and/or
   (ii) detection of at least one marker locus in the nucleotide sequence of the nucleic acid molecule according to any one of [3] to [6] or in one or more cosegregating regions thereof; and
   (iii) identification and optionally selection of the plant of the species *Beta vulgaris,* the portion thereof or the seed thereof comprising the nucleic acid molecule according to any one of [3] to [6], based on the detection of (i) and/or (ii).
[21] The method according to [19] or [20], wherein the detection of the at least one marker locus in the nucleotide sequence of the nucleic acid molecule according to [1] or [2] or in the nucleotide sequence of the nucleic acid molecule according to any one of [3] to [6] comprises the use of the oligonucleotide or the pair of oligonucleotides according to claim 14 or 15, and wherein the one or more cosegregating regions are genomic intervals in *Beta vulgaris* on chromosome 3 which comprise and are flanked by marker loci detectable by means of
   (i) the markers *s3e5985s01* (SEQ ID No. 33) and *s3p4348s01* (SEQ ID No. 27), and/or
   (ii) the markers *s3p4351s01* (SEQ ID No. 29) and *s3e2247xxx* (SEQ ID No. 40),
   preferably by means of
   (i) the markers *sxh1002s02* (SEQ ID No. 34) and *s3p4348s01* (SEQ ID No. 27), and/or
   (ii) the markers *s3p4351s01* (SEQ ID No. 29) and *sxn1017s02* (SEQ ID No. 39),
   more preferably means of
   (i) the markers *sxh2499s01* (SEQ ID No. 35) and *s3p4348s01* (SEQ ID No. 27), and/or
   (ii) the markers *sxi0698s01* (SEQ ID No. 36) and *s3p4348s01* (SEQ ID No. 27), and/or
   (iii) the markers *s3p4351s01* (SEQ ID No. 29) and *sxe7357s01* (SEQ ID No. 38), and/or
   (iv) the markers *s3p4351s01* (SEQ ID No. 29) and *sxh8485s01* (SEQ ID No. 37), and/or
   (v) the markers *s3p4351s01* (SEQ ID No. 29) and *s3e5853s01* (SEQ ID No. 26), and/or
   (vi) the markers *s3p4351s01* (SEQ ID No. 29) and *s3e5800s01* (SEQ ID No. 32), and/or
   (vii) the markers *s3p4351s01* (SEQ ID No. 29) and *s3e4913xxx* (SEQ ID No. 31), and/or
   (viii) the markers *s3p4351s01* (SEQ ID No. 29) and *s3e5782s01* (SEQ ID No. 30).
[22] The method according to [19] or [21] additionally comprising
   (A) detection of the presence and/or expression of the Rz2 gene or the presence of the polypeptide encoded by the Rz2 gene in the plant, the portion thereof or the seed thereof; and/or
   (B) detection of at least one marker locus in the Rz2 gene or in one or more regions cosegregating with the Rz2 gene, preferably cosegregating with a chromosomal interval comprising the nucleic acid molecule according to [1] or [2] and the Rz2 gene;
   wherein in (iii) of [19] the identified and optionally selected plant, the portion thereof or the seed thereof comprises the nucleic acid molecule according to [1] or [2], and the Rz2 gene, based on the detection of (i) and/or (ii) of [19] and detection of (B).
[23] The method according to [22], wherein the at least one marker locus in the nucleotide sequence of the Rz2 gene is detectable by means of the marker *s3e5853s01* (SEQ ID No. 26) and wherein the one or more regions cosegregating with the Rz2 gene are genomic intervals *in Beta vulgaris* on chromosome 3 which comprise and are flanked by marker loci detectable by means of
   (i) the markers *s3p4351s01* (SEQ ID No. 29) and *s3e5853s01* (SEQ ID No. 26), and/or
   (ii) the markers *s3e5853s01* (SEQ ID No. 26) and *s3e2247xxx* (SEQ ID No. 40),
   preferably by means of
   (i) the markers *s3p4351s01* (SEQ ID No. 29) and *s3e5853s01* (SEQ ID No. 26), and/or
   (ii) the markers *s3e5853s01* (SEQ ID No. 26) and *sxn1017s02* (SEQ ID No. 39),
   more preferably means of
   (i) the markers *s3p4351s01* (SEQ ID No. 29) and *s3e5853s01* (SEQ ID No. 26), and/or
   (ii) the markers *s3e5853s01* (SEQ ID No. 33) and *sxe7357s01* (SEQ ID No. 38), and/or
   (iii) the markers *s3e5853s01* (SEQ ID No. 26) and *sxh8485s01* (SEQ ID No. 37).
[24] The method according to [22], wherein the in one or more regions cosegregating with the chromosomal interval comprising the nucleic acid molecule according to [1] or [2] and the Rz2 gene are genomic intervals *in Beta vulgaris* on chromosome 3 which comprise and are flanked by marker loci detectable by means of
   (i) the markers *s3e5985s01* (SEQ ID No. 33) and *s3p4348s01* (SEQ ID No. 27), and/or
   (ii) the markers *s3e5853s01* (SEQ ID No. 26) and *s3e2247xxx* (SEQ ID No. 40),
   preferably by means of
   (i) the markers *sxh1002s02* (SEQ ID No. 34) and *s3p4348s01* (SEQ ID No. 27), and/or
   (ii) the markers *s3e5853s01* (SEQ ID No. 26) and *sxn1017s02* (SEQ ID No. 39),
   more preferably means of
   (i) the markers *sxh2499s01* (SEQ ID No. 35) and *s3p4348s01* (SEQ ID No. 27), and/or
   (ii) the markers *sxi0698s01* (SEQ ID No. 36) and *s3p4348s01* (SEQ ID No. 27), and/or
   (iii) the markers *s3e5853s01* (SEQ ID No. 26) and *sxe7357s01* (SEQ ID No. 38), and/or
   (iv) the markers *s3e5853s01* (SEQ ID No. 26) and *sxh8485s01* (SEQ ID No. 37).
[25] Use of the oligonucleotide or pair of oligonucleotides according to [14] or [15] for identifying and/or selecting a plant of the species *Beta vulgaris* capable of exhibiting resistance to BNYVV mediated by Rz2, if present in the genome of the plant, or which capability of exhibiting resistance to BNYVV mediated by Rz2, if present in the genome of the plant, is impaired.
[26] A method for cultivation of plants of the species *Beta vulgaris,* including
   (i) planting seedlings of the plant according to [10] or [11] or sowing seeds according to [12] or [13], and
   (ii) grown the plants from the seedlings or seeds, and
   (iii) optionally, harvest root beets from the grown plants;
   wherein the method counteracts an infestation of the cultivated plants with BNYVV.
[27] Use of the nucleic acid molecule according to [1] or [2] or the nucleic acid molecule according to any one of [3] to [6], the polypeptide according to [7], the vector or expression cassette according to [8], and/or the cell according to [9] for the identification of nucleic acid molecules which are involved in Rz2 mediated resistance to BNYVV.
[28] Use of the nucleic acid molecule according to [1] or [2] or the nucleic acid molecule according to any one of [3] to [6], the polypeptide according to [7] and/or the vector or expression cassette according to [8] for the production of BNYVV-resistant plants, preferably of plants of the species *Beta vulgaris,* more preferably of plants of *Beta vulgaris* ssp. *vulgaris* var. *vulgaris, Beta vulgaris* ssp. *vulgaris* var. *conditiva,* oder *Beta vulgaris* ssp. *vulgaris* var. *crassa*/*alba.*
[29] The nucleic acid molecule according to [1] to [6], the plant or a portion thereof according to [11], the oligonucleotide or a pair of oligonucleotides according to [14] or [15], the methods according to any one of [16] to [19] and [22] to [24], and the use according to [25] and [27], wherein the Rz2 gene or the Rz2 resistance gene is a nucleic acid molecule comprises a nucleotide sequence selected from
   a) a nucleotide sequence that encodes a polypeptide with an amino acid sequence according to SEQ ID NO. 22 or SEQ ID NO. 23,
   b) a nucleotide sequence comprising the coding sequence of the DNA sequence according to SEQ ID NO. 21,
   c) a nucleotide sequence that encodes a polypeptide derived by substitution, deletion and/or addition of an amino acid of the amino acid sequence encoded by the nucleotide sequence according to a) or b), from a polypeptide encoded by the nucleotide sequence according to a) or b),
   d) a nucleotide sequence that encodes a polypeptide having an amino acid sequence, which is at least 80% identical to an amino acid sequence encoded by the nucleotide sequence according to a) or b), or
   e) a nucleotide sequence which encodes at least the amino acid positions 168-227 of SEQ ID NO. 22 and at least the amino acid positions 591-613 of SEQ ID NO. 22 and at least the amino acid positions 1013-1072 of SEQ ID NO. 22 or which encodes at least the amino acid positions 182-241 of SEQ ID NO. 23, at least the amino acid positions 605-627 of SEQ ID NO. 23 and at least the amino acid positions 1027-1086 of SEQ ID NO. 23.

First, some of the terms used in this application are explained in detail in the following:
In conjunction with the specification of a length of a nucleotide sequence, the term, "approximately," means a deviation by +/- 200 base pairs - preferably, by +/- 100 base pairs, and, particularly preferably, by +/- 50 base pairs.

A "plant of the genus Beta" belongs to the amaranth family (Amaranthaceae). Numbering among these plants are plants of the species *Beta macrocarpa, Beta vulgaris, Beta lomatogona, Beta macrorhiza, Beta corolliflora, Beta trigyna,* and *Beta nana.* A plant of the species *Beta vulgaris* is, in particular, a plant of the subspecies *Beta vulgaris* subsp. *vulgaris.* For example, numbering among these are *Beta vulgaris* subsp. *vulgaris* var. *altissima* (sugar beet in a narrower sense), *Beta vulgaris* ssp. *vulgaris* var. *vulgaris* (chard), *Beta vulgaris* ssp. *vulgaris* var. *conditiva* (beetroot / red beet), *Beta vulgaris* ssp. *vulgaris* var. *crassa*/*alba* (fodder beet).

A "functional fragment" of a nucleotide sequence means a segment of a nucleotide sequence which has a functionality identical or comparable to that of the complete nucleotide sequence from which the functional fragment originates. As such, the functional fragment may possess a nucleotide sequence which is identical or homologous to the total nucleotide sequence over a length of at least 50%, 55%, 60%, 65%, 70%, 75%, 80%, 85%, 90%, 92%, 94% 96%, 97%, 98%, or 99%. This also explicitly encompasses the range of 90 - 100%. Furthermore, a "functional fragment" of a nucleotide sequence may also mean a segment of a nucleotide sequence which modifies the functionality of the entire nucleotide sequence, e.g., in the course of post-transcriptional or transcriptional gene silencing. As such, the functional fragment of a nucleotide sequence may comprise at least 14, 15, 16, 17, 18, 19, 20, 21, 22, 23, 24, or 25 - preferably, at least 30, 35, 40, 45, 50, 60, 70, 80, 90, 100, 120, or 140, and, particularly preferably, at least 160, 180, 200, 250, 300, 350, 400, 450, 500, 600, 700, 800, 900, or 1,000 - successive nucleotides of the total nucleotide sequence. This also explicitly encompasses the range of 21 to 50 nucleotides.

A "functional part" of a protein means a segment of a protein, or a section of the amino acid sequence, that encodes the protein, wherein the segment may exert functionality identical or comparable to that of the entire protein in a plant cell. Over a length of at least 50%, 55%, 60%, 65%, 70%, 75%, 80%, 85%, 90%, 92%, 94% 96%, 97%, 98%, or 99%, a functional part of a protein has an amino acid sequence that is identical or, with consideration of conservative and semi-conservative amino acid exchanges, similar to the protein from which the functional part originates.

The term, "heterologous," means that the introduced polynucleotide originates from a cell or an organism with a different genetic background, of the same species or a different species, or is homologous to the prokaryotic or eukaryotic host cell, but is then located in a different genetic environment and thus differs from a corresponding polynucleotide that is possibly naturally present. A heterologous polynucleotide may be present in addition to a corresponding endogenous gene.

In the sense of the invention, what is understood by a "homolog" is a protein of the same phylogenetic origin; what is understood by an "analog" is a protein which exerts the same function, but has a different phylogenetic origin; what is understood by an "ortholog" is a protein from a different species that exerts the same function; and what is understood by a "paralog" is a protein that has appeared within a species due to duplication, wherein this copy either retains the same protein function, alters its expression template, but not the function, changes its protein function, or divides up the original gene function between both copies.

What is to be understood by "hybridizing" or "hybridization" is a process in which a single-stranded nucleic acid molecule binds to a nucleic acid strand that is complementary to the greatest possible extent, i.e., forms base pairs with this. Standard methods for hybridization are described in, for example, Sambrook et al., Molecular Cloning: A Laboratory Manual, 3rd ed., Cold Spring Harbor Laboratory Press, Cold Spring Harbor, NY, 2001. What is preferably understood by this is that at least 60% - more preferably, at least 65%, 70%, 75%, 80%, or 85%, and, particularly preferably, 90%, 91%, 92%, 93%, 94%, 95%, 96%, 97%, 98%, or 99% - of the bases of the nucleic acid molecule form a base pairing with the nucleic acid strand that is complementary to the greatest possible extent. The possibility of such an annealing depends upon the stringency of the hybridization conditions. The term, "stringency," relates to the hybridization conditions. High stringency is present when a base pairing is made more difficult; low stringency is present if a base pairing is made easier. For example, the stringency of the hybridization conditions depends upon the salt concentration or ionic strength and the temperature. In general, the stringency may be increased by increasing the temperature and/or decreasing the salt content. What are to be understood by "stringent hybridization conditions" are those conditions given which a hybridization predominantly occurs only between homologous nucleic acid molecules. The term, "hybridization conditions," thereby relates not only to the conditions prevailing in the actual addition of the nucleic acids, but also to the conditions prevailing in the following washing steps. For example, stringent hybridization conditions are conditions under which, predominantly, only those nucleic acid molecules hybridize that have at least 70% - preferably, at least 75%, at least 80%, at least 85%, at least 90%, or at least 95% - sequence identity. Stringent hybridization conditions are, for example: hybridization in 4 x SSC at 65 °C, and subsequent repeated washing in 0.1 x SSC at 65 °C for approximately 1 hour in total. A hybridization preferably occurs under stringent conditions.

In relation to a nucleic acid in the form of a double-stranded DNA, "complementary" nucleotide sequence means that the second DNA strand complementary to the first DNA strand has the nucleotides that correspond to the bases of the first strand, in accordance with the base pairing rules. A complementary sequence is, preferably, entirely complementary to the counter-sequence, and thus preferably has the same length.

What is understood by an "isolated nucleic acid molecule" is a nucleic acid molecule extracted from its natural or original environment. The term also encompasses a synthetically-produced nucleic acid molecule. What is understood by an "isolated polypeptide" is a polypeptide extracted from its natural or original environment. The term also encompasses a synthetically-produced polypeptide.

A "molecular marker" is a nucleic acid that is polymorphic in a plant population and is used as a reference or orientation point. A marker for the detection of a recombination event should be suitable for monitoring differences or polymorphisms within a plant population. Such a marker is thus able to detect and differentiate between various allelic states (alleles). The term, "molecular marker," also relates to nucleotide sequences which are complementary or at least largely complementary or homologous to genomic sequences - for example, nucleic acids which are used as probes or primers. These differences at the DNA level are to be found as markers and are, for example, polynucleotide sequence differences, e.g., SSR's (*simple sequence repeats*), RFLP's (*restriction fragment length polymorphisms*), FLP's (*fragment length polymorphisms*) or SNP's (*single nucleotide polymorphisms*)*.* The markers may be derived from genomic or expressed nucleic acids, e.g., spliced RNA, cDNA, or EST's, and may also relate to nucleic acids that are used as probes or primer pairs and as such are suitable for amplifying a sequence fragment using PCR-based methods. Markers that describe genetic polymorphisms (between parts of a population) may be detected using well-established methods from the prior art (An Introduction to Genetic Analysis, 7th edition, Griffiths, Miller, Suzuki, et al., 2000). For example, among these are DNA sequencing, PCR-based, sequence-specific amplification, verification of RFLP's, verification of polynucleotide polymorphisms by means of allele-specific hybridization (ASH), detection of amplified variable sequences of the plant genome, detection of a 3SR (*self-sustained sequence replication*)*,* detection of SSR's, SNP's, RFLP's, or AFLP's (*amplified fragment length polymorphisms*)*.* Furthermore, the methods for detection of EST's (*expressed sequence tags*) and SSR markers derived from EST sequences and RAPD (*randomly amplified polymorphic DNA*) are also known. Depending upon the context, the term, "marker," in the description may also mean a specific chromosome position in the genome of a species where a specific marker (SNP, for example) may be found.

Markers also include synthetic oligonucleotides that may be connected with one or more detection molecules, wherein the detection molecules may be used for a detection reaction or the generation of a signal within the scope of a verification method. Synthetic oligonucleotides also include labeled primers. Synthetic oligonucleotides and labeled primers are artificial compounds, do not occur in nature, and cannot be isolated from nature. The production of such compounds is explained further below.

A "promoter" is a non-translated, regulatory DNA sequence, typically upstream of a coding region, which contains the binding point for the RNA polymerase and initiates the transcription of the DNA. A promoter additionally contains other elements that act as a regulator gene for gene expression (for example, cis-regulatory elements). A "core or minimal promoter" is a promoter that has the basic elements which are needed for transcription initiation (for example, TATA box and/or initiator).

A "pathogen" means an organism that, in interactions with a plant, leads to disease symptoms in one or more organs in the plant. For example, animal, fungal, bacterial, or viral organisms or oomycetes number among these pathogens.

What is to be understood by a "pathogenic infection" is the earliest point in time at which a pathogen interacts with a plant host tissue. In this sense, "infestation" means the occurrence of contact between pathogen and host. For example, the viral pathogen BNYVV is transmitted by the soil-borne fungus *Polymyxa betae.* Polymyxa forms spores which can survive in the soil for decades. The virus also survives in these spores. If these permanent spores lead to zoospores, the virus can germinate via these into cells of the plant host tissue and interact with the host (Esser (2000) Kryptogamen 1: Cyanobakterien Algen Pilze Flechten Praktikum und Lehrbuch. Springer-Verlag, Berlin, Heidelberg, 3rd edition).

Plant "organs" means, for example, leaves, shoot, stem, roots, hypocotyl, vegetative buds, meristems, embryos, anthers, ovula, seeds, or fruits. "Plant parts" include, but are not limited to, the shoot or the stalk, leaves, blossoms, inflorescence, roots, fruits, and seeds, as well as the pollen. The term, "plant parts," also means an association of multiple organs, *e.g.,* a blossom or a seed, or a part of an organ, e.g., a cross-section through the plant shoot. Plant "tissues" are, for example, callus tissue, storage tissue, meristematic tissue, leaf tissue, shoot tissue, root tissue, plant tumor tissue, or reproductive tissue, as well as the cambium, parenchyma, vascular tissue, sclerenchyma, and epidermis. However, the tissue is not limited to this listing. For example, what are to be understood by plant "cells" are, for example, isolated cells having a cell wall or aggregates thereof, or protoplasts.

The level of the resistance by way of example to BNYVV can be defined qualitatively in plants of the *Beta* genus by determination of "rating scores" (rating score schemes for plants of the *Beta* genus are known from the prior art, for example for sugar beet Mechelke (1997).

In conjunction with the present invention, the term, "regulatory sequence," relates to a nucleotide sequence which influences the specificity and/or the expression strength, e.g., in that the regulatory sequence confers a defined tissue specificity. Such a regulatory sequence may be located upstream of the transcription initiation point of a minimal promoter, but also downstream thereof, *e.g.,* in a transcribed, but not translated, leader sequence or within an intron.

The term, "resistance," is to be understood broadly and covers the range of the protection from a retardation up to a complete blocking of the development of the disease. One example of an important pathogen is the *Beet necrotic yellow vein virus* (BNYVV). A resistant plant cell of the invention or resistant plant of the invention preferably achieves a resistance to BNYVV. A resistance to a pathogen is to be equated to a resistance to the disease which this pathogen causes; for example, a resistance to BNYVV is also a resistance to "root madness" (Rhizomania) .

The "Rz2" resistance gene, which was identified and described in international patent application WO 2014/202044 A1 originates from the *Beta maritima* accession WB42 and is known to provide resistance to rhizomania when expressed in several varieties of sugar beet. Furthermore, it is known that Rz3 from the *Beta maritima* accession WB41 has been identified, which is most probably the same or a very similar allelic variant of Rz2. Thus, the Rz2 and Rz3 genes and respective proteins are substantially identical and the designation "Rz2" and "Rz3" can be used interchangeably.

"Remorins" are a family of plant-specific proteins containing a variable N-terminal region and conserved C-terminal domain and play a role in various biotic and abiotic stresses, including host-microbe interactions. The present invention distinguishes between a remorin gene, which encodes a functional remorin protein, *i.e.* a remorin protein which is involved in establishing Rz2 mediated BNYVV resistance in a plant in which the protein is expressed and a remorin gene which encodes a non-functional remorin protein, *i.e.* a remorin protein which interferes with Rz2 mediated BNYVV resistance in a plant in which the protein is expressed. In this context, the term "involved in" includes increasing the resistance as well as being essential for establishing the resistance against BNYVV in a plant additionally comprising the Rz2 resistance gene, in particular a high level of resistance against BNYVV. The term "non-functional" is used in terms of the inability to confer resistance in combination with the Rz2 gene.

Accordingly, the terms "functional form", "functional (remorin) protein", "functional (remorin) polypeptide", "functional (remorin) variant", "functional (remorin) allele", "functional (remorin) gene", and "functional nucleic acid molecule" of the present invention are used interchangeable in the present invention as well as the terms "non-functional form", "non-functional (remorin) protein", "non-functional (remorin) polypeptide", "non-functional (remorin) variant", "non-functional (remorin) allele", "non-functional (remorin) gene, and "non-functional nucleic acid molecule" of the present invention.

"Transgenic plant" relates to a plant into whose genome is integrated at least one polynucleotide. It may thereby be a heterologous polynucleotide. The polynucleotide is, preferably, stably integrated, which means that the integrated polynucleotide is stably preserved in the plant, is expressed, and also may be stably passed on to the descendants. The stable introduction of a polynucleotide into the genome of a plant also includes the integration into the genome of a plant of the preceding parental generation, wherein the polynucleotide may be stably passed on further. The term, "heterologous," means that the introduced polynucleotide originates from a cell or an organism with a different genetic background, of the same species or a different species, or is homologous to the prokaryotic or eukaryotic host cell, for example, but then is located in a different genetic environment and thus differs from a corresponding polynucleotide that is possibly naturally present. A heterologous polynucleotide may be present in addition to a corresponding endogenous gene.

"Raw material for industrial sugar production" means plant material which can be fed into a sugar production facility which is specialized in the extraction of sugar from sugar beets. Such raw material is typically the beet body (taproot) of the harvested sugar beet. To ensure the conformity with the extraction process the beet body needs to have sufficient mass, volume and a conical shape so that the raw material can be mechanically cut into shreds (beet strips). These beet strips maximize the surface area for sugar extraction and should have a low content of Sodium, Potassium and Nitrogen to allow an efficient extraction. After the extraction remaining beet pulp is pressed, dried and used as animal feed.

The "sucrose concentration" is expressed as percentage of the fresh weight of the root.

"Monogerm" means that a seed grows into exactly one plant whereas a polygerm or multigerm seed (also called "seed ball") grows into several plants.

"Bolting" is the production of a flowering stem (or stems) on a sugar beet in a natural attempt to produce seeds and reproduce. Bolting is triggered in sugar beet due to vernalization, i.e. a chilling stress which might occur e.g. during overwintering. However, commercially grown sugar beets are harvested before bolting as the bolting process and subsequent seed setting reduces the sucrose content in the beet body.

Designs and embodiments of the present invention are described by way of example with reference to the pending sequences and figures.

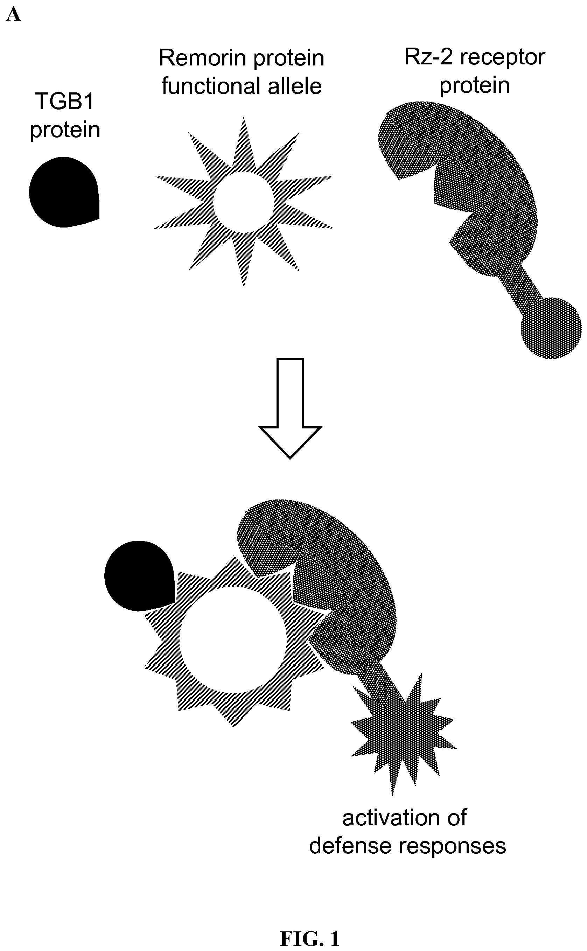

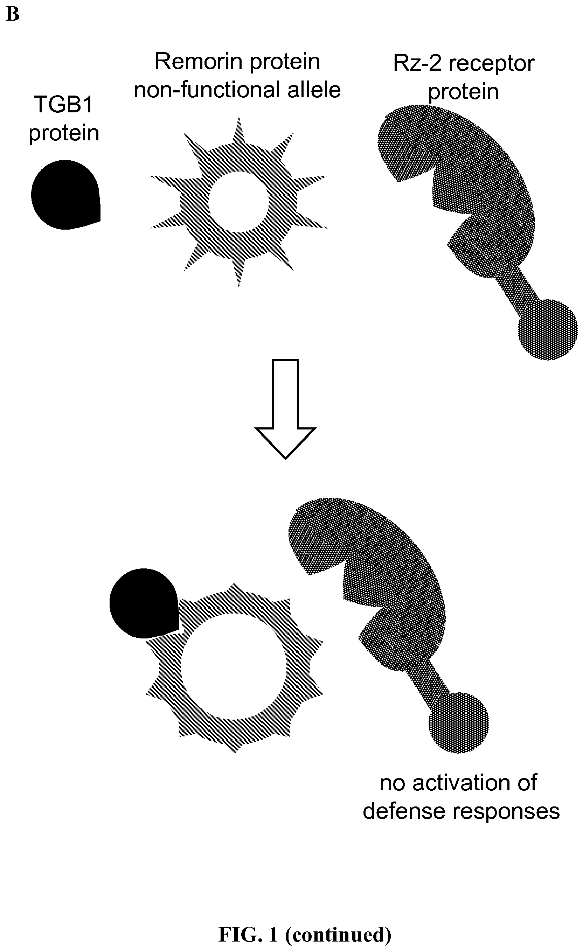

- **Fig. 1:**: Model depicting a possible mode of interaction between the TGB1 protein of *Beet necrotic yellow vein virus* (BNYVV), the Rz2 NBS-LRR receptor protein and (**A**) the functional variant of the remorin protein or (**B**) the non-functional variant of the remorin protein. In variant (**A**), the TGB1 protein binds to and modifies the functional remorin protein, which can be detected by the Rz2 NBS-LRR receptor protein of the resistance allele and thus, a resistance reaction is initiated. In variant (**B**), the Rz2 NBS-LRR receptor protein cannot detect the non-functional remorin protein modified by TGB1 and thus, no resistance reaction is initiated.

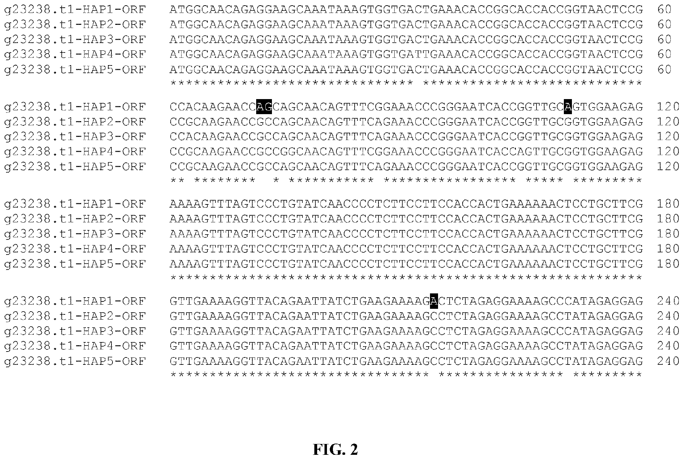

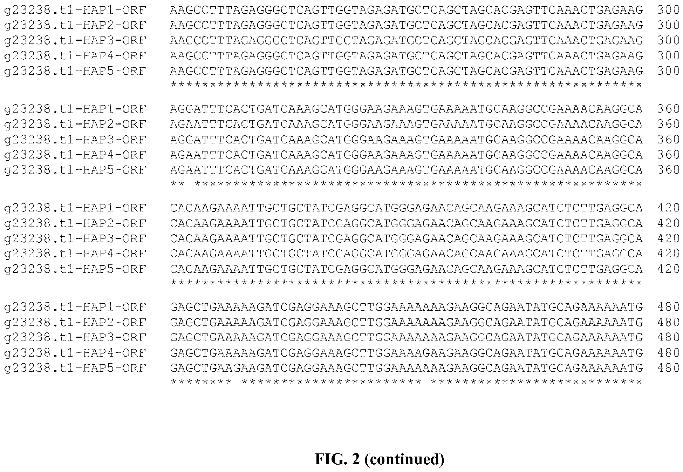

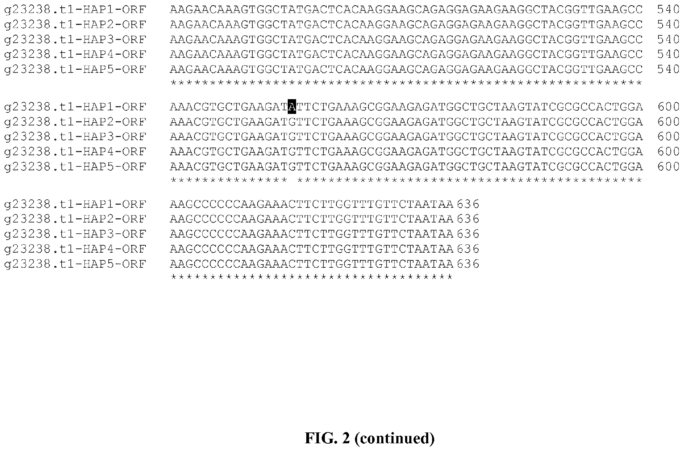

- **Fig. 2:**: Sequence alignment between the coding DNA (cDNA) sequence of the remorin gene encoding a non-functional remorin protein, *i.e.* a remorin protein which interferes with Rz2 mediated BNYVV resistance in a plant in which the protein is expressed (haplotype 1, Hap1-ORF), and the cDNA sequences of 4 haplotypes of the remorin gene encoding functional remorin proteins, *i*.*e*. remorin proteins which are involved in establishing Rz2 mediated BNYVV resistance in a plant in which the proteins are expressed (Hap2-ORF, Hap3-ORF, Hap4-ORF and Hap5-ORF). The polymorphisms are highlighted in gray.

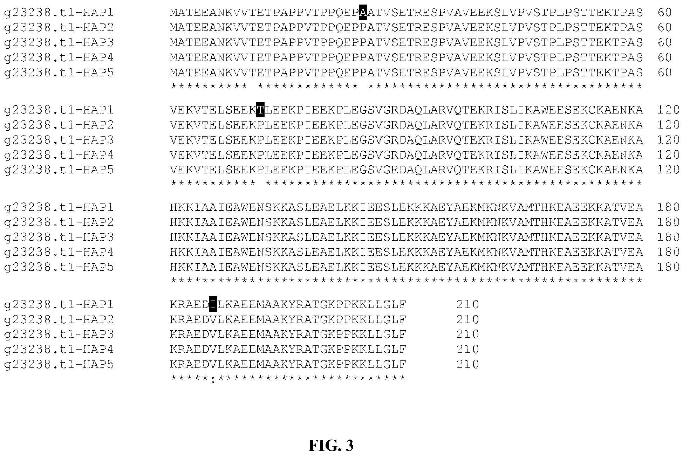

- **Fig. 3:**: Protein sequence alignment between a non-functional remorin protein, *i.e.* a remorin protein which interferes with Rz2 mediated BNYVV resistance in a plant in which the protein is expressed (Hap 1), and 4 functional remorin proteins, *i.e.* remorin proteins which are involved in establishing Rz2 mediated BNYVV resistance in a plant in which the proteins are expressed (Hap2, Hap3, Hap4 and Hap5). The polymorphisms are highlighted in gray.

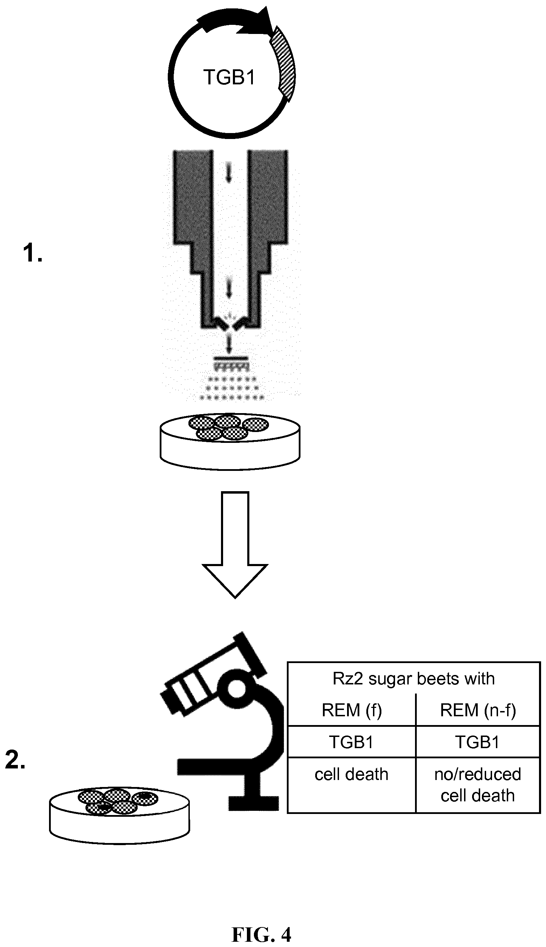

- **Fig. 4:**: Schematic overview of a transient assay for comparing the cell death reaction after expression of the TGB1 gene derived from BNYVV in leaf tissue of sugar beet comprising the resistance allele of Rz2 and either additionally comprising the functional allele of remorin (REM (f)) or the non-functional allele of remorin (REM (n-f)). The detailed experiment is explained in Example 2.

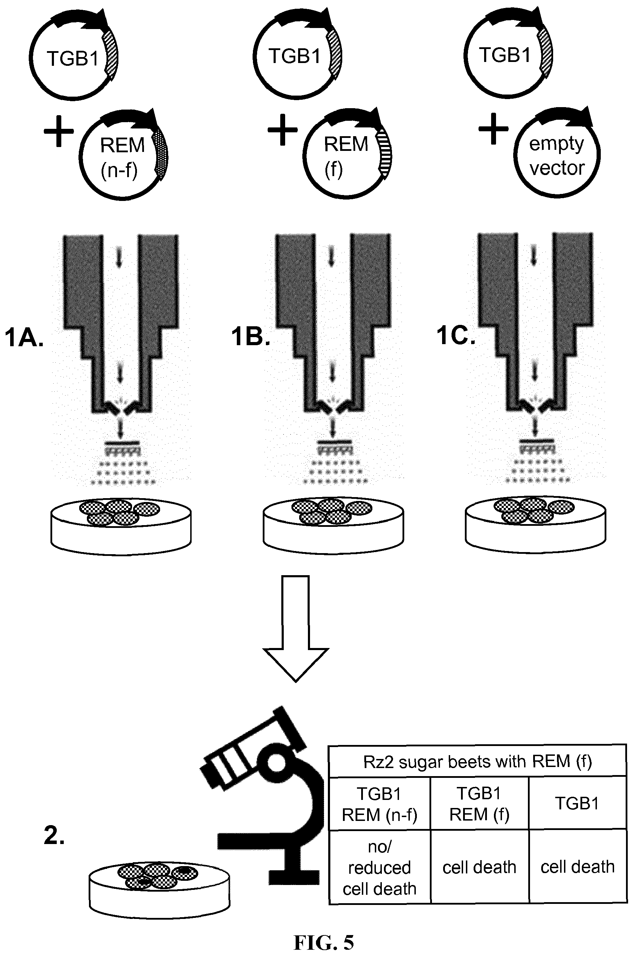

- **Fig. 5:**: Schematic overview of a transient assay for comparing the cell death reaction after expression of the TGB1 gene derived from BNYVV either in combination with the non-functional allele of remorin (REM (n-f)), with the functional allele of remorin (REM (f)), or with an empty vector control in leaf tissue of sugar beet comprising the resistance allele of Rz2 and the functional allele of remorin (REM (f)). The detailed experiment is explained in Example 2.

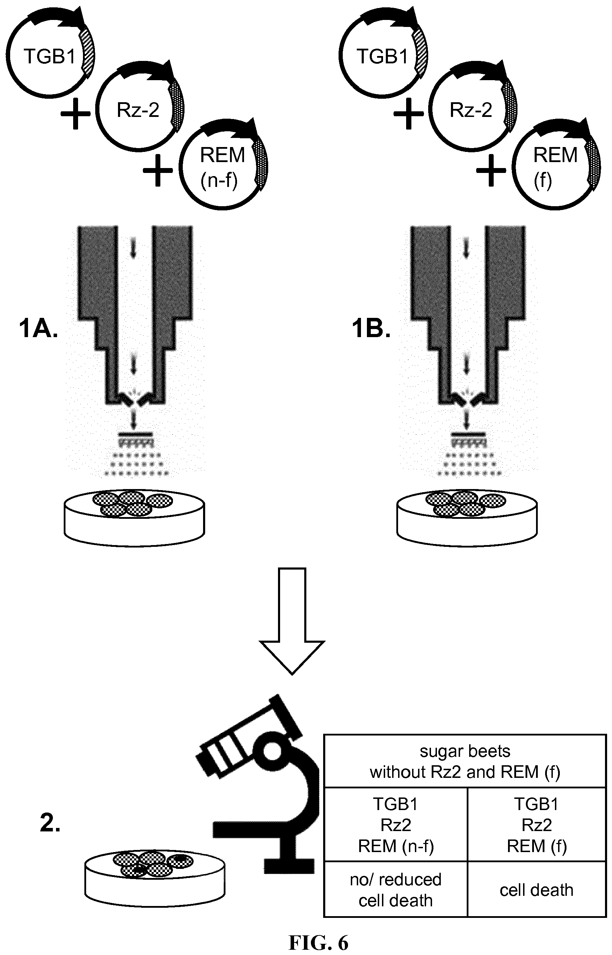

- **Fig. 6:**: Schematic overview of a transient assay for comparing the cell death reaction after expression of the TGB1 gene derived from BNYVV and the resistance allele of Rz2 either in combination with the non-functional allele of remorin (REM (n-f)) or with the functional allele of remorin (REM (f)) in leaf tissue of sugar beet which does neither comprise the resistance allele of Rz2 nor the functional allele of remorin (REM (f)). The detailed experiment is explained in Example 2.

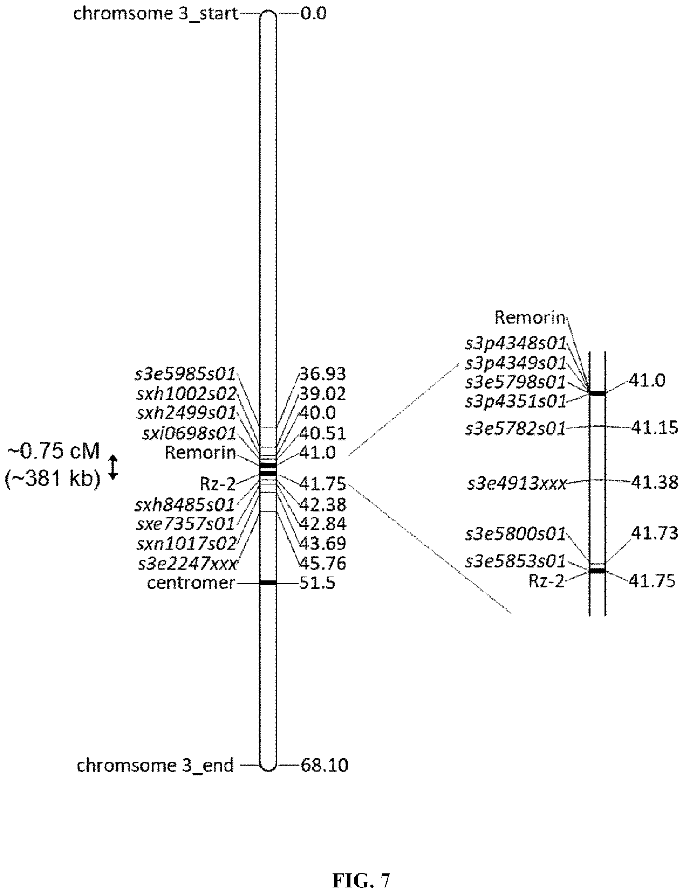

- **Fig. 7:**: Schematic view of chromosome 3 of *Beta vulgaris* indicating the positions of the remorin gene and the Rz2 gene as well as the positions of various markers. Remorin is positioned about 0.75 cM distal to the Rz2 gene on the long arm of chromosome 3. **Left:** marker positions distal to remorin and proximal to Rz2, which define flanking areas that can be used for marker assisted breeding (MAS); **Right:** markers binding in the remorin gene, in the Rz2 gene as well as in the region between both genes.

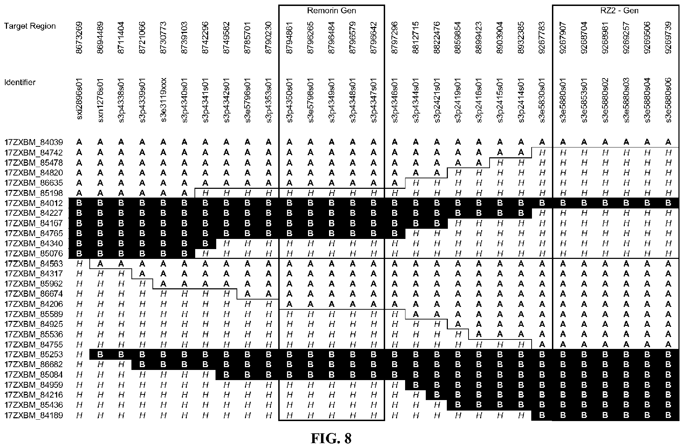

- **Fig. 8:**: Visual illustration of the marker-based analysis of 2730 plants of a dividing population with regard to the presence of different allele combinations of the Rz2 resistance gene and the remorin gene in the recombinants, wherein **A** indicates the presence of the remorin gene encoding a non-functional remorin protein and **B** indicates the presence of the remorin gene encoding a functional remorin protein. **H** designates the genotypes are heterozygous for the non-functional remorin allele.

### DETAILED DESCRIPTION OF THE INVENTION

In order to reveal the mechanism behind the observed effect that in certain sugar beet varieties the Rz2 mediated resistance against *Beet necrotic yellow vein virus* (BNYVV) is weakened or even completely lost, although the resistance allele of Rz2 is present and to use the gained knowledge for improving breeding processes, extensive crossing experiments as well as molecular biological experiments have been performed including genetic fine mapping, identification, isolation, and characterization of a gene encoding for a protein which in its functional form is important or crucial for establishing Rz2 mediated BNYVV resistance in plants and which in its non-functional form leads to suppression of said resistance.

The identification of both allelic variants, *i.e.* the one encoding a functional protein and the other encoding a non-functional protein is of high relevance in the breeding of BNYVV-resistant varieties that are to be equipped with Rz2 resistance. On the one hand, it is important to be able to separate the non-functional allele from the Rz2 resistance allele in order to use sugar beet lines showing a reduced or lost Rz2 mediated BNYVV resistance but having otherwise good breeding and agronomic properties in the breeding of BNYVV-resistant sugar beet varieties. On the other hand, it is of particular interest to understand the mechanism behind the development of Rz2 mediated resistance against BNYVV in plants, *i.e.* to identify the functional allele in order to efficiently generate plants being resistant to BNYVV.

Accordingly, the present invention generally relates to a nucleic acid molecule encoding a polypeptide which is involved in Rz2 mediated resistance against BNYVV. In this context, the term "involved in" includes increasing the resistance as well as being essential for establishing the resistance against BNYVV, in particular a high level of resistance against BNYVV. Thus, the nucleic acid molecule of the present invention and the encoded polypeptide, respectively, modifies the Rz2 mediated resistance against BNYVV in a plant in which the polypeptide is expressed. Preferably, the nucleic acid molecule of the present invention increases the resistance to BNYVV or is essential for establishing the resistance against BNYVV in a plant in which the corresponding polynucleotide is expressed. For the sake of simplicity, this nucleic acid molecule is referred to as "functional form", "functional variant", "functional allele", "functional gene", or "functional nucleic acid molecule" of the present invention.

Preferably, the nucleic acid molecule of the present invention modifies the Rz2 mediated resistance against BNYVV in a plant belonging to the genus *Beta,* preferably to the species *Beta vulgaris,* more preferably to the subspecies *Beta vulgaris subsp. vulgaris.*

The present invention further relates to a nucleic acid molecule which specifically hybridizes to the nucleic acid molecule of the present invention and encodes a non-functional allele of the polypeptide, *i.e.* a non-functional variant, which interferes with the resistance against BNYVV mediated by the Rz2 resistance gene in a plant in which the polypeptide is expressed. For the sake of simplicity, this nucleic acid molecule is referred to as "non-functional form", "non-functional variant", "non-functional allele", "non-functional gene", or "non-functional nucleic acid molecule" of the present invention.

In particular, the non-functional nucleic acid molecule of the present invention is able to reduce or suppress Rz2 mediated resistance to BNYVV in a plant in which the corresponding polynucleotide is expressed. Preferably said plant belongs to the genus *Beta,* preferably to the species *Beta vulgaris,* more preferably to the subspecies *Beta vulgaris subsp. vulgaris.*

The Rz2 resistance gene, which was previously identified and described in international patent application WO 2014/202044 A1 originates from the *Beta maritima* accession WB42 and is known to provide resistance to rhizomania when expressed in several varieties of sugar beet. The corresponding nucleic acid sequence is disclosed in WO 2014/202044 A1 and incorporated herein by reference. Rz3 from the *Beta maritima* accession WB41 has been identified, which is probably an allelic version of Rz2. Thus, the Rz2 and Rz3 genes are substantially identical and it is prudent to expect, but without to be bound by theory, that the nucleic acid molecule of the present invention is also involved in or interferes with Rz3 mediated resistance to BNYVV.

The identification and genetic localization of the nucleic acid molecule of the present invention has been performed as described in detail in Example 1. Initially, the focus was on the identification and localization of the non-functional allele. By genetic mapping it has been shown that the non-functional allele is localized close by the Rz2 locus. However, this area still contained too many genes to name a specific candidate. A particular challenge was the close genetic coupling/linkage of the non-functional allele with the Rz2 resistance gene. If it was located on another chromosome or at a greater distance from the Rz2 gene, the effect would be visible in QTL mapping as a separate effect. When using common breeding processes it was thus not possible to explain why genotypes were susceptible to rhizomania despite the presence of the resistance allele of Rz2.

Parallel to genetic mapping, the patterns of molecular markers in multigenic breeding material were analyzed. The marker *s3e5798s01* (see Table 5) showed the highest evidence for the positioning of the repressor effect. Analysis of the reference sequence for this region showed that the marker *s3e5798s01* is located in an annotated gene model (g23238.t1). The gene contains a remorin domain (C-terminal region) and is expressed in the plasmalemma. Remorins, a family of plant-specific proteins containing a variable N-terminal region and conserved C-terminal domain, play a role in various biotic and abiotic stresses, including host-microbe interactions. However, until now, their function is not fully understood.

There are indications in the literature that remorins can play a role in the spread of viruses through plasmodesmata (PDs) and in particular that they can act as negative regulator of virus movement (Raffaele et al., Plant Cell (2009), 21(5), 1541-1555; Perraki et al., FEMS Letters (2014), 588(9), 1699-1705). In general, PDs provide cell-to-cell connections that enable the symplastic transport of molecules from 1 to 60 kD. Viruses can move through PDs to achieve a local spreading infection, and symplastic loading into the phloem to initiate systemic infection.

In more detail, Raffaele *et al.* 2009 reported that in potato remorin interferes with cell-to-cell movement of *Potato Virus X* and binds directly to the virus movement protein TGBp1 (Triple Gene Block 1 protein). In plants underaccumulating remorin, a significant increase in viral accumulation in the leaves of infected plants has been observed and the infection foci were significantly larger than in wild-type plants. Consistently, reduced virus accumulation and smaller infection foci were observed in plants overexpressing remorin. Thus, remorin seems to have an antagonist effect on virus propagation. In this context, Perraki *et al.* 2014 have shown that in potatoes a remorin gene limits the ability of *Potato Virus X* TGBp1 to increase the permeability of PDs.

Furthermore, a recently published work by Wetzel and Varrelmann (International Plant Immunity Symposium - 1st IRTG 2172 PRoTECT Symposium June 14th and 15th 2018, Gottingen, Germany) suggests that the protein produced by the Rz2 gene, *i.e.* the Rz2 NBS-LRR receptor protein, may interact with the TGBp1 protein of BNYVV, the virus causing rhizomania. The missing link between the Rz2 NBS-LRR receptor protein and the TGBp1 protein of BNYVV might be the remorin identified in accordance with the present invention. From this a possible mode of action can be derived, which is shown in Figure 1A. Thus, it is to be assumed - but without being bound by one theory - that the remorin gene of the present invention has a similar or even the same function in *Beta vulgaris* as described above, *i.e.* that it acts as negative regulator of BNYVV movement.

The non-functional allele of the remorin gene identified in accordance with the present invention seems to be a non-functional protein. One possible mode of action is presented in Figure 1B, wherein the Rz2 NBS-LRR receptor protein of the resistance allele cannot detect the modification of this allele by the TGB1 protein of BNYVV. Therefore, no resistance reaction is initiated.

Further experiments performed in accordance with the present invention confirmed this hypothesis in that first results showed that the combination of the resistance allele of Rz2 with the non-functional allele of remorin leads to plants still being susceptible to rhizomania, while plants comprising the resistance allele of Rz2 and the functional allele of remorin show resistance; see Example 2.

The knowledge gained throughout identification and analysis of the non-functional remorin allele has been used afterwards to identify the functional remorin allele. In particular, a comparative analysis of genomic sequences of a sugar beet variety comprising the non-functional remorin allele and thereby showing no or reduced BNYVV-resistance despite the presence of the Rz2 gene with sequences of 80 additional sugar beet genotypes has been performed in order identify the sequence of the functional remorin gene.

A total of five different haplotypes (alleles) were found. The haplotype of the non-functional remorin gene (haplotype 1) can be distinguished from the other four haplotypes, *i.e.* the functional remorin genes, by three KASP markers (*s3p4348s01* (SEQ ID NO: 27; non-functional allele: G), *s3p4349s01* (SEQ ID NO: 28; non-functional allele: T), *s3p4351s01* (SEQ ID NO: 29; non-functional allele: T); see Table 5. The coding sequence, *i.e.* the open reading frame (ORF) of the non-functional allele set forth in SEQ ID NO: 3 has five small nucleotide polymorphisms (SNPs) which distinguish the haplotype (Hap1) from the other four haplotypes (Hap2-5; SEQ ID NOs: 7, 11, 15 and 19) (Figure 2). Accordingly, the coding sequence (CDS) of Hap1 has an A instead of a G at position 72, a G instead of a C at position 73, an A instead of a G at position 111, an A instead of a C at position 214, and an A instead of a G at position 556; see Table 1. The protein sequence of the non-functional allele set forth in SEQ ID NO: 4 differs clearly in three amino acids from the other four haplotypes (SEQ ID NOs: 8, 12, 16 and 20); see Table 2. As shown in Figure 3, the translated non-functional polypeptide encoded by the non-functional remorin gene has Ala instead of Pro at position 25, Thr instead of Pro at position 72 and Ile instead of Val at position 186; see also Table 2.

**Table 1: Identified SNPs in coding sequences (ORFs) of the non-functional remorin allele (= haplotype 1, Hap1) in comparison with the ORFs of the other four haplotypes, i.e. the functional remorin alleles. Positions marked with * lead to amino acid exchanges, which could be the reason for the non-functionality of the remorin.**

| CDS nucleotide position | Hap1 (SEQ ID No: 3) | Hap2 (SEQ ID No: 7) | Hap3 (SEQ ID No: 11) | Hap4 (SEQ ID No: 15) | Hap5 (SEQ ID No: 19) |
|---|---|---|---|---|---|
| 72 | A | G | G | G | G |
| 73* | G | C | C | C | C |
| 111 | A | G | G | G | G |
| 214* | A | C | C | C | C |
| 556* | A | G | G | G | G |

**Table 2: Identified amino acid substitutions in the predicted sequence of the polypeptide of the non-functional remorin allele (= haplotype 1) in comparison with the predicted sequence of the polypeptide of other four haplotypes, i.e. the functional alleles of remorin.**

| amino acid position | Hap1 (SEQ ID NO: 4) | Hap2 (SEQ ID NO: 8) | Hap3 (SEQ ID NO: 12) | Hap4 (SEQ ID NO: 16) | Hap5 (SEQ ID NO: 20) |
|---|---|---|---|---|---|
| 25 | Ala | Pro | Pro | Pro | Pro |
| 72 | Thr | Pro | Pro | Pro | Pro |
| 186 | Ile | Val | Val | Val | Val |

For the sake of simplicity, a consensus sequence has been generated combining the cDNA sequences of haplotypes 2 to 5 (SEQ ID No. 44) and another one combining the amino acid sequences of haplotypes 2 to 5 (SEQ ID No. 45).

Accordingly, in one embodiment the present invention relates to a nucleic acid molecule encoding a polypeptide which is involved in Rz2 mediated resistance against BNYVV, wherein the amino acid sequence of the encoded polypeptide has a proline (P) at position 25 with reference to SEQ ID No. 45, a proline (P) at position 72 with reference to SEQ ID No. 45 or a valine (V) at position 186 with reference to SEQ ID No. 45. Of course, the amino acid sequence of the encoded polypeptide may have two of the mentioned amino acids at the indicated positions, *i.e.* a proline (P) at position 25 and a proline (P) at position 72 with reference to SEQ ID No. 45; a proline (P) at position 25 and a valine (V) at position 186 with reference to SEQ ID No. 45; or a proline (P) at position 72 and a valine (V) at position 186 with reference to SEQ ID No. 45; as well as three of the mentioned amino acids at the indicated positons, *i.e.* a proline (P) at position 25, a proline (P) at position 72 and a valine (V) at position 186 with reference to SEQ ID No. 45.

With reference to the coding DNA sequence of said nucleic acid molecule, the coding DNA sequence has a cytosine (c) at position 73 with reference to SEQ ID No. 44, a cytosine (c) at position 214 with reference to SEQ ID No. 44, or a guanine (g) at position 556 with reference to SEQ ID No. 44. Of course, the coding DNA sequence may have two of the mentioned amino acids at the indicated positons, *i*.*e*. a cytosine (c) at position 73 and a cytosine (c) at position 214 with reference to SEQ ID No. 44; a cytosine (c) at position 73 and a guanine (g) at position 556 with reference to SEQ ID No. 44; or a cytosine (c) at position 214 and a guanine (g) at position 556 with reference to SEQ ID No. 44; as well as three of the mentioned amino acids at the indicated positons, *i.e.* a cytosine (c) at position 73, a cytosine (c) at position 214 and a guanine (g) at position 556 with reference to SEQ ID No. 44.

In a preferred embodiment, the present invention relates to a nucleic acid molecule encoding a polypeptide which is involved in Rz2 mediated resistance against BNYVV, wherein the polypeptide has the amino acid sequence of SEQ ID No. 8, 12, 16, or 20, or the nucleotide sequence that encodes said polypeptide is SEQ ID No. 5, 6, 7, 9, 10, 11, 13, 14, 15, 17, 18 or 19, or the coding DNA sequence is a nucleotide sequence according to SEQ ID No. 7, 11, 15, or 19.

Furthermore, substitutions, deletions, insertions, additions, and/or any other change may be introduced into the nucleotide sequence according to the invention that, alone or in combinations, do in fact change the nucleotide sequence, wherein the modified nucleotide sequence may, however, perform the same function as the initial sequence. The present case deals with the coding of an amino acid sequence which is involved in resistance against BNYVV mediated by the Rz2 resistance gene.

In a further embodiment, the invention therefore includes a nucleotide sequence that encodes a polypeptide which represents a derivative of the polypeptide which is encoded by the nucleotide sequence according to the invention, or which includes the amino acid sequence according to the invention. A derived amino acid sequence which has at least one substitution, deletion, insertion, or addition of one or more amino acids, wherein the functionality of the encoded polypeptide/protein is preserved, represents a derivative of the polypeptide. Substitutions, deletions, insertions, additions, and/or any other change, either solely or in combinations, that do in fact change the nucleotide sequence, but perform the same function as the initial sequence, may thereby be introduced into the nucleotide sequence using conventional methods that are known in the prior art, *e.g.,* via site-directed mutagenesis, PCR-mediated mutagenesis, transposon mutagenesis, genome editing, etc.

The substitution of one amino acid by a different amino acid having the same or equivalent or similar chemical/physical properties is referred to as a "conservative substitution" or "semi-conservative substitution." Examples of physical/chemical properties of an amino acid are, for example, hydrophobia or the charge. Which amino acid substitution represents a conservative or semi-conservative substitution is known to the person skilled in the art. Moreover, general expertise allows the person skilled in the art to recognize, identify, and detect which amino acid deletions and additions are harmless to the functionality of the resistance protein, and at which positions these are possible. The person skilled in the art is aware that, in the case of the present functional remorin protein for modifications of the amino acid sequence (substitutions, deletions, insertion, or additions of one or more amino acids), the functionality, in particular, of the conserved domains, *e.g.* the remorin domain must be preserved, and that therefore only limited preceding modifications are possible in these domains.

The invention thus includes a functional fragment of the nucleotide sequence according to the invention. The term, "fragment," thereby includes genes with a nucleotide sequence sufficiently similar to the aforementioned nucleotide sequence. The term, "sufficiently similar," means that a first nucleotide sequence or amino acid sequence has a sufficient or minimum number of identical or equivalent nucleotides or amino acid groups relative to a second nucleotide sequence or a second amino acid sequence.

With regard to the amino acid sequence, after modification via an aforementioned method, this also has a common structural domain and/or possesses common functional activity. Nucleotide sequences or amino acid sequences that have an identity of at least approximately 70%, at least approximately 75%, at least approximately 80%, at least approximately 85%, at least approximately 90%, at least approximately 91%, at least approximately 92%, at least approximately 93%, at least approximately 94%, at least approximately 95%, at least approximately 96%, at least approximately 97%, at least approximately 98%, at least approximately 99%, or at least approximately 100% with the nucleotide sequence or amino acid sequence according to the invention are defined here as being sufficiently similar. This also explicitly encompasses the range of 90% to 100%. For the functional fragments, a sufficient similarity is established if the nucleotide sequence or amino acid sequence generally has the same property as the previously-named nucleotide sequence or amino acid sequence of the present invention. Those nucleotide sequences which encode a derivative or for a derived amino acid sequence are generated either directly or indirectly (for example, via amplification or replication steps) from an initial nucleotide sequence which corresponds to the nucleotide sequence according to the invention over the entire length, or at least in part.

Accordingly, the present invention includes a nucleotide sequence that is able to hybridize, under stringent conditions, with a nucleotide sequence complementary to a nucleotide sequence according to the invention or to the nucleotide sequence that encodes the amino acid sequence according to the invention.

The present invention further relates to a nucleic acid molecule which specifically hybridizes to the nucleic acid molecule of the present invention and encodes a non-functional allele of the polypeptide, *i.e.* a non-functional remorin variant, which interferes with resistance against BNYVV mediated by the Rz2 resistance gene in a plant in which the polypeptide is expressed. For the sake of simplicity, this nucleic acid molecule is referred to as "non-functional form", "non-functional variant", "non-functional allele", or "non-functional gene", or "non-functional nucleic acid molecule" of the present invention.

As shown in Example 2, in particular in Assay 2, the non-functional polypeptide (remorin) allele seems to be dominant over the functional allele of the polypeptide. Accordingly, in a preferred embodiment of the present invention, the non-functional polypeptide encoded by the nucleic acid molecule according to [3] is dominant over the functional allele of the polypeptide encoded by a nucleic acid molecule according to [1] or [2]. Dominant means that in the heterozygous state of the plant, *e.g.,* the plant comprising a non-functional allele and a functional allele of the polypeptide the non-functional allele inhibits or prevents the development of resistance and as a result the plant is susceptible to BNYVV infection and spread of the virus within the plant, respectively.

In particular, the non-functional nucleic acid molecule of the present invention is able to reduce or suppress Rz2 mediated resistance to BNYVV in a plant in which the corresponding polynucleotide is expressed. Preferably said plant belongs to the genus *Beta,* preferably to the species *Beta vulgaris,* more preferably to the subspecies *Beta vulgaris subsp. vulgaris.*

The nucleotide and amino acid encoding sequence of said non-functional allele is characterized by polymorphisms, which differentiates it from the functional allele, *i.e.* the nucleic acid molecule of the present invention which modifies the Rz2 mediated resistance against BNYVV in a plant in which the polypeptide is expressed. In principle, the sequence of the non-functional gene of the present invention and the corresponding polypeptide, respectively is based on the sequence of the nucleic acid molecule of the present invention as described above but including one or more mutations as already defined above leading to a non-functional variant. There are of course plenty of possibilities for mutations leading to the non-functional variant and the person skilled in the art of course knows how to introduce single mutations and how to test whether the generated gene and polypeptide, respectively is indeed inactive. Details regarding suitable methods are described elsewhere herein.

In a preferred embodiment, the non-functional nucleic acid molecule of the present invention comprises a nucleotide sequence encoding a polypeptide having an amino acid sequence according to SEQ ID No. 4 or a polypeptide that is of at least approximately 70%, at least approximately 75%, at least approximately 80%, at least approximately 85%, at least approximately 90%, at least approximately 91%, at least approximately 92%, at least approximately 93%, at least approximately 94%, at least approximately 95%, at least approximately 96%, at least approximately 97%, at least approximately 98%, at least 99% or 100% identical to SEQ ID No. 4, wherein the polypeptide comprises an amino acid sequence having at least one amino acid substitution due to one or more mutations in the nucleotide sequence, preferably wherein proline (P) at position 25 with reference to SEQ ID No. 45, proline (P) at position 72 with reference to SEQ ID No. 45, and/or valine (V) at position 186 with reference to SEQ ID No. 45 is substituted with another amino acid. Of course, the amino acid sequence may have one, two or three amino acid substitutions at the indicated positions. Thus, in one embodiment, proline (P) at position 25, proline (P) at position 72, or valine (V) at position 186 with reference to SEQ ID No. 45 is substituted with another amino acid. In a further embodiment, proline (P) at position 25 and proline (P) at position 72 with reference to SEQ ID No. 45 are substituted with another amino acid; or proline (P) at position 25 and valine (V) at position 186 with reference to SEQ ID No. 45 are substituted with another amino acid; or proline (P) at position 72 and valine (V) at position 186 with reference to SEQ ID No. 45 are substituted with another amino acid. Alternatively, proline (P) at position 25, proline (P) at position 72 and valine (V) at position 186 with reference to SEQ ID No. 45 are substituted with another amino acid.

In a preferred embodiment, the non-functional remorin polypeptide of the present invention comprises an amino acid sequence having alanine (A) instead of proline (P) at position 25 with reference to SEQ ID No. 45, threonine (T) instead of proline (P) at position 72 with reference to SEQ ID No. 45, and/or isoleucine (I) instead of valine (V) at position 186 with reference to SEQ ID No. 45. Of course, the amino acid sequence may have one, two or all three amino acid substitutions at the indicated positions as explained above.

With respect to the coding DNA sequence, in a preferred embodiment, the non-functional remorin gene of the present invention comprises a nucleotide sequence that comprises the coding DNA sequence according to SEQ ID No. 3, that comprises a coding DNA sequence which is at least 70% identical to the DNA sequence according to SEQ ID No. 3, or that hybridizes with a complementary sequence of SEQ ID No. 3 under stringent conditions, wherein the nucleotide sequence comprises at least one nucleic acid substitution due to at least one mutation leading to an amino acid substitution, preferably wherein one or more nucleotides are substituted at positions 73-75 with reference to SEQ ID No. 44, at positions 214-216 with reference to SEQ ID No. 44 and/or at positions 556-558 with reference to SEQ ID No. 44. Of course, the coding DNA sequence may have substituted nucleotides at one, two or all three positions indicated. Thus, in one embodiment, one or more nucleotides are substituted at positions 73-75, at positions 214-216, or at positions 556-558 with reference to SEQ ID No. 44. In a further embodiment, one or more nucleotides are substituted at positions 73-75 and at positions 214-216 with reference to SEQ ID No. 44; or one or more nucleotides are substituted at positions 73-75 and at positions 556-558 with reference to SEQ ID No. 44; or one or more nucleotides are substituted at positions 214-216 and at positions 556-558 with reference to SEQ ID No. 44. Alternatively, one or more nucleotides are substituted at positions 73-75, at positions 214-216, and at positions 556-558 with reference to SEQ ID No. 44.

In a preferred embodiment, the coding DNA sequence has guanine (g) instead of cytosine (c) at position 73 with reference to SEQ ID No. 44, adenine (a) instead of cytosine (c) at position 214 with reference to SEQ ID No. 44, and/or adenine (a) instead of guanine (g) at position 556 with reference to SEQ ID No. 44. Of course as mentioned above, the coding DNA sequence may have substituted nucleotides at one, two or all three positions indicated.

Optionally, the coding DNA sequence has in addition adenine (a) instead of guanine (g) at that position 72 with reference to SEQ ID No. 44, and/or adenine (a) instead of guanine (g) at position 111 with reference to SEQ ID No. 44.

However, as described above with respect to the functional remorin allele, the person skilled in the art of course knows that further substitutions, deletions, insertions, additions, and/or any other change may be introduced that, either alone or in combinations, do in fact change the nucleotide sequence, but perform the same function as the initial sequence - here, the nucleotide sequence encoding the non-functional variant of the remorin gene.

The nucleic acid molecule according to the invention, including the nucleic acid molecule which is involved in conferring a resistance against the pathogen BNYVV and the corresponding non-functional variant interfering with said resistance, may be an isolated nucleic acid molecule. It is preferably DNA, and, particularly preferably, cDNA (coding DNA). The functional remorin protein is involved in conferring a resistance against the pathogen BNYVV which causes the plant disease rhizomania, more preferably it modifies the Rz2 mediated resistance against BNYVV in a plant in which the protein is expressed, *i.e.* which increases said resistance to BNYVV or is essential for establishing said resistance. The non-functional remorin protein interferes with said resistance. Furthermore, the polypeptide is involved in conferring a resistance to this pathogen and interferes with said resistance, respectively, in particular in a plant of the genus *Beta.* The plant is preferably a plant of the species *Beta vulgaris* - particularly preferably, a plant of the subspecies *Beta vulgaris* subsp. *vulgaris;* among these are, for example, the cultivars sugar beet, beetroot, fodder beet, chard, and Swiss chard.

When not stated otherwise, if referred to the nucleic acid molecule or the polypeptide of the present invention, the nucleic acid molecule and the polypeptide, respectively which is involved in Rz2 mediated resistance against BNYVV, *i.e.* the function remorin gene and the functional remorin polypeptide/protein, as well as the nucleic acid molecule and the polypeptide, respectively which interferes with the Rz2 mediated resistance against BNYVV, *i.e.* the non-function remorin gene and the non-functional remorin polypeptide/protein, are included.

Such a gene that modifies the Rz2 mediated resistance, or that encodes a polypeptide that is able to modify the Rz2 mediated resistance is not known from the prior art. As was already described above, in previously available varieties on the market, it has been observed that certain genetic backgrounds led to a weakening or loss of resistance against BNYVV, although the resistant allele of Rz2 was present the corresponding plants. With the identification of the gene being responsible for this effect, *i.e.* the non-functional variant of the remorin gene, which allows the generation of suitable markers, it is now possible to diagnose the non-functional remorin allele due to its unique haplotype. Furthermore, the exact knowledge of the genetic and physical positions of the Rz2 gene and the remorin gene allows for the first time to identify recombinants between the genes and thus to separate the non-functional remorin gene from the Rz2 resistance allele. In new crossings with markers located on or between remorin and Rz2, recombinants showing the functional allele of the crossing partner can be selected. Furthermore, the identification of the functional remorin allele allows the incorporation of this gene into plants, e.g. into high-yield varieties, having the Rz2 resistance gene but being no longer resistant to BNYVV due to the presence of the non-functional variant of remorin.

Thus, via the incorporation of the nucleic acid molecule of the present invention in said plants, *i.e.* the function remorin gene, optionally in combination with removing the non-functional remorin gene, *e.g.* via inhibiting its expression at all, or via mutating the non-functional remorin gene in order to establish the functional variant, it is now possible to very quickly develop high-yield varieties with a restored resistance to BNYVV. Accordingly, in the framework of the present invention there are provided for the first time a sugar beet plant, a chard plant, a red beet or beetroot plant, a fodder beet plant having the resistance mediated by the Rz2 gene and the functional remorin gene according to the invention against BNYVV and thus being encompassed by the present invention. As the listed plants are all cultivated plants, crops or plants which are suitable for the agricultural cultivation and which have the resistance according to the invention, are part of the invention. Especially such crops are part of the invention which comprise a subterrestrial storage organ usable as food, raw material or industrial source of sugar and which comprise the resistance according to the invention are a further aspect of the present invention. The storage organ can be for example the sugar containing beet body of the sugar beet, the consumable beet body of the red beet or the feedable beet body of the fodder beet. The subterrestrial storage organ can sum up to more than 50% and for the sugar beet even to more than 70% of the total mass of the full-grown plant. Furthermore, also seeds or seeding material of these plants are part of the invention. The seeds or the seeding material can be technically treated as described further below.

Furthermore, the present invention relates to a recombinant and/or heterologous DNA molecule that comprises the sequences of the nucleic acid molecule according to the invention, *i.e.* the nucleic acid molecule involved in Rz2 mediated resistance to BNYVV and the nucleic acid molecule which interferes with said resistance, respectively. This DNA molecule, furthermore, preferably has a regulatory sequence. It may thereby be operatively linked with this regulatory sequence or be under the influence of this regulatory sequence. This regulatory sequence is preferably a promoter sequence and/or other sequences of transcription or translation control elements - for example, *cis*-elements. The regulatory sequence, which controls the expression of a gene that includes the nucleic acid molecule according to the invention, is preferably a sequence that is able to confer or modulate the expression, as a result of a pathogenic infection. This promoter is preferably able to control the expression of the DNA sequence specifically in roots of the plant. The regulatory sequence may be heterologous to the expressing sequence. Such an approach has the advantage that the person skilled in the art may better adjust the expression rate of the expressing sequence, the tissue in which the expression occurs, and the point in time at which the expression occurs, in that he selects that regulatory sequence which is best suited to the respective use case. The heterologous DNA sequence preferably includes a nucleotide sequence which encodes a component of the plant pathogen defense (example: resistance genes (R-genes) or genes which encode enzymes involved in signal transfer, such as kinases or phosphatases, and for G-protein, or which encode a pathogenic effector (what are known as avirulence genes (*avr*))). The heterologous DNA sequence may be one of the DNA sequences according to the invention. The heterologous DNA sequence may also additionally encode further components of the plant pathogen defense. The heterologous DNA sequence may therefore be designed such that a polycistronic mRNA is created after its transcription.

The present invention further relates to a polypeptide which can be encoded by the nucleic acid molecule according to the invention and a functionally and/or immunologically active fragment thereof, as well as an antibody that specifically binds to the polypeptide or to its fragment. The polypeptide particularly preferably has an amino acid sequence according to any one of SEQ ID Nos. 4, 8, 12, 16 and 20. The recombinant production of proteins, polypeptides, and fragments is familiar to the person skilled in the art (Sambrook et al., Molecular Cloning: A Laboratory Manual, 3rd ed., Cold Spring Harbor Laboratory Press, Cold Spring Harbor, NY, 2001, or Wingfield, P. T., 2008, Production of Recombinant Proteins, Current Protocols in Protein Science, 52:5.0:5.0.1-5.0.4). Polyclonal or monoclonal antibodies to the protein according to the invention may be produced by the person skilled in the art according to known methods (E. Harlow et al., editor, Antibodies: A Laboratory Manual (1988)). The production of monoclonal antibodies, as well as of Fab and F(ab')₂ fragments that are also useful in protein detection methods, may be performed via various conventional methods (Goding, Monoclonal Antibodies: Principles and Practice, pp. 98-118, New York: Academic Press (1983)). The antibodies may then be used for the screening of expression cDNA libraries in order to identify identical, homologous, or heterologous genes by means of immunological screening (Sambrook et al., Molecular Cloning: A Laboratory Manual, Cold Spring Harbor Laboratory Press, Cold Spring Harbor, NY, 1989, or Ausubel et al., 1994, "Current Protocols in Molecular Biology." John Wiley & Sons), or may be used for western blot analyses.

In particular, the present invention relates to antibodies that selectively detect a polypeptide encoded by the nucleic acid molecule of the present invention involved in Rz2 mediated resistance to BNYVV, *i.e.* the functional remorin allele and essentially do not detect the polypeptide encoded by the nucleic acid molecule of the present invention which interferes with said resistance, *i.e.* the non-functional remorin allele. In particular, they detect less, by a factor of 2 - preferably, a factor of 5, and, more preferably, a factor or 10 or more - of the polypeptide encoded by the correspondingly non-functional remorin allele than the polypeptide encoded by the functional remorin allele. In another embodiment, the present invention relates to antibodies that selectively detect a polypeptide encoded by the nucleic acid molecule of the present invention which interferes with the Rz2 mediated resistance to BNYVV, *i.e.* the non-functional remorin allele and essentially do not detect the polypeptide encoded by the nucleic acid molecule of the present invention which is involved in said resistance, *i.e*. the functional remorin allele. In particular, they detect less, by a factor of 2 - preferably, a factor of 5, and, more preferably, a factor or 10 or more - of the polypeptide encoded by the correspondingly functional remorin allele than the polypeptide encoded by the non-functional remorin allele.

In a preferred embodiment, the antibody according to the invention is characterized in that it is a synthetic polypeptide which does not occur in nature.

Furthermore, the antibodies according to the invention may be linked with a fluorescent dye in order to be usable in an immunohistochemical method, for example, and evoke an antibody coloration. The fluorescent dye may be fluorochrome. The antibodies according to the invention may also be present linked with other signaling molecules. Among these are, for example, biotin, radioisotopes, reporter enzymes such as alkaline phosphatase, or oligonucleotides.

An additional subject matter of the invention are vectors or expression cassettes that include the nucleic acid molecule or the recombinant DNA molecule according to the invention - possibly under control of regulatory elements and, in particular, under control of functional regulatory elements in plants, as well as negative and/or positive selection markers. The vector backbone is thereby heterologous to the nucleic acid molecule according to the invention, which means that such a vector does not occur in nature and cannot be isolated from nature. The vector is a plasmid, a cosmid, a phage or an expression vector, a transformation vector, shuttle vector or cloning vector; it may be double-stranded or single-stranded, linear or circular; or it may transform a prokaryotic or eukaryotic organism either via integration into its genome or extrachromosomally. The nucleic acid molecule or DNA molecule according to the invention in an expression vector or expression cassette is, preferably, operatively linked with one or more regulatory sequences which allow the transcription and, optionally, the expression in a prokaryotic or eukaryotic cell; (Sambrook et al., Molecular Cloning: A Laboratory Manual, 3rd ed., Cold Spring Harbor Laboratory Press, Cold Spring Harbor, NY, 2001). These regulatory sequences are preferably promoters or terminators - in particular, a transcription initiation starting point, a ribosome binding location, an RNA-processing signal, a transcription termination location, and/or a polyadenylation signal. For example, the nucleic acid molecule is here under the control of a suitable promoter and/or a terminator. Suitable promotors may be constitutive promotors (example: 35S promoter from the "Cauliflower mosaic virus" (Odell et al., Nature 313 (1985), 810 - 812); those promoters which are pathogenically inducible are especially suitable (example: PR1 promoter from parsley (Rushton et al., EMBO J. 15 (1996), 5,690-5,700)). Particularly suitable pathogenically-inducible promoters are synthetic or chimeric promoters which do not occur in nature, are composed of multiple elements, and contain a minimal promoter, and have at least one *cis*-regulatory element upstream of the minimal promoter, which at least one *cis*-regulatory element serves as a binding location for special transcription factors. Chimeric promoters are designed according to the desired requirements and are induced or repressed via different factors. Examples of such promoters are found in WO 00/29592, WO 2007/147395, and WO 2013/091612. For example, a suitable terminator is the nos-terminator (Depicker et al., J. Mol. Appl. Genet. 1 (1982), 561-573). Suitable promoters and terminators may also be the native promoter and the native terminator. The vectors or expression cassettes additionally contain for conventional indicator/reporter genes or resistance genes for the detection of the transfer of the desired vector or DNA molecule/nucleic acid molecule, and for selection of the individuals that contain these, since a direct detection via the expression of the gene is for the most part rather difficult.

Examples of indicator/reporter genes are, for example, the luciferase gene and the gene encoding green fluorescent protein (GFP). These, furthermore, also allow tests for the activity and/or regulation of a promoter of the gene. Examples of resistance genes - especially, for plant transformations - are the neomycin phosphotransferase gene, the hygromycin phosphotransferase gene, or the gene encoding phosphinothricin acetyltransferase. Additional positive selection markers may be enzymes which provide the transformed plant a selection advantage over the non-transformed plant - in particular, a nutrient advantage, e.g., the mannose-6-phosphate isomerase or the xylose isomerase. However, this does not preclude additional indicator/reporter genes or resistance genes known to the person skilled in the art. In a preferred embodiment, the vector is a plant vector. Furthermore, the expression cassette may be present as integrated into a plant genome.

In a further aspect, the present invention relates to cells that include the vectors, recombinant DNA molecules, and/or nucleic acid molecules according to the invention. A cell in the sense of the invention may be a prokaryotic (for example, bacterial) or eukaryotic cell (for example, a plant cell or a yeast cell). The cell is preferably an agrobacterium such as *Agrobacterium tumefaciens* or *Agrobacterium rhizogenes,* an *Escherichia coli* cell, or a plant cell; the plant cell is particularly preferably a cell of a plant of the genus *Beta,* the species *Beta vulgaris,* or the subspecies *Beta vulgaris* subsp. *vulgaris.* The cell may also be present as a culture. The invention also consequently covers a cell culture which contains such cells. The cell culture is preferably a pure culture or an isolate that contains no cells of another type.

Known to the person skilled in the art are both numerous methods, such as conjugation or electroporation, with which he may introduce the nucleic acid molecule according to the invention, the recombinant DNA molecule, and/or the vector or the expression cassette of the present invention into an agrobacterium, and methods such as diverse transformation methods (biolistic transformation, agrobacterium-mediated transformation) with which he may introduce the nucleic acid molecule according to the invention, the DNA molecule, and/or the vector of the present invention into a plant cell (Sambrook et al., Molecular Cloning: A Laboratory Manual, 3rd ed., Cold Spring Harbor Laboratory Press, Cold Spring Harbor, NY, 2001.

Furthermore, the present invention relates to a plant - preferably, plant of the species *Beta vulgaris* subsp. *vulgaris* or a portion thereof - that contains the nucleic acid molecule according to the invention which is involved in resistance against BNYVV, *i.e.* the functional remorin gene. The nucleic acid molecule of the present invention might be either introduced or introgressed into the genome of the plant. In one embodiment, the nucleic acid molecule of the present invention is foreign to the plant, *i.e.* it is a heterologous nucleic acid molecule.

Thus, the plant of the present invention may comprise the nucleic acid molecule according to the invention as a transgene or as endogene or may comprise the vector or expression cassette of the present invention which comprises said nucleic acid molecule as transgene. Furthermore, the plant of the present invention may comprise the cells of the present invention harboring the vector and the nucleic acid molecule of the present invention, respectively. In a preferred embodiment, the plant of the present invention is heterozygous or preferably homozygous for the nucleic acid molecule of the present invention.

In one embodiment, the plant of the present invention further comprises the Rz2 resistance gene mediating BNYVV resistance endogenously or transgenically. Accordingly, in the framework of the present invention there are provided for the first time a sugar beet plant, a chard plant, a red beet or beetroot plant, a fodder beet plant having the resistance against BNYVV mediated by the Rz2 gene and the functional remorin gene according to the invention.

A portion of the plant of the present invention may thereby be a cell, a tissue, an organ, or a combination of multiple cells, tissues, or organs. A combination of multiple organs is, for example, a blossom or a seed. A plant of the present invention comprising the Rz2 gene and the functional remorin gene according to the invention preferably shows a higher resistance against BNYVV than a corresponding plant that does contain the Rz2 gene but does not contain the nucleic acid molecule according to the invention, *i*.*e*. the functional remorin gene and/or which does contain the nucleic acid molecule of the present invention which interferes with the Rz2 mediated BNYVV resistance, *i.e.* the non-functional variant of the remorin (control plants). The control plants ideally have the identical genotype as the transgenic plant, and have been cultured under identical conditions, but do not contain the nucleic acid molecule of the present invention involved in Rz2 mediated BNYVV resistance and/or do contain the nucleic acid molecule of the present invention which interferes with the Rz2 mediated BNYVV resistance.

A plant cell or plant or portion thereof of the present invention that contains the nucleic acid molecule according to the invention - in particular, a plant of the genus *Beta* - preferably shows a higher resistance to a pathogen - in particular, to BNYVV- than a corresponding plant cell or plant or portion thereof that does not contain the nucleic acid molecule according to the invention, or contains the nucleic acid molecule of the present invention which interferes with the Rz2 mediated BNYVV resistance.

In the case of a transgenic plant cell, or plant or portion thereof, this comprises the nucleic acid molecule or DNA molecule according to the invention, *i.e*. the functional remorin gene, as a transgene or the vector or the expression cassette of the present invention. Such a transgenic plant cell or plant or portion thereof is, for example, one that is transformed - preferably, stably - with said nucleic acid molecule, DNA molecule, or with the corresponding vector or the expression cassette. In a preferred embodiment, the nucleic acid molecule is operatively linked with one or more regulatory sequences which allow the transcription and, optionally, the expression in the plant cell. The total structure made up of said nucleic acid molecule and the regulatory sequence(s) then represents the transgene. Such regulatory sequences are, for example, a promoter or a terminator. Numerous functional promoters and terminators that are applicable in plants are known to the person skilled in the art.

The invention also includes a vacuole of the cell according to the invention, and the content substances stored therein.

Furthermore, the invention also relates to the cell extract from a cell - preferably, from a plant cell, particularly preferably, from a cell of *Beta vulgaris,* and, especially preferably, from a cell of one of the following crops: sugar beet, chard, or beetroot. No plant can be regenerated from the cell extract.

Likewise encompassed by the invention is a plant genome containing the nucleic acid according to the invention, *i.e*. the functional remorin gene. No plant can be regenerated from the plant genome. Also encompassed by the invention is the use of the cell extract for the production of sugar (sucrose) or for the production of juice - preferably, beetroot juice. Likewise encompassed by the invention is the sugar - in particular, sucrose - contained in the cells according to the invention and their vacuoles.

An additional aspect of the invention is a seed stock comprising seeds that contain the nucleic acid molecule according to the invention, *i.e.* the functional remorin gene. Said nucleic acid molecule may be present transgenically or endogenously. The seed stock and the seeds may be technically treated. The invention thus also comprises technically-treated seed stock and technically-treated seeds. The various embodiments of technically-treated seed stock are explained in detail in the following whereby the term seed stock also includes seeds: Technically-treated seed stock may be present in polished form. The outermost layer of the seed is thereby removed, so that the seed assumes a more rounded form. This is helpful in sowing, where an optimally uniform shape leads to a uniform distribution of the seed stock grains. Technically-treated seed stock furthermore encompasses pelleted seed stock. The seed stock is thereby embedded in a pelleting mass that protects the seed stock contained therein and leads to a larger mass, such that the pelleted seed stock shows a greater resistance capability with regard to wind drift and is thus less susceptible to being blown away by the wind, and, at the same time, a more precise positioning during sowing is enabled. In a preferred embodiment of the invention, all pelleted seed stock grains of a batch or unit designated for sale have essentially the same shape and the same mass. Deviations of 5% in diameter and mass are possible. However, the deviations preferably do not exceed 1%. As one of the main components, the pelleting mass may contain for example a mineral compound such as clay, bentonite, kaolin, humus and/or peat, for example. It is possible to add an adhesive material like polyacrylamide. Additional possible components are cited in US 4,067,141. Moreover, the pelleting mass may contain additional chemical agents that positively influence the cultivation in practice. These may here be substances that are counted among fertilizing agents. These include compounds rich of one or more of the following elements: Nitrogen, Phosphorus and Potassium (macronutrients). Therefore, the fertilizing ingredients may contain for example Nitrate nitrogen, Ammonium nitrogen, Magnesium Nitrate, Calcium Ammonium Nitrate, Mono Ammonium Phosphate, Mono Potassium Phosphate and Potassium Nitrate. Furthermore, pelleting mass may contain fungicides, insecticides, and/or antifeedants. The fungicides may be thiram and/or hymexazol and/or other fungicides. The insecticide may be a substance from the neonicotinoid group. The substance from the neonicotinoid group is preferably imidacloprid (ATC Code: QP53AX17) and/or clothianidin (CAS number 210880-92-5). Furthermore, the insecticide may also be cyfluthrin (CAS number 68359-37-5), beta-cyfluthrin or tefluthrin. It is worth mentioned that the compound included in the dressing or pelleting mass are taken up by the plant and show systemic effect thereby providing suitable protection of the whole plant. Plants resulting from pelleted seed including one or more pesticides therefore differ from naturally occurring plants and show better performance under biotic stress conditions. In this context the invention also encompasses a mixture of a pelleting mass and a seed according to the invention. Furthermore, the invention also encompasses a method for producing a pelleted seed according to the invention comprising the following steps:
a) providing a sugar beet plant seed comprising the nucleic acid according to the invention, *i*.*e*. the functional remorin gene,
b) embedding the sugar beet plant seed in a pelleting mass, and
c) allowing the pelleting mass to dry, wherein the seed may be optionally a primed or pregerminated seed or the seed may be allowed to be primed during step b).

The pelleted seed stock is a specific embodiment of dressed seed stock. In this context technically-treated seed stock encompasses also the dressed seed stock. However, the invention is not limited to pelleted seed stock, but, rather, may be applied with any form of dressed seed stock. The invention thus also relates to dressed seed stock, which includes pelleted seed stock, but is not limited to this. Dry dressing, wet dressing, and suspension dressing are thus also encompassed. The dressing may thereby also contain at least one dye (coloring), such that the dressed seed stock may be quickly differentiated from undressed seed stock, and, furthermore, good visibility in the environment is ensured after sowing. The dressing may also contain those agrochemicals which are described in the context of the pilling mass. The invention includes thus such dressed seed stock whereby the dressing contains at least one anti-feedant such as an insecticide and / or at least one fungicide. Optionally, so called electonical dressing (dressing by application of electric energy) may be applied. However, electronic dressing is not a dressing in the strict sense of the word.

An additional form of technically-treated seed stock is encrusted seed stock. What is known as coating is also spoken of in this context as well as of seed stock treated with a coating. The difference to pelleted seed stock is that the seed grains retain their original shape, wherein this method is especially economical. The method is described in EP 0 334 258 A1, for example. An additional form of technically-treated seed stock is sprouted or primed seed stock. Sprouted seed stock is pretreated via a pre-germination, whereas primed seed stock has been pretreated via a priming ("germination"). Pre-germinated and primed seed stock have the advantage of a shorter emergence time. The point in time of the emergence after sowing is, at the same time, more strongly synchronized. This enables better agrotechnical processing during cultivation and especially during the harvest, and, additionally, increases the yield quantity. In pre-germination, the seed stock is germinated until the radicle exits the seed stock shell, and the process is subsequently stopped. In the priming, the process is stopped before the radicle exits the seed stock shell. Compared to pre-germinated seed stock, seed stock that has been subjected to a priming is insensitive to the stress of a re-drying and, after such a re-drying, has a longer storage life in comparison to pre-germinated seed stock, for which a re-drying is generally not advised. In this context, technically pre-treated seed stock also includes primed and re-dried seed stock. The process of pre-germination is explained in US 4,905,411 A. Various embodiments of priming are explained in EP 0 686 340 A1. In addition to this, it is also possible to simultaneously pill and prime seed stock in one process. This method is described in EP 2 002 702 B1. Primed seed stock which is moreover pelleted, is encompassed by the present invention.

The technically-treated seed stock may additionally be furnished with one or more of the herbicide resistances explained above. This allows a further-improved agrotechnical cultivation, since the technically-treated seed stock may be deployed on a field that has previously been treated with weed killer, and that therefore is weed-free.

In addition to this, the invention also encompasses a mixture containing the seed stock according to the invention or the seeds according to the invention, and a dressing mass as defined above. The dressing mass is thereby preferably embodied as a pelleting mass, as defined above.

With storage of seed stock according to the invention, storage conditions are preferably to be chosen that do not negatively affect the stability or storage life of the seed stock. Fluctuations in humidity may, especially, have a disadvantageous effect here. Part of the invention is a method for the storage of the seed stock in a bag or container that is via simultaneously water-repellent and breathable. Such a bag or container may be designed as a carton or packing. Such a carton or packing may optionally possess an inner vapor barrier. If the carton or packing is designed as a duplex carton, its stability increases. A container, bag, carton or packing comprising the seed stock according to the invention, or technically-treated seed stock according to the invention, is likewise a part of the invention. It is likewise part of the invention to store seed stock according to the invention or technically-treated seed stock according to the invention in such a bag, container, packing or carton.

In one embodiment, the plant according to the invention is a hybrid plant or a double haploid plant. Hybrid plants and double haploid plants do not occur in nature and cannot be isolated from nature. In a further embodiment of the plant according to the invention, the nucleic acid molecule according to the invention which is involved in Rz2 mediated resistance to BNYVV is present in heterozygous or more preferably in homozygous form. The invention also encompasses hybrid seeds and double haploid seeds which contain said nucleic acid molecule or the corresponding polypeptide.

A further embodiment of the present invention comprises a plant - preferably, of the species *Beta vulgaris* - that is characterized in that the resistance to BNYVV in this plant is further increased.

For example, this may be realized by means of "gene stacking," *i.e.* the resistance is increased using this dose effect. For this, the plants according to the invention that contain the BNYVV resistance-conferring Rz2 gene and the nucleic acid molecule of the present invention which is involved in the Rz2 mediated resistance are over-transformed with at least the latter one or alternative with both mentioned genes in order to increase the amount of the transcription of the gene(s) in the plant. Further possibilities for conferring, restoring or increasing the resistance to BNYVV in a plant of the species *Beta vulgaris* comprising the Rz2 resistance gene are described below.

An additional embodiment of the present invention relates to a sugar beet plant or a portion thereof or a pelleted seed of such a plant which is harvestable before bolting because no bolting of the sugar beet plant occurs during the first 10, 11, 12, 13, 14 or 15 months after germination and the development of a beet body is finished during this period.

In one embodiment of the present invention the sugar beet plant or a portion thereof or a pelleted seed of such a plant has a genome allowing the development of a beet body having a mass summing up to at least 50%, 60%, 70%, 80% or even 90% of the total mass of the full-grown plant.

In another embodiment of the present invention the sugar beet plant or a portion thereof or a pelleted seed of such a plant has a genome allowing the development of a beet body having a minimum mass of 200g, 250g, 300g, 350g, 400g, 450g or 500g and a maximum mass of 1000g, 1100g, 1200g, 1300g, 1400g, 1500g, 1600g, 1700g, 1800g, 1900g or even 2000g via photosynthesis.

An additional embodiment of the present invention is directed to a sugar beet plant or a portion thereof or a pelleted seed of such a plant wherein the genome of the sugar beet plant allows development of a beet body having a sucrose concentration of at least 10%, 11%, 12%, 13%, 14%, 15%, 16%, 17%, 18%, 19% or even 20%.

The present invention further encompasses a method for conferring, restoring or increasing the resistance to BNYVV in a plant of the species *Beta vulgaris* comprising the Rz2 resistance gene endogenously or transgenically mediating BNYVV resistance. Possible ways for conferring, restoring or increasing said resistance are listed in the following, however without being limited to those.

Conferring, increasing and/or restoring the resistance against BNYVV may take place via integration of the nucleic acid molecule according to the invention, *i.e.* the functional remorin gene, into the genome of at least one cell of a plant of the species *Beta vulgaris,* as well as possible regeneration of a plant from the plant cell. The integration may take place both by means of sexual crossing, *e.g.,* crossing of a plant of the genus *Beta* comprising the Rz2 resistance gene mediating BNYVV resistance endogenously or transgenically with a plant comprising the functional remorin gene of the present invention, optionally in combination with the Rz2 resistance gene, and subsequent selection, and by means of homology-directed repair or homologous recombination. The two latter methods cited are preferably supported by site-directed nucleases which may be selected from, but are not limited to, the following: CRISPR nuclease, including Cas9, CasX, CasY, or Cpfl nuclease, TALE nuclease, zinc finger nuclease, meganuclease, Argonaut nuclease, restriction endonuclease, including FokI or a variant thereof, recombinase, or two, site-specific, nicking endonucleases.

Another way for conferring, increasing and/or restoring the resistance against BNYVV may take place via transformation of a plant cell with the nucleic acid molecule according to the invention, *i.e*. the functional remorin gene, the corresponding recombinant DNA molecule, or with the vector or the expression cassette of the invention, and regeneration of the transgenic plant from the transformed plant cell.

In general, the activity and/or stability of the polypeptide which is encoded by the nucleic acid molecule of the present invention which modifies the Rz2 mediated resistance to BNYVV, *i.e.* the functional remorin gene as well as the expression of the corresponding nucleic acid molecule can be increased via modification of the nucleic acid sequence of the functional remorin gene and/or its native promoter.

The mutations leading to said modifications can be either performed within the organism itself, or it is possible to modify the corresponding nucleic acids chemically in an isolated state to achieve the desired effects. The advantage of the latter approach is that the compounds can be edited even more precisely.

The method for the production of an organism which comprises a mutated version of the nucleic acid molecule according to the above given embodiment [1] or [2] and/or a mutated version of the native promoter includes the following steps:
(I) provision of an organism or a cell comprising the nucleic acid molecule and/or the promoter;
(II) increase of the mutation rate of the organism or the cell or mutagenesis of the organism or the cell;
(III) phenotypic selection of an organism, which as a result of a mutation exhibits an altered resistance or altered resistance level towards BNYVV or genotypic selection of an organism or a cell which comprises a mutation in the nucleic acid molecule and / or the promoter, wherein the mutation has been created via step (II); and, optionally
(IV) regeneration of the organism from the cell obtained via step (III).

The organism can be a plant. Preferably the plant is a *Beta vulgaris.* However, it is also possible to use unicellular organisms as bacteria. The bacterium can be *E. coli.* If the organism is a plant then the method can be applied *in vivo* as well as *in vitro.* If the organism is a plant and the method is applied *in vitro,* a cell culture of the plant may be established and the increase of the mutation rate or the mutagenization may occur in the cell culture. The increase of the mutation rate encompasses for example the application of mutagenic agents like for example 5-bromouracil or ethylmethane sulfonate (EMS) or the application of physical mutagens like ionizing radiation or UV light. The mutagenization encompasses also the targeted mutagenesis. The targeted mutagenesis can be achieved by precise methods as gene editing (as explained further below). The regeneration of organism out of cells is explained in various standard references of the cell biology. The regeneration of plants is for example explained in the standard reference "Plant biotechnology: comprehensive biotechnology, second supplement" (Michael W. Fowler, Graham Warren, Murray Moo-Young - Pergamon Press - 1992). The regeneration of *Beta vulgaris* out of the cell culture is described in Lindsey & Gallois (1990).

These references also describe how plant cell cultures are established. The mutated version of the nucleic acid molecule respectively the promoter characterizes themselves preferably due to the expression rate of the nucleic acid molecule of the present invention, *i.e.* the functional remorin gene which is increased by the mutation. Such an effect can also rely on the presence of several mutations. For example, it is possible to introduce two, three, four, five or more mutations in the promoter or the nucleic acid molecule.

By the introduction of mutations thus more resistance imparting protein can be built in the cell or the protein has a better effect. Thereby, the resistance in comparison to a control plant comprising the unaltered nucleic acid molecule according to the invention, *i.e.* the functional remorin gene, can be increased for example by at least 1, 2, 3, 4, 5 or more percent. The increase can be measured as explained further below. Moreover, the resistance due to the mutation or mutations can be increased by at least one rating score. The determination of rating scores is explained elsewhere herein. Furthermore, the protein modifying the Rz2 mediated resistance can impart - as a result of the mutations - an altered effect and in some circumstances can exhibit effect against such pathogens which have adapted themselves to the initial resistance mechanism. In this context the invention encompasses also such mutated variants of the nucleic acids according to the invention and mutated variants of the protein according to the invention. Preferably the invention encompasses such variants which do not occur in nature and cannot be isolated from nature to make sure that the pathogen had no opportunity to adapt itself to such variants. The above described method for the production of an organism which comprises a mutated version of the nucleic acid molecule may furthermore include a further step, in which those organisms or respectively plants are identified, which have a further increased resistance due to the mutation or mutations. If an increase of resistance has taken place may be determined by the herein explained rating scores or the measuring of the resistance level.

As already mentioned before, besides the above described method for the production of organisms which comprise a mutated version of the nucleic acid molecule or of the promoter it is also possible to modify the according nucleic acids chemically in an isolated state to achieve the desired effects. For this purpose, the following method is offered:
Production of a chemically modified nucleic acid molecule according to the above given embodiment [1] or [2] and / or a chemically modified promoter comprises the following steps:
(I) provision of the nucleic acid molecule as stated above in isolated form;
(II) chemical modification of the nucleic acid molecule or the promoter by one of the following steps:
(IIa) mutagenization,
(IIb) gene editing,
(IIc) restriction and ligation respectively insertion or deletion.

Furthermore, chemical modifications can be generated by such approaches as stated elsewhere herein in context of allelic variants. The gene editing given under step (II) above is equal to the term "Genome-Editing". Optionally the chemically modified nucleic acid molecule or the chemically modified promoter can be subsequently introduced into a cell or can be stably integrated. With the help of such a cell, the chemically modified nucleic acid molecule and the modified promoter can be propagated in context of the cell proliferation. They can be subsequently isolated in vast number and expression analyses may be performed. Expression analyses are especially suitable when the chemical modification concerns the promoter. It is possible to harvest the cells and to isolate the chemically modified resistance protein for chemical analyses. If the cell which comprises the chemically modified nucleic acid molecule or the modified promoter is a plant cell, a complete plant may be regenerated out of this cell. The approaches described within this passage can be performed subsequently to the above given method for the production of a modified form of the nucleic acid molecule and / or a modified promoter and the obtained variants are also a part of this invention. Moreover, a plant comprising the chemically modified nucleic acid molecule or the modified promoter are also part of the invention. Thus, the invention is also related to a plant obtained by this method. Furthermore, the invention relates also to the chemically modified nucleic acid molecules obtained by this method and to the encoded polypeptides. These compounds may be optimized versions of the original (not modified) compounds, wherein the resulting resistance level - as explained further above - may be increased by at least by 1, 2, 3, 4, 5, or more percent or may be increased by at least one rating score. In this regard the method for the production of a chemically modified nucleic acid molecule is also a method for the optimization of the nucleic acid molecule. The method for optimization may furthermore contain an additional step, in which those modified variants of the nucleic acid molecule are identified which lead in comparison to the unamended variants to an increased resistance in a plant.

In one embodiment, increasing said resistance to BNYVV can be performed via increasing the expression of the nucleic acid molecule of the present invention which modifies the Rz2 mediated resistance to BNYVV, *i.e.* the functional remorin gene, in the plant. This may take place via modification of the native promoter, wherein the modification preferably takes place by means of gene editing or site-directed mutagenesis which is mediated via site-directed nucleases, and, optionally, repair models. Examples of such nucleases have already been cited above. The increase in the expression of the nucleic acid molecule according to the invention may likewise take place via fusion of the nucleic acid molecule with a heterologous promoter, which exhibits a higher activity in comparison to the native promoter - in particular, after BNYVV infection. The fusion may likewise take place via site-directed nuclease and repair models, but also by means of direct insertion after double-strand break.

As has already been mentioned above, a method for increasing the BNYVV resistance, may also be performed via increasing the activity and/or stability of the polypeptide according to the invention, via modification of the nucleotide sequence of the nucleic acid molecule according to the invention and may be performed by means of the techniques known to the person skilled in the art. This approach is explained in detail further below.

All the mentioned approaches dealing either with the introduction of the nucleic acid molecule of the present invention, *i.e.* the functional remorin gene, into the genome of a target plant or via modification of said nucleic acid molecule to increase its expression and the stability/activity of the corresponding protein can be optionally performed in combination with inhibiting the expression of the non-functional remorin allele.

Alternatively, a method for conferring, restoring or increasing the resistance against BNYVV might be performed via mutagenesis of a plant or plant cells comprising the nucleic acid molecule of the present invention interfering with the BNYVV resistance, *i.e.* the non-functional remorin allele and screening and optionally selecting for plants or plant cells in which a functional allele of the polypeptide encoded by the functional remorin allele has been restored and/or the expression of the non-functional polypeptide has been abolished. This can be performed by means of random or directed mutagenesis of the nucleic acid sequence of the non-functional remorin gene. Examples of polymorphisms which differentiate the sensitive allele from the resistant allele are presented in Tables 1 and 2.

For example, the non-functional allele may be modified via gene mutation by means of TALE nucleases (TALEN's) or zinc finger nucleases (ZFN's), as well as CRISPR/Cas systems, which - among other things - are described by way of example in WO 2014/144155 A1 (Engineering plant genomes using CRISPR/Cas systems) and in Osakabe & Osakabe, Plant Cell Physiol., 56 (2015), 389-400. This may also be achieved via use of the method designated as TILLING (*Targeted Induced Local Lesions in Genomes*), wherein it is described, *e.g.,* in the German patent application DE 10 2013 101 617, how point mutations are caused in a sensitive gene, and plants are subsequently selected that exhibit a suitable, *i.e.,* resistance-conferring, mutation, *e.g.,* a barley resistant to yellow mosaic virus; see DE 10 2013 101 617 on pp. 4, 8, and 12, in paragraphs [0014], [0026], and [0038]. The TILLING method is also described in detail in the publication by Henikoff *et al.* (Henikoff et al., Plant Physiol. 135, 2004, 630-636).

In order to enable a break at a predetermined target site, the enzymes for targeted mutagenesis preferably include a binding/recognition domain and a cleavage domain. Particular enzymes capable of inducing double or single-stranded breaks are nucleases or nickases (DSBI - double strand break inducing- or SSBI - single strand break inducing - enzymes) as well as variants thereof, including such molecules no longer comprising a nuclease or nickase function but rather operating as recognition molecules in combination with another enzyme. In recent years, many suitable nucleases, especially tailored endonucleases have been developed comprising meganucleases, zinc finger nucleases, TALE nucleases, Argonaute nucleases, derived, for example, from *Natronobacterium gregoryi,* and CRISPR nucleases, comprising, for example, Cas9, Cpf1, Csm1, CasX or CasY nucleases as part of the Clustered Regularly Interspaced Short Palindromic Repeats (CRISPR) system. Thus, in a preferred aspect of the invention, the genome engineering component comprises a DSB- or SSB-inducing enzyme or a variant thereof selected from a CRISPR/Cas endonuclease, preferably a CRISPR/Cas9 endonuclease a CRISPR/Cpf1 endonuclease, or a CRISPR/Csm1 endonuclease, a zinc finger nuclease (ZFN), a homing endonuclease, a meganuclease and a TAL effector nuclease.

Rare-cleaving endonucleases are DSBI/SSBI enzymes that have a recognition site of preferably about 14 to 70 consecutive nucleotides, and therefore have a very low frequency of cleaving, even in larger genomes such as most plant genomes. Homing endonucleases, also called meganucleases, constitute a family of such rare-cleaving endonucleases. They may be encoded by introns, independent genes or intervening sequences, and present striking structural and functional properties that distinguish them from the more classical restriction enzymes, usually from bacterial restriction-modification Type II systems. Their recognition sites have a general asymmetry which contrast to the characteristic dyad symmetry of most restriction enzyme recognition sites. Several homing endonucleases encoded by introns or inteins have been shown to promote the homing of their respective genetic elements into allelic intronless or inteinless sites. By making a site-specific double strand break in the intronless or inteinless alleles, these nucleases create recombinogenic ends, which engage in a gene conversion process that duplicates the coding sequence and leads to the insertion of an intron or an intervening sequence at the DNA level. A list of other rare cleaving meganucleases and their respective recognition sites is provided in Table I of WO 03/004659 (pages 17 to 20) (incorporated herein by reference).

Furthermore, methods are available to design custom-tailored rare-cleaving endonucleases that recognize basically any target nucleotide sequence of choice. Briefly, chimeric restriction enzymes can be prepared using hybrids between a zinc-finger domain designed to recognize a specific nucleotide sequence and the non-specific DNA-cleavage domain from a natural restriction enzyme, such as Fokl. Such methods have been described e.g. in WO 03/080809, WO 94/18313 or WO 95/09233 and in Isalan et al. (2001). A rapid, generally applicable method to engineer zinc fingers illustrated by targeting the HIV-1 promoter. Nature biotechnology, 19(7): 656; Liu et al. (1997). Design of polydactyl zinc-finger proteins for unique addressing within complex genomes. Proceedings of the National Academy of Sciences, 94(11): 5525-5530.

Another example of custom-designed endonucleases includes the TALE nucleases (TALENs), which are based on transcription activator-like effectors (TALEs) from the bacterial genus *Xanthomonas* fused to the catalytic domain of a nuclease (e.g. *Fok*I or a variant thereof). The DNA binding specificity of these TALEs is defined by repeat-variable di-residues (RVDs) of tandem-arranged 34/35-amino acid repeat units, such that one RVD specifically recognizes one nucleotide in the target DNA. The repeat units can be assembled to recognize basically any target sequences and fused to a catalytic domain of a nuclease create sequence specific endonucleases (see e.g. Boch et al. (2009). Breaking the code of DNA binding specificity of TAL-type III effectors. Science, 326(5959), 1509-1512; Moscou & Bogdanove (2009). A simple cipher governs DNA recognition by TAL effectors. Science, 326(5959), 1501-1501; and WO 2010/079430, WO 2011/072246, WO 2011/154393, WO 2011/146121, WO 2012/001527, WO 2012/093833, WO 2012/104729, WO 2012/138927, WO 2012/138939). WO 2012/138927 further describes monomeric (compact) TALENs and TALEs with various catalytic domains and combinations thereof.

Recently, a new type of customizable endonuclease system has been described; the so-called CRISPR/Cas system. A CRISPR system in its natural environment describes a molecular complex comprising at least one small and individual non-coding RNA in combination with a Cas nuclease or another CRISPR nuclease like a Cpfl nuclease or a Csm1 nuclease (Zetsche et al., "Cpfl Is a Single RNA-Guides Endonuclease of a Class 2 CRISPR-Cas System", Cell, 163, pp. 1-13, October 2015.; US 2017/0233756 A1) which can produce a specific DNA double-stranded break. Presently, CRISPR systems are categorized into 2 classes comprising five types of CRISPR systems, the type II system, for instance, using Cas9 as effector and the type V system using Cpfl as effector molecule (Makarova et al., Nature Rev. Microbiol., 2015). In artificial CRISPR systems, a synthetic non-coding RNA and a CRISPR nuclease and/or optionally a modified CRISPR nuclease, modified to act as nickase or lacking any nuclease function, can be used in combination with at least one synthetic or artificial guide RNA or gRNA combining the function of a crRNA and/or a tracrRNA (Makarova *et al.,* 2015, supra). The immune response mediated by CRISPR/Cas in natural systems requires CRISPR-RNA (crRNA), wherein the maturation of this guiding RNA, which controls the specific activation of the CRISPR nuclease, varies significantly between the various CRISPR systems which have been characterized so far. Firstly, the invading DNA, also known as a spacer, is integrated between two adjacent repeat regions at the proximal end of the CRISPR locus. Type II CRISPR systems code for a Cas9 nuclease as the key enzyme for the interference step, which system contains both a crRNA and also a trans-activating RNA (tracrRNA) as the guide motif. These hybridize and form double-stranded (ds) RNA regions which are recognized by RNAseIII and can be cleaved in order to form mature crRNAs. These then in turn associate with the Cas molecule in order to direct the nuclease specifically to the target nucleic acid region. Recombinant gRNA molecules can comprise both the variable DNA recognition region and also the Cas interaction region and thus can be specifically designed, independently of the specific target nucleic acid and the desired Cas nuclease.

As a further safety mechanism, PAMs (protospacer adjacent motifs) must be present in the target nucleic acid region; these are DNA sequences which follow on directly from the Cas9/RNA complex-recognized DNA. The PAM sequence for the Cas9 from *Streptococcus pyogenes* has been described to be "*NGG*" or "*NAG*" (Standard IUPAC nucleotide code) (Jinek et al, "A programmable dual-RNA-guided DNA endonuclease in adaptive bacterial immunity", Science 2012, 337: 816-821). The PAM sequence for Cas9 from *Staphylococcus aureus* is "*NNGRRT*" or "*NNGRR(N)*". Further variant CRISPR/Cas9 systems are known. Thus, a *Neisseria meningitidis* Cas9 cleaves at the PAM sequence NNNNGATT. A *Streptococcus thermophilus* Cas9 cleaves at the PAM sequence NNAGAAW. Recently, a further PAM motif NNNNRYAC has been described for a CRISPR system of *Campylobacter* (WO 2016/021973 A1). For Cpfl nucleases it has been described that the Cpfl-crRNA complex, without a tracrRNA, efficiently recognize and cleave target DNA proceeded by a short T-rich PAM in contrast to the commonly G-rich PAMs recognized by Cas9 systems (Zetsche et al., supra). Furthermore, by using modified CRISPR polypeptides, specific single-stranded breaks can be obtained. The combined use of Cas nickases with various recombinant gRNAs can also induce highly specific DNA double-stranded breaks by means of double DNA nicking. By using two gRNAs, moreover, the specificity of the DNA binding and thus the DNA cleavage can be optimized. Further CRISPR effectors like CasX and CasY effectors originally described for bacteria, are meanwhile available and represent further effectors, which can be used for genome engineering purposes (Burstein et al., "New CRISPR-Cas systems from uncultivated microbes", Nature, 2017, 542, 237-241).

The cleavage site of a DSBI/SSBI enzyme relates to the exact location on the DNA or RNA where the break is induced. The cleavage site may or may not be comprised in (overlap with) the recognition site of the DSBI/SSBI enzyme and hence it is said that the cleavage site of a DSBI/SSBI enzyme is located at or near its recognition site. The recognition site of a DSBI/SSBI enzyme, also sometimes referred to as binding site, is the nucleotide sequence that is (specifically) recognized by the DSBI/SSBI enzyme and determines its binding specificity. For example, a TALEN or ZNF monomer has a recognition site that is determined by their RVD repeats or ZF repeats respectively, whereas its cleavage site is determined by its nuclease domain (e.g. FokI) and is usually located outside the recognition site. In case of dimeric TALENs or ZFNs, the cleavage site is located between the two recognition/binding sites of the respective monomers, this intervening DNA or RNA region where cleavage occurs being referred to as the spacer region.

A person skilled in the art would be able to either choose a DSBI/SSBI enzyme recognizing a certain recognition site and inducing a DSB or SSB at a cleavage site at or in the vicinity of the preselected/predetermined site or engineer such a DSBI/SSBI enzyme. Alternatively, a DSBI/SSBI enzyme recognition site may be introduced into the target genome using any conventional transformation method or by crossing with an organism having a DSBI/SSBI enzyme recognition site in its genome, and any desired nucleic acid may afterwards be introduced at or in the vicinity of the cleavage site of that DSBI/SSBI enzyme.

As used herein, a "double-stranded DNA break inducing enzyme", "enzyme inducing a double-stranded break", or "DSBI enzyme" is an enzyme capable of inducing a double-stranded DNA break at a particular nucleotide sequence, called the "recognition site" or "predetermined site" or "predetermined location". Accordingly, a "single-stranded DNA or RNA break inducing enzyme", "enzyme inducing a single-stranded break", or "SSBI enzyme" is an enzyme capable of inducing a single-stranded DNA or RNA break at a particular nucleotide sequence, called the "recognition site" or "predetermined site" or "predetermined location".

As used herein, a "repair nucleic acid molecule" or "repair matrix" is a single-stranded or double-stranded DNA molecule or RNA molecule that is used as a template for modification of the genomic DNA or the RNA at the preselected site in the vicinity of or at the cleavage site. As used herein, "use as a template for modification of the genomic DNA", means that the repair nucleic acid molecule is copied or integrated at the preselected site by homologous recombination between the flanking region(s) and the corresponding homology region(s) in the target genome flanking the preselected site, optionally in combination with non-homologous end-joining (NHEJ) at one of the two end of the repair nucleic acid molecule (e.g. in case there is only one flanking region).

As used herein, "a modification of the genome", means that the genome has changed in at least one nucleotide or by at least one epigenetic editing.

As used herein "a preselected site", "a predetermined site" or "predefined site" indicates a particular nucleotide sequence in the genome (e.g. the nuclear genome or the chloroplast genome) at which location it is desired to insert, replace and/or delete one or more nucleotides.

In various embodiments, the at least one base editor according to the present invention is temporarily or permanently linked to at least one site-specific DSBI/SSBI enzyme complex or at least one modified site-specific DSBI/SSBI enzyme complex, or optionally to a component of said at least one site-specific DSBI/SSBI enzyme complex. The linkage can be covalent and/or non-covalent. Any base editor or site-specific DSBI/SSBI enzyme complex, or a catalytically active fragment thereof, or any component of a base editor complex or of a site-specific DSBI/SSBI enzyme complex as disclosed herein can be introduced into a cell as a nucleic acid fragment, the nucleic acid fragment representing or encoding a DNA, RNA or protein effector, or it can be introduced as DNA, RNA and/or protein, or any combination thereof.

The base editor is a protein or a fragment thereof having the capacity to mediate a targeted base modification, i.e., the conversion of a base of interest resulting in a point mutation of interest. Preferably, the at least one base editor in the context of the present invention is temporarily or permanently fused to at least one DSBI/SSBI enzyme, or optionally to a component of at least one DSBI/SSBI. The fusion can be covalent and/or non-covalent. Multiple publications have shown targeted base conversion, primarily cytidine (C) to thymine (T), using a CRISPR/Cas9 nickase or non-functional nuclease linked to a cytidine deaminase domain, Apolipoprotein B mRNA-editing catalytic polypeptide (APOBEC1), e.g., APOBEC derived from rat. The deamination of cytosine (C) is catalyzed by cytidine deaminases and results in uracil (U), which has the base-pairing properties of thymine (T). Most known cytidine deaminases operate on RNA, and the few examples that are known to accept DNA require single-stranded (ss) DNA. Studies on the dCas9-target DNA complex reveal that at least nine nucleotides (nt) of the displaced DNA strand are unpaired upon formation of the Cas9-guide RNA-DNA 'R-loop' complex (Jore et al., Nat. Struct. Mol. Biol., 18, 529-536 (2011)). Indeed, in the structure of the Cas9 R-loop complex, the first 11 nt of the protospacer on the displaced DNA strand are disordered, suggesting that their movement is not highly restricted. It has also been speculated that Cas9 nickase-induced mutations at cytosines in the non-template strand might arise from their accessibility by cellular cytosine deaminase enzymes. It was reasoned that a subset of this stretch of ssDNA in the R-loop might serve as an efficient substrate for a dCas9-tethered cytidine deaminase to effect direct, programmable conversion of C to U in DNA (Komor et al., supra). Recently, Goudelli et al., Programmable base editing of A• T to G• C in genomic DNA without DNA cleavage, Nature, 2017, 551(7681), 464, described adenine base editors (ABEs) that mediate the conversion of A•T to G•C in genomic DNA.

These methods preferably lead to an improvement in the resistance by at least one rating score - particularly preferably, to an improvement in the resistance by at least two, three, or more rating scores. After mutagenesis of the plant cells and subsequent regeneration of plants from the mutagenized plant cells, or mutagenesis of plants, the plants may then be identified that exhibit one or more mutations, as depicted in Tables 1 and 2, in an endogenous nucleic acid molecule. In this context, the already mentioned plant according to the invention may be characterized by that the resistance is increased by at least one rating score, preferably by at least two or more rating scores. Alternatively, the resistance of the plants according to the invention may be increased for example by at least 1, 2, 3, 4, 5 or more percent in comparison to a control plant, which does not comprise the nucleic acid according to the invention and/or which comprises the non-functional variant thereof. The increase can be measured by inoculation of one healthy leaf with an isolate of the pathogen and the determination of the infested surface after 15 days. A reduce of 5% of the infested surface corresponds to an increase of the resistance of 5%. Further parameters for the conduction of the measuring can be derived from the below given embodiment "resistance test".

The method for production of a BNYVV-resistant plant comprising the nucleic acid molecule of the present invention, *i.e.* the functional remorin gene, alternatively includes, as described above, the provision of a plant of the genus *Beta* or a plant cell of the genus *Beta* comprising the Rz2 resistance gene mediating BNYVV resistance endogenously or transgenically, transformation of said plant cell with the nucleic acid molecule of the present invention, *i.e*. the functional remorin gene, or the corresponding vector or expression cassette and regeneration of a transgenic plant from the transformed plant cell.

In an alternative embodiment, the method for production of a BNYVV-resistant plant comprises the provision of a plant of the genus *Beta* or a plant cell of the genus *Beta* comprising the Rz2 resistance gene mediating BNYVV resistance endogenously or transgenically, the introduction of a site-directed nuclease or nickase and optionally a repair matrix or a site-directed base editor into a cell of the plant, wherein the site-directed nuclease is able to generate at least one single-strand break of the DNA or at least one double-strand break of the DNA in the genome of the cell - preferably in the nucleic acid molecule of the present invention interfering with the BNYVV resistance, *i.e*. the non-functional remorin, or upstream or downstream from said nucleic acid molecule - and the repair matrix comprises the nucleic acid molecule according to the invention which is involved in BNYVV resistance, *i.e*. the functional remorin gene, or wherein the site-directed base editor is able to generate at least one single-strand break of the DNA at one of the mentioned locations and to convert at least one nucleobase. The nucleobases which are preferably to be converted are listed in Table 1, above. The method furthermore optionally includes the cultivation of the generated cell under conditions that allows modification of the genome at the mentioned location, wherein the modification can be a replacement, a deletion and/or an insertion of at least one nucleotide. The modification might optionally occur by homology-directed repair or homologous recombination, wherein the nucleic acid molecule is integrated into the genome of the plant. However, substitution of single nucleotide might also occur with base editors, e.g. CRISPR tools; thus the repair matrix is not necessarily required. Furthermore, the regeneration of a plant from the modified plant cell is encompassed.

The method for production of a BNYVV-resistant plant comprising the nucleic acid molecule of the present invention, *i.e.* the functional remorin gene, alternatively includes the crossing of a plant of the genus *Beta* comprising the Rz2 resistance gene mediating BNYVV resistance endogenously or transgenically with a plant comprising the functional remorin gene of the present invention, optionally in combination with the Rz2 resistance gene, and identifying and optionally selecting a plant comprising the Rz2 resistance gene and the nucleic acid molecule of the present invention, *i.e.* the functional remorin gene both endogenously or transgenically, wherein the Rz2 resistance gene and/or said nucleic acid molecule of the present invention is present homozygously in the genome of the plant.

The present invention likewise relates to a method for the identification, and possibly the provision, of a plant of the species *Beta vulgaris,* of a portion or a seed thereof comprising the nucleic acid molecule of the present invention, *i.e*. the functional remorin gene, and which is capable of exhibiting resistance against BNYVV mediated by the Rz2 gene, characterized in that the method includes a step of the detection of the presence and/or of the expression of said nucleic acid molecule or of the encoded polypeptide in the plant or a sample, a portion or a seed thereof. Accordingly, the presence and expression, respectively, of said nucleic acid molecule indicates that the plant is capable of exhibiting Rz2 mediated resistance against BNYVV. Alternatively, the absence and lack of expression, respectively, of said nucleic acid molecule and/or the presence and/or expression of the nucleic acid molecule of the present invention interfering with the resistance against BNYVV, *i.e.* the non-functional remorin gene indicates that the plant is not capable of or impaired in exhibiting Rz2 mediated resistance against BNYVV. In addition, the method may include the identification and optionally selection of the plant of the species *Beta vulgaris,* the portion or the seed thereof comprising the nucleic acid molecule of the present invention, *i.e*. the functional remorin gene, which is capable of exhibiting resistance against BNYVV mediated by Rz2, if present in the genome of the plant.

In the same way, the present invention relates to a method for the identification and optionally selection of a plant of the species *Beta vulgaris* comprising the nucleic acid molecule of the present invention which interferes with the resistance against BNYVV, *i.e.* the non-functional remorin gene, characterized in that the method includes a step of the detection of the presence and/or of the expression of said nucleic acid molecule or of the encoded polypeptide in the plant or a sample, a portion or a seed thereof.

The presence and/or the expression of a nucleic acid molecule according to the invention, or of the polypeptide according to the invention, may be tested by means of standard methods known to the person skilled in the art, *e.g.,* by means of PCR, RT-PCR, or Western Blot.

With the fine mapping, the position of the remorin gene in the genome of *Beta vulgaris* subsp. *vulgaris* has been determined, and the gene itself and the surrounding sequence regions have been identified. In particular, remorin is positioned about 0.75 cM distal to the Rz2 gene on the long arm of chromosome 3. This in turn represents the basis for the development of DNA hybridization probes or genetic markers in the target region allowing to distinguish between plants comprising the functional remorin allele and plants comprising the non-functional remorin allele and allowing to develop markers in one or more coseggregating regions which can be used to identify recombinants between the genes and thus to separate the repressor allele from the Rz2 resistance allele. The genetic position of both genes in relation to each other are shown in Figure 7.

DNA hybridization probes may be derived from the sequence of the remorin gene and be used for the screening of genomic and/or cDNA banks of the desired organism. The probes may be used to amplify identified homologous genes via the known process of polymerase chain reaction (PCR), and to check whether the remorin gene is present endogenously in an organism, or has been successfully introduced heterologously.

The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. The person skilled in the art may also synthesize and use oligonucleotide primers to amplify sequences of the remorin gene. In order to achieve a specific hybridization, such probes should be specific and have at least a length of 15 nucleotides - preferably, at least 20 nucleotides. A detailed guide to hybridization of nucleic acids may be found in Tijssen, Laboratory Techniques in Biochemistry and Molecular Biology - Hybridization with Nucleic Acid Probes, Part 1, Chapter 2, "Overview of principles of hybridization and the strategy of nucleic acid probe assays." Elsevier, New York (1993); and in Current Protocols in Molecular Biology, Chapter 2, Ausubel et al., eds., Greene Publishing and Wiley lnterscience, New York (1995).

Thus, in one embodiment, the present invention relates to oligonucleotides of at least 15, 16, 17, 18, 19, or 20 - preferably, at least 21, 22, 23, 24, or 25, particularly preferably, at least 30, 35, 40, 45, or 50, and, especially preferably, at least 100, 200, 300, 500 or 1,000 - nucleotides in length that specifically hybridize with a nucleotide sequence of the nucleic acid molecule according to the invention which is involved in Rz2 mediated resistance against BNYVV, or that specifically hybridize with a nucleotide sequence of the nucleic acid molecule according to the invention which interferes with the resistance against BNYVV, or the nucleic acid molecules that are complementary to both nucleic acid sequences. Furthermore, the invention relates to a pair of nucleic acid molecules - preferably, in the form of oligonucleotides - that is suitable for attachment as a forward and reverse primer to a region that is specific to one of said nucleic acid molecules, and for amplifying these in a polymerase chain reaction (PCR).

The present invention thus also relates to markers as oligonucleotides - in particular, primer oligonucleotides. These comprise a nucleic acid molecule of at least 15 nucleotides in length that specifically hybridizes with a nucleotide sequence defined as in the preceding.

In particular, the present invention encompasses a pair of nucleic acid molecules - preferably, in the form of oligonucleotides or a kit containing this pair of oligonucleotides - that is suitable for hybridization as a forward and reverse primer to a region that is specific to the nucleic acid molecule according to the invention, and for amplifying this in a polymerase chain reaction (PCR), or that is suitable as a forward and reverse primer for hybridization to a region in the *Beta vulgaris* genome that, in *Beta vulgaris,* exhibits a cosegregation with the nucleic acid molecule according to the invention which is involved in Rz2 mediated resistance against BNYVV, *i.e.* with the functional remorin gene, or with the nucleic acid molecule according to the invention which interferes with the resistance against BNYVV, *i.e.* the non-functional remorin gene. Alternatively or additionally, the oligonucleotide or the pair of oligonucleotides is suitable for hybridization to a region in the *Beta vulgaris* genome that, in *Beta vulgaris,* cosegregates with the functional remorin gene together with the Rz2 gene, or with the non-functional remorin gene together with the Rz2 gene.

**Table 3: Markers flanking remorin and the Rz2 gene as shown in Figure 7.**

| marker name | genetic position [cM] | physical position [bp] | nucleobases in the genome of *Beta vulgaris* comprising the non-functional remorin gene | SEQ ID No: |
|---|---|---|---|---|
| s3e5985s01 | 36,9318639 | 7077769 | C | 33 |
| sxh1002s02 | 39,0210334 | 7957425 | G | 34 |
| sxh2499s01 | 39,9958561 | 8403820 | A | 35 |
| sxi0698s01 | 40,5090706 | 8648395 | T | 36 |
| sxh8485s01 | 42,3807876 | 9605012 | A | 37 |
| sxe7357s01 | 42,8377616 | 9856523 | C | 38 |
| sxn1017s02 | 43,6903085 | 10348480 | T | 39 |
| s3e2247xxx | 45,7583869 | 11699138 | A | 40 |

In a preferred embodiment, the oligonucleotide or the pair of oligonucleotides of the present invention is selected from the group consisting of those set forth in SEQ ID Nos. 24 to 40. In particular, 74 markers have been identified between remorin and Rz2, three of which are exemplified in Figure 7: *s3e5782s01* (SEQ ID NO: 30; non-functional allele: T), *s3e4913xxx* (SEQ ID NO: 31; non-functional allele: T) and *s3e5800s01* (SEQ ID NO: 32; non-functional allele: T). Figure 7 also shows marker positions distal to Remorin and proximal to Rz2, which define flanking areas that can be used for marker assisted breeding (MAS) with the aim of introducing a functional remorin. These markers and their respective genetic and physical positions are shown in Tables 3 to 5.

**Table 4: Markers located between remorin and the Rz2 gene as shown in Figure 7.**

| marker name | genetic position [cM] | physical position [bp] | nucleobases in the genome of *Beta vulgaris* comprising the non-functional remorin gene | SEQ ID No: |
|---|---|---|---|---|
| s3e5782s01 | 41,15 | 8961406 | T | 30 |
| s3e4913xxx | 41,38 | 9079399 | T | 31 |
| s3e5800s01 | 41,73 | 9260730 | T | 32 |

**Table 5: Markers located in the remorin gene as shown in Figure 7.**

| marker name | genetic position [cM] | physical position [bp] | nucleobases in the genome of *Beta vulgaris* comprising the non-functional remorin gene | SEQ ID No: |
|---|---|---|---|---|
| s3p4348s01 | ∼41,0 | 8886686 | G | 27 |
| s3p4349s01 | ∼41,0 | 8886781 | T | 28 |
| s3p4351s01 | ∼41,0 | 8891242 | T | 29 |

Furthermore, the oligonucleotide according to the invention may be connected to a fluorescent dye in order to generate a fluorescence signal, *e.g.,* under excitation via light of the corresponding wavelength. The fluorescent dye may be fluorochrome. The oligonucleotides according to the invention may be coupled with other compounds that are suitable for generating a signal. Such oligonucleotides do not occur in nature and also cannot be isolated from nature. The following is executed to produce such marked oligonucleotides: DNA may be marked bio-orthogonally. For this, DNA may be marked in vivo or in vitro with nucleoside analogs, which, for example, may subsequently be coupled with a fluorophore per Staudinger reaction. In addition to this, DNA may also be chemically provided with fluorophores. Oligonucleotides may be marked via a phosphoramidite synthesis with fluorophores that, for example, are used in QPCR, DNA sequencing, and in situ hybridization. Furthermore, DNA may be generated enzymatically in the course of a polymerase chain reaction with fluorescent nucleotides, or be marked with a ligase or a terminal deoxynucleotidyl transferase. DNA may also be detected indirectly via a biotinylation and fluorescent avidin. For couplings, fluorescein, fluorescent lanthanides, gold nanoparticles, carbon nanotubes, or quantum dots, among other things, are used as fluorophores. One of the most commonly used fluorescent substances is FAM (carboxyfluorescein). Consequently, oligonucleotides and, in particular, primers that possess a FAM marking are encompassed by the invention. FAM is preferably present as 6-FAM, wherein - depending upon the desired wavelength of the emission and excitation - other FAM variants, *e.g.,* 5-FAM, may, however, also be used. Examples of additional fluorescence markers are AlexaFluor, ATTO, Dabcyl, HEX, Rox, TET, Texas Red, and Yakima Yellow. Depending upon the field of use, the oligonucleotides may be furnished with modifications of the bases or of the sugar phosphate spine. Among these are, among others, amino-dT, azide-dT, 2-aminopurine,5-Br-dC, 2'-deoxyinosine (INO), 3'-deoxy-A, C, G, 5-Met-dC, 5-OH-Met-dCN6-Met-dA, and others.

Thus, the identification method according to the invention also includes the detection of the nucleic acid molecule according to the invention, *i.e*. the functional remorin gene, by means of detection of at least one polymorphism between the functional remorin gene and the non-functional remorin gene, *i.e*. between the sequences of the nucleic acid molecule according to the invention which is involved in Rz2 mediated resistance against BNYVV and the sequences of the variant of the nucleic acid molecule according to the invention which interferes with said resistance, using molecular markers that detect one or more polymorphisms. Examples for those polymorphisms are given in Table 1. Accordingly, the presence of at least one marker locus in the nucleotide sequence of the nucleic acid molecule of the present invention, *i.e*. the functional remorin gene, or in a cosegregating region, indicates that the plant is capable of exhibiting Rz2 mediated resistance against BYNVV. Alternatively, the absence of a marker locus in the nucleotide sequence of said nucleic acid molecule and/or or the presence of at least one marker locus in the nucleotide sequence of the nucleic acid molecule of the present invention interfering with the resistance against BNYVV, *i.e.* the non-functional remorin gene or in a cosegregating region indicates that the plant is not capable of or impaired in exhibiting Rz2 mediated resistance against BNYVV. In addition, the method may include the identification and optionally selection of the plant of the species *Beta vulgaris,* the portion or the seed thereof comprising the nucleic acid molecule of the present invention, *i.e*. the functional remorin gene, which is capable of exhibiting resistance against BNYVV mediated by Rz2, if present in the genome of the plant.

Following the same principle, a plant comprising the nucleic acid molecule according to the invention which interferes with the BNYVV resistance, *i.e.* the non-functional remorin gene, can be identified.

In a preferred embodiment of the present invention, the non-functional remorin variant comprises the nucleic acid and amino acid sequence, respectively as defined above, *i.e.* having the specific nucleic acid and amino acid substitutions, respectively as shown in Tables 1 and 2 and depicted in Figures 2 and 3. A preferred embodiment of the method according to the invention consequently includes the detection of at least one polymorphism that is presented in Figure 2 using molecular markers which detect the polymorphisms - in particular, diagnostic polymorphisms. This detection preferably occurs using at least one molecular marker per polymorphism - in particular, per diagnostic polymorphism. It is known to the person skilled in the art which marker techniques are to be applied to detect a corresponding polymorphism, and how molecular markers for this are constructed (see Advances in Seed Science and Technology Vol. I, Vanangamudi et al., 2008).

As already outlined above, the identification method of the invention includes a step of detecting at least one marker locus in a region cosegregating with the nucleic acid molecule of the present invention. Preferably the cosegregating region is a genomic interval in *Beta vulgaris* on chromosome 3 which comprises and is flanked by marker loci detectable by means of
(i) the markers *s3e5985s01* (SEQ ID No. 33) and *s3p4348s01* (SEQ ID No. 27), and/or
(ii) the markers *s3p4351s01* (SEQ ID No. 29) and *s3e2247xxx* (SEQ ID No. 40),
preferably by means of
(i) the markers *sxh1002s02* (SEQ ID No. 34) and *s3p4348s01* (SEQ ID No. 27), and/or
(ii) the markers *s3p4351s01* (SEQ ID No. 29) and *sxn1017s02* (SEQ ID No. 39),
more preferably means of
(i) the markers *sxh2499s01* (SEQ ID No. 35) and *s3p4348s01* (SEQ ID No. 27), and/or
(ii) the markers *sxi0698s01* (SEQ ID No. 36) and *s3p4348s01* (SEQ ID No. 27), and/or
(iii) the markers *s3p4351s01* (SEQ ID No. 29) and *sxe7357s01* (SEQ ID No. 38), and/or
(iv) the markers *s3p4351s01* (SEQ ID No. 29) and *sxh8485s01* (SEQ ID No. 37), and/or
(v) the markers *s3p4351s01* (SEQ ID No. 29) and *s3e5853s01* (SEQ ID No. 26), and/or
(vi) the markers *s3p4351s01* (SEQ ID No. 29) and *s3e5800s01* (SEQ ID No. 32), and/or
(vii) the markers *s3p4351s01* (SEQ ID No. 29) and *s3e4913xxx* (SEQ ID No. 31), and/or
(viii) the markers *s3p4351s01* (SEQ ID No. 29) and *s3e5782s01* (SEQ ID No. 30).

The location of the markers in visualized in Figure 7.

The method of the present invention used for the identification of a plant of the species *Beta vulgaris* comprising the nucleic acid molecule of the present invention, *i.e.* the functional remorin gene, and which is capable of exhibiting resistance against BNYVV mediated by the Rz2 gene, if present in the genome of the plant, is characterized by optionally further comprising the detection of the presence and/or expression of the Rz2 gene or the presence of the polypeptide encoded by the Rz2 gene in the plant, the portion thereof or the seed thereof; and/or the detection of at least one marker locus in the Rz2 gene or in one or more regions cosegregating with the Rz2 gene, preferably cosegregating with a chromosomal interval comprising the nucleic acid molecule of the present invention, *i.e*. the functional remorin gene, and the Rz2 gene. Accordingly, the identified and optionally selected plant, the portion thereof or the seed thereof comprises said nucleic acid molecule and the Rz2 gene.

In a preferred embodiment of said method, the at least one marker locus in the nucleotide sequence of the Rz2 gene is detectable by means of the marker *s3e5853s01* (SEQ ID No. 26) and wherein the in one or more regions cosegregating with the Rz2 gene are genomic intervals in *Beta vulgaris* on chromosome 3 which comprise and are flanked by marker loci detectable by means of
(i) the markers *s3p4351s01* (SEQ ID No. 29) and *s3e5853s01* (SEQ ID No. 26), and/or
(ii) the markers *s3e5853s01* (SEQ ID No. 26) and *s3e2247xxx* (SEQ ID No. 40),
preferably by means of
(i) the markers *s3p4351s01* (SEQ ID No. 29) and *s3e5853s01* (SEQ ID No. 26), and/or
(ii) the markers *s3e5853s01* (SEQ ID No. 26) and *sxn1017s02* (SEQ ID No. 39),
more preferably means of
(i) the markers *s3p4351s01* (SEQ ID No. 29) and *s3e5853s01* (SEQ ID No. 26), and/or
(ii) the markers *s3e5853s01* (SEQ ID No. 33) and *sxe7357s01* (SEQ ID No. 38), and/or
(iii) the markers *s3e5853s01* (SEQ ID No. 26) and *sxh8485s01* (SEQ ID No. 37).

In an alternative preferred embodiment of said method, the in one or more regions cosegregating with the chromosomal interval comprising the nucleic acid molecule of the present invention, *i.e.* the functional remorin gene, and the Rz2 gene are genomic intervals in *Beta vulgaris* on chromosome 3 which comprise and are flanked by marker loci detectable by means of
(i) the markers s3e5985s01 (SEQ ID No. 33) and *s3p4348s01* (SEQ ID No. 27), and/or
(ii) the markers *s3e5853s01* (SEQ ID No. 26) and *s3e2247xxx* (SEQ ID No. 40),
preferably by means of
(i) the markers *sxh1002s02* (SEQ ID No. 34) and *s3p4348s01* (SEQ ID No. 27), and/or
(ii) the markers *s3e5853s01* (SEQ ID No. 26) and *sxn1017s02* (SEQ ID No. 39),
more preferably means of
(i) the markers *sxh2499s01* (SEQ ID No. 35) and *s3p4348s01* (SEQ ID No. 27), and/or
(ii) the markers *sxi0698s01* (SEQ ID No. 36) and *s3p4348s01* (SEQ ID No. 27), and/or
(iii) the markers *s3e5853s01* (SEQ ID No. 26) and *sxe7357s01* (SEQ ID No. 38), and/or
(iv) the markers *s3e5853s01* (SEQ ID No. 26) and *sxh8485s01* (SEQ ID No. 37).

The oligonucleotides or the pairs of oligonucleotides of the present invention are used for identifying and/or selecting a plant of the species *Beta vulgaris* capable exhibiting resistance to BNYVV mediated by Rz2, if present in the genome of the plant, or which capability of resistance to BNYVV mediated by Rz2, if present in the genome of the plant, is impaired.

Furthermore, the present invention also relates to a marker chip ("DNA chip" or microarray) which contains at least one oligonucleotide according to the invention that is suitable for detection. The marker chip is suitable for application in one or more detection methods according to the invention.

The invention likewise includes a method for production of the protein according to the invention. The method includes the provision or cultivation of a cell culture which contains the nucleic acid molecule of the present invention, *i.e*. the functional and non-functional remorin gene, respectively, and the subsequent expression of the corresponding protein.

Furthermore, the present invention also relates to a BNYVV-resistant plant or a portion thereof which was identified, and, if applicable, selected, via a method as described above. In particular, the present invention relates to a population of plants comprising plants that are available according to one of the methods of the invention as described above, and that preferably are resistant to rhizomania, *i.e.* to BNYVV infestation, and are characterized by the presence of a nucleic acid molecule according to the invention which is involved in conferring Rz2 mediated resistance against BNYVV, *i.e.* the functional remorin gene. The population preferably has at least 10 - preferably, at least 50, more preferably, at least 100, particularly preferably, at least 500, and, particularly in agricultural farming, preferably at least 1,000 - plants. The proportion of plants in the population that do not carry said nucleic acid molecule, do carry the nucleic acid molecule according to the present invention which interferes with the BNYVV resistance, *i.e*. the non-functional remorin, and/or are susceptible to BNYVV is preferably below 25% - preferably, below 20%, more preferably, below 15%, even more preferably, 10%, and, in particular, preferably below 5%, if present at all.

The following advantages for the breeding and development of new resistant plant lines of the genus *Beta* may also be achieved via the present invention. Sequence information, as well as the identified polymorphisms which allow a differentiation between functional and non-functional variants of the disclosed gene, *i.e*. between the alleles that are involved in Rz2 mediated BNYVV resistance and the alleles interfering with said resistance, enable the marker development directly in the gene, as described above, as well as in the upstream and downstream regions, which represents an important improvement for the plant breeder - in particular, with regard to the development of optimized elite lines without "linkage drag." Moreover, knowledge about the sequential structure may be used for the identification of additional remorin or remorin-like genes which are homologous or orthologous, for example.

Therefore, the present invention also encompasses a method for the identification of additional nucleic acid molecules encoding polypeptides or additional proteins that are involved in Rz2 mediated resistance against BNYVV in a plant in which the polypeptide is expressed. The person skilled in the art may thereby use databases, employing suitable search profiles and computer programs for the screening for homologous sequences or for sequence comparisons. Moreover, by means of conventional molecular biology techniques, the person skilled in the art may himself derive additional DNA sequences encoding remorin proteins, and use these within the scope of the present invention. For example, suitable hybridization probes may be derived from the sequence of the nucleic acid molecule according to the invention and be used for the screening of genomic and/or cDNA banks of the desired organism. The person skilled in the art may here resort to customary hybridization, cloning, and sequencing methods, which, for example, are listed in Sambrook et al., Molecular Cloning: A Laboratory Manual, 3rd ed., Cold Spring Harbor Laboratory Press, Cold Spring Harbor, NY, 2001. Using known sequences, the person skilled in the art may also synthesize and use oligonucleotide primers to amplify sequences of Rz2 resistance-involved nucleic acid molecules, nucleic acid molecules encoding polypeptides or additional proteins that are involved in Rz2 mediated resistance against BNYVV in a plant, or nucleic acid molecules encoding other or new remorin alleles.

In one embodiment, the present invention therefore encompasses a method for the identification of a nucleic acid molecule which encodes a polypeptide that is involved in conferring a resistance against BNYVV in a plant of the species *Beta vulgaris* in which the polypeptide is expressed. The method thereby includes the comparison of the amino acid sequence of the polypeptide according to the invention with amino acid sequences from a sequence database, or with sequences of allelic variants of the polypeptide according to the invention in genotypes of the species *Beta vulgaris.* Furthermore, the method according to the invention includes the identification of an amino acid sequence or of an allelic variant that is at least 80% identical to the amino acid sequence of the polypeptide according to the invention, as well as the introduction of a nucleic acid molecule encoding the identified amino acid sequence or allelic variant in a plant of the species *Beta vulgaris* comprising the Rz2 resistance gene; expression of the nucleic acid molecule in said plant; and, optionally, subsequent verification of the resistance against BNYVV.

As described in the preceding, additional proteins being involved in Rz2 mediated BNYVV resistance or their coding genes, *i.e.,* homologs, analogs, and orthologs, that are at least 70% - preferably, at least 80%, particularly preferably, at least 90%, especially preferably, at least 95%, or even 98% - identical to the amino acid sequence of the polypeptide which is encoded by the nucleic acid molecule according to the invention may be identified via classical bioinformatic approaches (database searches and computer programs for screening for homologous sequences).

The term, homolog(s), thereby means that the genes concerned (from two different plant species) have essentially the same function and a common ancestor, and therefore typically show a significant identity in their nucleic acid or coded amino acid sequences. However, there are also many genes that are homologous to one another, without protein sequences resulting in a meaningful paired alignment. In contrast to this, the term, analog(s), describes genes or proteins that (likewise) have an identical or similar function, but are not created from the same structure, *i.e.,* have no common ancestor. In this case, often, no significant identity can be established in their nucleic acid or encoded amino acid sequence, or, in the best case, in specific functional domains.

In the context of genome sequencing, homologs are, for annotation, more finely classified. The terms, orthology and paralogy, have been introduced for this. Orthologs are genes that are connected via a speciation event. Paralogs are genes that trace back to a duplication event.

A gene is fundamentally a homolog or analog or ortholog in the sense of the present invention if it is involved in conferring Rz2 mediated BNYVV resistance in a plant. To verify this, methods which have already been described in the preceding and known to the person skilled in the art are used, *e.g.,* the amplification of the identified homolog or analog or ortholog by means of PCR, cloning in expression vectors, introduction into the target plant or plant cell, and checking the resistance.

The present invention also relates to the use of the nucleic acid molecule of the present invention, *i.e*. the functional remorin allele, in a genetic or molecular stack with other genetic elements which may confer agronomically advantageous properties to the plant. The economic value of cultivated plants may thereby be markedly increased, in that, for example, the yield performance is increased in comparison to plants that possess the same genetics, but have not been furnished with said nucleic acid molecule or which comprise the non-functional variant of the nucleic acid molecule of the present invention. In particular, the present invention relates to the use of the identified remorin gene in methods for controlling an infestation with the pathogen BNYVV in the agricultural or horticultural cultivation of plants of the genus *Beta, e.g.,* encompassing the identification and selection of plants of the genus *Beta* with the aid of one of the methods described in the preceding and/or the cultivation of the plants so selected or descendants thereof. The present invention thus includes a method for the cultivation of plants of the species *Beta vulgaris,* including, in a first step, the provision of BNYVV-resistant plants of the species *Beta vulgaris* according to the invention, or the production of plants of the species *Beta vulgaris* with the aid of the production method according to the invention, or the identification and selection of plants of the species *Beta vulgaris* with the aid of the identification method according to the invention that has been described in the preceding, wherein the provision may include planting the corresponding seedlings or sowing the corresponding seeds of the present invention; and including, in a second step, the cultivation of the plants from the first step, or the deployment of seed stock of the plants from the first step, or the raising of plants from the first step. The cultivation method thereby counteracts an infestation of the cultivated plants by BNYVV. The cultivation method may be part of a method for producing sugar. The method for the production of sugar includes the steps of the cultivation method, and additionally, as a penultimate step, the harvesting of the cultivated plants, and, as a last step, the extraction of sugar from the aforesaid plants.

The cultivation method may also be part of a method for producing seed stock. The method for the production of seed stock includes the steps of the cultivation method, and additionally, as a penultimate step, the vernalization of the cultivated plants, and, as a last step, the extraction of seeds from the aforesaid plants.

The extracted seeds may optionally be pelleted, in order to obtain pelleted seed stock of the species *Beta vulgaris.* In this instance, it is a method for the production of pelleted seed stock. Moreover, the method for the production of seed stock may be designed as a method for the production of BNYVV-resistant seed stock. The method for the production of BNYVV-resistant seed stock includes the steps of the method described above for the production of seed stock, and additionally, as a last step, the verification of the nucleic acid according to the invention, *i.e*. the functional remorin gene according to a method described herein in at least one of the extracted seeds - preferably, in at least 0.1% or in at least 1% of the extracted seeds. The verification is particularly preferably implemented so that the seed remains germinable. This means that the extraction of the DNA required for verification from the seed does not neutralize the germinability of the seed. In such an instance, the verification of the nucleic acid according to the invention may have taken place in an especially large proportion of all extracted seeds. For example, the verification may take place in at least 2% - preferably, at least 3%, particularly preferably, at least 4% - of all extracted seeds.

The plants according to the invention, their cells, or seeds or seed stock according to the invention may possess additional, agronomically advantageous properties, or be furnished with such. One example is the tolerance or resistance to an herbicide such as glyphosate, glufosinate, or ALS inhibitors. The tolerance to glyphosate or an ALS-inhibitor herbicide is preferred. A specific embodiment of the glyphosate resistance is disclosed in US 7,335,816 B2. Such a glyphosate resistance is, for example, available from seed stock stored at the NCIMB, Aberdeen (Scotland, UK), under the access number, NCIMB 41158 or NCIMB 41159. Such seeds may be used in order to obtain a glyphosate-tolerant sugar beet plant. The glyphosate resistance may also be introduced into other species of the genus *Beta* via crossing.

A specific embodiment of the ALS-inhibitor herbicide resistance is disclosed in the document, WO2012/049268 A1. For example, such an ALS-inhibitor herbicide resistance is available from a deposit of NCIMB, Aberdeen, UK, under the number NCIMB 41705. Furthermore, such an ALS-inhibitor resistance may be produced via tilling or site-directed mutagenesis, e.g., via gene editing, such as through the use of CRISPR/Cas, CRISPR/Cpf1, TALENS or zinc finger nucleases. Numerous additional herbicides and their applicability are known to the person skilled in the art from the prior art. He may resort to the prior art in order to achieve knowledge of which genetic elements are to be used in what manner in order to implement a corresponding tolerance in plants.

Further examples of agronomically advantageous properties are additional pathogen resistances, wherein pathogens may be amongst other insects, viruses, nematodes, bacteria, or fungi, cold tolerance or frost tolerance as well as water usage efficiency, nitrogen usage efficiency, and yield. Here, the person skilled in the art may also resort to the prior art to find suitable genetic elements.

In addition to relating to the plant according to the invention, the present invention also relates to seeds or descendants, or to an organ, a plant part, a tissue, or a cell thereof in the production of products that are typically produced from sustainable raw materials, such as foodstuffs and animal feed - preferably, sugar or syrup (molasses), wherein the molasses is also used for industrial applications, e.g., in alcohol production or as a growing medium for the production of biotechnological products, in the production of materials or substances for the chemical industry, e.g., refined chemicals, pharmaceuticals or precursors thereof, diagnostics, cosmetics, bioethanol, or biogas. An example of the use of sugar beet as a biogenic raw material in biogas plants is described in the application DE 10 2012 022 178 A1; see, for example, paragraph 10.

The following examples explain the invention, but without limiting the subject matter of the invention. Unless indicated otherwise, standard molecular biology methods have been used; see, for example, Sambrook et al., Molecular Cloning: A Laboratory Manual, 3rd ed., Cold Spring Harbor Laboratory Press, Cold Spring Harbor, NY, 2001, Fritsch *et al.,* Cold Spring Harbor Laboratory Press: 1989; Mayer et al., Immunochemical Methods in Cell and Molecular Biology, eds., Academic Press, London, 1987, and Weir et al., Handbook of Experimental Immunology, Volumes I-IV, Blackwell, eds., 1986.

Some of the most important sequences according to the invention are explained in detail in the following:
SEQ ID No. 2 - genomic sequence of the non-functional remorin gene (haplotyp 1)
SEQ ID No. 3 - open reading frame of the non-functional remorin gene (haplotyp 1)
SEQ ID No. 4 - amino acid sequence of the non-functional remorin polypeptide (haplotype 1)
SEQ ID No. 6 - genomic sequence of the functional remorin gene (haplotyp 2)
SEQ ID No. 7 - open reading frame of the functional remorin gene (haplotyp 2)
SEQ ID No. 8 - amino acid sequence of the functional remorin polypeptide (haplotype 2)
SEQ ID No. 10 - genomic sequence of the functional remorin gene (haplotyp 3)
SEQ ID No. 11 - open reading frame of the functional remorin gene (haplotyp 3)
SEQ ID No. 12 - amino acid sequence of the functional remorin polypeptide (haplotype 3)
SEQ ID No. 14 - genomic sequence of the functional remorin gene (haplotyp 4)
SEQ ID No. 15 - open reading frame of the functional remorin gene (haplotyp 4)
SEQ ID No. 16 - amino acid sequence of the functional remorin polypeptide (haplotype 4)
SEQ ID No. 18 - genomic sequence of the functional remorin gene (haplotyp 5)
SEQ ID No. 19 - open reading frame of the functional remorin gene (haplotyp 5)
SEQ ID No. 20 - amino acid sequence of the functional remorin polypeptide (haplotype 5)

### EXAMPLES

### Example 1: Identification and genetic mapping of a gene being involved in Rz2 mediated BNYVV resistance, i.e. the functional remorin gene, as well as of the variant interfering with said resistance, i.e. the non-functional remorin gene.

In order to localize the non-functional remorin gene leading after expression of the corresponding polypeptide to suppression of the Rz2 mediated BNYVV resistance in plants of the species *Beta vulgaris,* genetic mapping has been performed. For this purpose, two *Beta vulgaris* lines comprising the Rz2 resistance allele were crossed, wherein one parent line comprised the non-functional remorin gene and the second parent line was resistant to rhizomania. The gene of interest could then be genetically localized in the offspring population near the Rz2 locus. However, this area still contained too many genes to name a specific candidate.

Parallel to genetic mapping, the patterns of molecular markers in multigenic breeding material were analyzed. Five genotypes with a recombination between the Rz2 gene and the predicted locus containing the non-functional remorin gene were self-pollinated. Phenotypic resistance data were collected from 20 plants of the offspring. A variance analysis was performed and markers were determined. The marker *s3e5798s01* (allele X: A, allele Y: G; primer allele X (SEQ ID NO: 24), primer allele Y (SEQ ID NO: 25)) showed the highest evidence for the positioning of the remorin gene. The genetic position of the marker is 40.99598509 cM on chromosome 3 of a genetic map. The physical position of the underlying SNP on the reference sequence (SBKv7) is 8,887,000 bp on chromosome 3.

Thus, this marker is genetically located only about 0.75 cM and physically about 381 kbp away from the RZ-2 resistance gene, detectable for example by marker *s3e5853s01* (SEQ ID NO: 26) at genetic position 41.7471132 cM on chromosome 3 (physical position: 9,268,704 bp).

Analysis of the reference sequence for this region showed that the marker *s3e5798s01* (SEQ ID NOs. 24 and 25) is located in an annotated gene model (g23238.t1), wherein the gene contains a remorin domain (C-terminal region).

Furthermore, genetic fine mapping has been performed to better resolve the region of interest. In particular recombinants were searched for which show the different allele combinations of Rz2 (BNYVV resistant) and remorin (presence of the non-function allele - yes (A) / no (B); Figure 8). For this purpose, 2730 plants of a dividing population were analyzed with markers. There are sufficient genetic markers between the Rz2 gene and the identified remorin gene to reliably identify such recombinants; see Figure 8. These recombinants were then tested again in the resistance test: In the test of progeny, genotypes at the H positions (= heterozygous for the non-functional allele) split 1:2:1 (A:H:B). The A and B offspring are tested as contrasts in a resistance test.

### Example 2: Functional validation of the remorin gene

Several variants of transient assays for functional analysis are performed, preferably on leaf tissue. These are based on the transient gene expression of test constructs (for example plasmids, wherein the genes to be tested are under the expression control of a promoter active in plants) and the subsequent measurement of a hypersensitive response (cell death reaction) as a proxy for triggering resistance reactions. For the transfer of the test constructs into plant tissue, for example, a particle bombardment or a local transformation by means of *Agrobacterium* infiltration is used. Cell death reactions can be measured visually (cell collapse, yellowing, fluorescence under UV light) or by measuring the reporter gene activity of a co-transformed reporter gene (*e.g.* luciferase). A reduction in reporter gene activity correlates with increased cell death activity.

### Assay 1: Comparison of the cell death reaction after expression of the TGB1 gene in two genotypes comprising the resistance allele of Rz2 (Figure 4)

Both genotypes tested contain the resistance allele of Rz2. Genotype 1 contains the functional allele of the remorin, genotype 2 contains the non-functional allele of the remorin. After the transfer of the TGB1 gene of *Beet necrotic yellow vein virus* (BNYVV) (SEQ ID NOs: 41, 42) or homologous TGB1 genes, for example from *Beet soil borne mosaic virus* (SEQ ID NO: 43; Wetzel and Varrelmann (2018)) into the two genotypes, a cell death reaction can be measured in genotype 1. In genotype 2, a corresponding cell death reaction does not occur or its expression is significantly reduced compared to genotype 1.

This test can also be used to screen new genotypes for functional alleles or allele combinations of Rz2 and remorin. It is also potentially suitable for screening genotypes having other genes interfering with the Rz2 mediated BNYVV resistance.

### Assay 2: Comparison of the cell death reaction after expression of the TGB1 gene and remorin genes in genotypes comprising the resistance allele of Rz2 (Figure 5)

A genotype comprising the resistance allele of Rz2 and the functional allele of remorin is used for the assay. In this genotype, TGB1 is transiently expressed in combination with the non-functional allele of remorin. For comparison, transient expression of TGB1 in combination with an empty vector control or transient expression of TGB1 in combination with the functional allele of remorin is performed. In the latter two combinations, cell death triggering can be measured. In comparison, the degree of cell death triggering for the combination of TGB1 and non-functional remorin allele is reduced or absent altogether.

These results indicate that the non-functional remorin allele is dominant over the functional allele of remorin. Dominant means that even in the heterozygous state (non-functional allele on one chromosome set / functional remorin allele on the other chromosome set), it develops its full effect, *i.e.* the non-functional allele inhibits or prevents the development of resistance in any case. Conversely, this means that Rz2 can only develop its resistance effect if the functional remorin gene is homozygous.

### Assay 3: Comparison of the cell death reaction after expression of the TGB1 gene with combinations of alleles of remorin and Rz2 (Figure 6)

The transient expression of TGB1, Rz2 and a functional or non-functional allele of remorin can take place in a genotype without Rz2 or in alternative test systems such as *Nicotiana* species. When functional remorin is used, a higher cell death rate can be measured compared to the use of non-functional remorin.

The test can also be used in a modified form to compare genotypes with allelic variations of remorin or Rz2. The test construct with the respective gene to be examined is kept out of the transient expression and only the other components are expressed transiently. The expression of the cell death reaction can be used as a measure for the functionality of the variable alleles.

## Claims

1. A nucleic acid molecule encoding a polypeptide which is involved in resistance against *Beet necrotic yellow vein virus* (BNYVV) mediated by the Rz2 resistance gene in a plant in which the polypeptide is expressed, wherein the nucleic acid molecule comprises a nucleotide sequence which is selected from the group consisting of:
(a) a nucleotide sequence that encodes a polypeptide having the amino acid sequence according to SEQ ID No. 45;
(b) a nucleotide sequence that comprises the coding DNA sequence according to SEQ ID No. 44
(c) a nucleotide sequence that hybridizes with a complementary sequence of the nucleotide sequence according to (a) or (b) under stringent conditions;
(d) a nucleotide sequence that encodes a polypeptide which, via substitution, deletion, and/or addition of one or more amino acids of the amino acid sequence, differs from a polypeptide that is encoded by the nucleotide sequence according to (a) or (b);
(e) a nucleotide sequence that encodes a polypeptide which has an amino acid sequence that is at least 70% identical to the amino acid sequence according to SEQ ID No. 45; and
(f) a nucleotide sequence that comprises the coding DNA sequence which is at least 70% identical to the DNA sequence according to SEQ ID No. 44,
wherein the amino acid sequence of the encoded polypeptide has a proline (P) at position 25 with reference to SEQ ID No. 45, a proline (P) at position 72 with reference to SEQ ID No. 45 or a valine (V) at position 186 with reference to SEQ ID No. 45, or wherein the nucleic acid of the coding DNA sequence has a cytosine (c) at position 73 with reference to SEQ ID No. 44, a cytosine (c) at position 214 with reference to SEQ ID No. 44, or a guanine (g) at position 556 with reference to SEQ ID No. 44, preferably wherein the polypeptide has the amino acid sequence of SEQ ID No. 8, 12, 16, or 20, or the nucleotide sequence that encodes said polypeptide is SEQ ID No. 5, 6, 7, 9, 10, 11, 13, 14, 15, 17, 18 or 19, or the coding DNA sequence is a nucleotide sequence according to SEQ ID No. 7, 11, 15, or 19.

2. A nucleic acid molecule which specifically hybridizes to the nucleic acid molecule according to claim 1 and encodes a non-functional allele of the polypeptide, which interferes with resistance against BNYVV mediated by the Rz2 resistance gene in a plant in which the polypeptide is expressed, wherein the nucleic acid molecule preferably comprises a nucleotide sequence which is selected from
(a) a nucleotide sequence that encodes a polypeptide having an amino acid sequence according to SEQ ID No. 4 or a polypeptide having an amino acid sequence that is at least 70% identical to SEQ ID No. 4, wherein the polypeptide comprises an amino acid sequence having at least one amino acid substitution due to one or more mutations in the nucleotide sequence, preferably wherein proline (P) at position 25 with reference to SEQ ID No. 45, proline (P) at position 72 with reference to SEQ ID No. 45, and/or valine (V) at position 186 with reference to SEQ ID No. 45 is substituted with another amino acid, preferably wherein proline (P) at position 25 is substituted with alanine (A), proline (P) at position 72 is substituted with threonine (T), and/or valine (V) at position 186 is substituted with isoleucine (I); and/or
(b) a nucleotide sequence that comprises the coding DNA sequence according to SEQ ID No. 3, that comprises a coding DNA sequence which is at least 70% identical to the DNA sequence according to SEQ ID No. 3, or that hybridizes with a complementary sequence of SEQ ID No. 3 under stringent conditions, wherein the nucleotide sequence comprises at least one nucleic acid substitution due to at least one mutation leading to an amino acid substitution, preferably wherein one or more nucleotides are substituted at positions 73-75 with reference to SEQ ID No. 44, at positions 214-216 with reference to SEQ ID No. 44 and/or at positions 556-558 with reference to SEQ ID No. 44, preferably wherein cytosine (c) at position 73 is substituted with guanine (g), cytosine (c) at position 214 is substituted with adenine (a), and/or guanine (g) at position 556 is substituted with adenine (a).

3. A polypeptide which is encoded by the nucleic acid molecule according to claim 1 or 2.

4. A vector or expression cassette which comprises the nucleic acid molecule according to claim 1 or 2, preferably wherein the nucleic acid molecule is operably linked to a heterologous regulatory element.

5. A cell which comprises the nucleic acid molecule according to claim 1 or 2, a polypeptide according to claim 3, or a vector or expression cassette according to claim 4.

6. A plant or a portion thereof comprising the nucleic acid molecule according to claim 1 endogenously or transgenically or the vector or the expression cassette which comprises the nucleic acid molecule according to claim 1 as defined in claim 4 as transgene, preferably wherein the plant additionally comprises the Rz2 resistance gene mediating BNYVV resistance endogenously or transgenically.

7. Seed or a descendant of the plant according to claim 6, wherein the seed or the descendant comprises the nucleic acid molecule according to claim 1 endogenously or transgenically, preferably wherein the seed has been technically treated, whereby the technical treatment is selected from the group consisting of:
(a) Polishing,
(b) Dressing, preferably pelleting,
(c) Incrustation, and
(d) Colouring.

8. Oligonucleotide or a pair of oligonucleotides of at least 15, 16, 17, 18, 19, or 20, preferably at least 21, 22, 23, 24, or 25, particularly preferably at least 30, 35, 40, 45, or 50, and particularly preferably at least 100, 200, 300, or 500 nucleotides in length, wherein the oligonucleotide or the pair of oligonucleotides
(i) specifically hybridizes to a nucleotide sequence as defined in any claim 2;
(ii) specifically hybridizes to a nucleotide sequence as defined in claim 1;
(iii) specifically hybridizes to a region in the *Beta vulgaris* genome that, in *Beta vulgaris,* cosegregates with the nucleic acid molecule according to claim 2 and/or with the nucleic acid molecule according to claim 2 together with the Rz2 gene; and/or
(iv) specifically hybridizes to a region in the *Beta vulgaris* genome that, in *Beta vulgaris,* cosegregates with the nucleic acid molecule according to claim 1 and/or with the nucleic acid molecule according to claim 1 together with the Rz2 gene,
preferably wherein the oligonucleotide or pair of oligonucleotides is selected from the group consisting of SEQ ID NO: 24 to 40.

9. A method for conferring, restoring or increasing the resistance to BNYVV in a plant of the species *Beta vulgaris* comprising the Rz2 resistance gene mediating BNYVV resistance endogenously or transgenically, including the following steps:
(i) integration or introgression of the nucleic acid molecule according to claim 1 by means of homology-directed repair or homologous recombination - preferably, promoted by site-directed nuclease - into the genome of at least one cell of a plant of the species *Beta vulgaris,* and optional regeneration of a plant from the plant cell; or
(ii) increasing the expression of the nucleic acid molecule according to claim 1 in the plant - preferably, via modification of the native promoter or via fusion of the nucleic acid molecule with a heterologous promoter that exhibits a higher activity in comparison to the native promoter - in particular, after BNYVV infection; or
(iii) increase in the activity and/or stability of the functional allele of the polypeptide which is encoded by the nucleic acid molecule according to claim 1 as defined in claim 3 via modification of the nucleotide sequence according to claim 1;
(iv) transformation of a plant cell with the nucleic acid molecule according to claim 1, or the vector or the expression cassette which comprises the nucleic acid molecule according to claim 1 as defined in claim 4, and optionally regeneration of a transgenic plant from the transformed plant cell; or
(v) mutagenesis of a plant or plant cells comprising the nucleic acid molecule according to claim 2, and screening/selecting for plants or plant cells, respectively, in which a functional allele of the polypeptide encoded by the nucleic acid molecule according to claim 1 has been restored and/or the expression of the non-functional polypeptide has been abolished.

10. A method for producing a BNYVV-resistant plant comprising the nucleic acid molecule according to claim 1, including the following steps:
(I) provision of a plant of the genus *Beta* or a plant cell of the genus *Beta* comprising the Rz2 resistance gene mediating BNYVV resistance endogenously or transgenically; and
(IIa) transformation of the plant cell of (I) with the nucleic acid molecule according to claim 1, or the vector or the expression cassette which comprises the nucleic acid molecule according to claim 1 as defined in claim 4; and
(IIb) regeneration of a transgenic plant from the transformed plant cell; or
(IIIa) introduction of a site-directed nuclease or nickase and optionally a repair matrix, or a site-directed base editor into the plant cell, wherein the site-directed nuclease is able to generate at least one single-strand break of the DNA or at least one double-strand break of the DNA at a predetermined location in the genome of the cell and the repair matrix comprises the nucleic acid molecule according to claim 1, or wherein the site-directed base editor is able to generate at least one single-strand break of the DNA at a predetermined location in the genome of the cell and to convert at least one nucleobase; and
(IIIb) optionally, cultivation of the cell from (IIIa) under conditions that allow modification of the genome at the predetermined location, selected from
a) a replacement of at least one nucleotide;
b) a deletion of at least one nucleotide;
c) an insertion of at least one nucleotide;
d) any combination of a) - c),
optionally by homology-directed repair or homologous recombination,
wherein the nucleic acid molecule is integrated into the genome of the plant; and
(IIIc) regeneration of a plant from the cell modified in (IIIb);
preferably wherein the predetermined location is in the nucleic acid molecule according to claim 2 or a position which is at most 10.000 base pairs upstream or downstream away from the nucleic acid molecule according claim 2; or
(IVa) crossing of the plant of (I) with the plant according to claim 6; and
(IVb) identifying and selecting a plant comprising the Rz2 resistance gene mediating BNYVV endogenously or transgenically and the nucleic acid molecule according to claim 1 endogenously or transgenically, wherein the Rz2 resistance gene and/or the nucleic acid molecule according to claim 1 is present homozygously in the genome of the plant.

11. A method for identifying a plant of the species *Beta vulgaris,* a portion thereof or a seed thereof comprising the nucleic acid molecule according to claim 1, wherein the plant is capable of exhibiting resistance against BNYVV mediated by Rz2, if present in the genome of the plant, comprising:
(i) detection of the presence and/or expression of the nucleic acid molecule according to claim 1, or the presence of the polypeptide which is encoded by the nucleic acid molecule according to claim 1 as defined in claim 3 in the plant, the portion thereof or the seed thereof; and/or
(ii) detection of at least one marker locus in the nucleotide sequence of the nucleic acid molecule according to claim 1 or in one or more cosegregating regions thereof, preferably wherein the detection of the at least one marker locus in the nucleotide sequence of the nucleic acid molecule according to claims 1 comprises the use of the oligonucleotide or the pair of oligonucleotides according to claim 8, and wherein the one or more cosegregating regions are genomic intervals in *Beta vulgaris* on chromosome 3 which comprise and are flanked by marker loci detectable by means of
(a) the markers *s3e5985s01* (SEQ ID No. 33) and *s3p4348s01* (SEQ ID No. 27), and/or
(b) the markers *s3p4351s01* (SEQ ID No. 29) and *s3e2247xxx* (SEQ ID No. 40); and
(iii) identification and optionally selection of the plant of the species *Beta vulgaris,* the portion thereof or the seed thereof comprising the nucleic acid molecule according to claim 1, which is capable of exhibiting resistance against BNYVV mediated by Rz2, if present in the genome of the plant, based on the detection of (i) and/or (ii).

12. A method for identifying a plant of the species *Beta vulgaris,* a portion thereof or a seed thereof comprising the nucleic acid molecule according to claim 2, comprising:
(i) detection of the presence and/or expression of the nucleic acid molecule according to claim 2, or the presence of the polypeptide which is encoded by the nucleic acid molecule according to claim 2 as defined in claim 3 in the plant, the portion thereof or the seed thereof; and/or
(ii) detection of at least one marker locus in the nucleotide sequence of the nucleic acid molecule according to claim 2 or in one or more cosegregating regions thereof, preferably wherein the detection of the at least one marker locus in the nucleotide sequence of the nucleic acid molecule according to claims 2 comprises the use of the oligonucleotide or the pair of oligonucleotides according to claim 8, and wherein the one or more cosegregating regions are genomic intervals in *Beta vulgaris* on chromosome 3 which comprise and are flanked by marker loci detectable by means of
(a) the markers *s3e5985s01* (SEQ ID No. 33) and *s3p4348s01* (SEQ ID No. 27), and/or
(b) the markers *s3p4351s01* (SEQ ID No. 29) and *s3e2247xxx* (SEQ ID No. 40); and
(iii) identification and optionally selection of the plant of the species *Beta vulgaris,* the portion thereof or the seed thereof comprising the nucleic acid molecule according to claim 2, based on the detection of (i) and/or (ii),

13. The method according to claim 11 additionally comprising
(A) detection of the presence and/or expression of the Rz2 gene or the presence of the polypeptide encoded by the Rz2 gene in the plant, the portion thereof or the seed thereof; and/or
(B) detection of at least one marker locus in the Rz2 gene or in one or more regions cosegregating with the Rz2 gene, preferably cosegregating with a chromosomal interval comprising the nucleic acid molecule according to claim 1 and the Rz2 gene;
wherein in (iii) of claim 11 the identified and optionally selected plant, the portion thereof or the seed thereof comprises the nucleic acid molecule according to claim 1, and the Rz2 gene, based on the detection of (i) and/or (ii) of claim 11 and detection of (B).

14. A method for cultivation of plants of the species *Beta vulgaris,* including
(i) planting seedlings of the plant according to claim 6 or sowing seeds according to claim 7, and
(ii) grown the plants from the seedlings or seeds, and
(iii) optionally, harvest root beets from the grown plants;
wherein the method counteracts an infestation of the cultivated plants with BNYVV.

15. Use of the nucleic acid molecule according to claim 1 or 2, the polypeptide according to claim 3 and/or the vector or expression cassette according to claim 4 for the production of BNYVV-resistant plants, preferably of plants of the species *Beta vulgaris,* more preferably of plants of *Beta vulgaris* ssp. *vulgaris* var. *vulgaris, Beta vulgaris* ssp. *vulgaris* var. *conditiva,* oder *Beta vulgaris* ssp. *vulgaris* var. *crassa*/*alba.*
